# EUROPEAN PATENT APPLICATION

(11) **EP 2 883 955 A1**
(43) Date of publication of application: **17.06.2015**
(21) Application number: 13825285.3
(22) Date of filing: 31.07.2013
(51) Int. Cl.: C12N 15/09, A01H 1/00, A01H 5/00, C07K 14/415, C12P 19/34, C12Q 1/68

(54) **PARTHENOCARPY REGULATION GENE AND USE THEREOF**

(30) Priority: 31.07.2012 JP 2012170506
(71) Applicant: Incorporated Administrative Agency National Agriculture And Food Research Organization, Tsukuba-shi, Ibaraki 305-8517 (JP)
(72) Inventor: NUNOME, Tsukasa, Tsu-shi Mie 514-2392 (JP); HONDA, Ichiro, Maebashi-shi Gunma 371-0801 (JP); OHYAMA, Akio, Tsu-shi Mie 514-2392 (JP); FUKUOKA, Hiroyuki, Tsu-shi Mie 514-2392 (JP); YAMAGUCHI, Hirotaka, Tsu-shi Mie 514-2392 (JP); MIYATAKE, Koji, Tsu-shi Mie 514-2392 (JP)
(74) Representative: Graf von Stosch, Andreas
(86) International application number: PCT/JP2013/070801
(87) International publication number: WO 2014/021398

(57) **Abstract**

The present invention provides a parthenocarpy regulatory gene including any of the following polynucleotides and use of the parthenocarpy regulatory gene. (1) A polynucleotide that encodes an amino acid sequence represented by SEQ ID NO: 1, (2) a polynucleotide that encodes an amino acid sequence having a sequence identity of 70% or higher relative to the amino acid sequence represented by SEQ ID NO: 1, (3) a polynucleotide that encodes an amino acid sequence in which 1 to 87 amino acids are substituted and/or the like in the amino acid sequence represented by SEQ ID NO: 1, and (4) a polynucleotide that is hybridized with a polynucleotide having a sequence complementary to the polynucleotide described in the above (1) under a stringent condition.

## Description

### Technical Field

The present invention relates to a parthenocarpy regulatory gene and a method for producing a plant with the use of the parthenocarpy regulatory gene.

### Background Art

Parthenocarpy is a trait that fruit is enlarged without pollination and/or fertilization. This trait is advantageous in terms of harvesting fruits without carrying out, for example, work such as pollination induction with the use of a flower visiting insect or fructification induction by plant chemical application.

Conventionally, with regard to tomato, a parthenocarpic tomato line has been distributed as a genetic resource, and breeding of a commercial cultivar has been carried out by the use of the parthenocarpic tomato line as a breeding material. Moreover, a pat-2 gene exists as a parthenocarpy-related gene of tomato, and the gene reportedly has a monogenic recessive trait.

Patent Literature 1 discloses a method for producing a seedless parthenocarpic tomato by crossbreeding (i) a tomato strain that includes at least one parthenocarpy-related gene as a male and (ii) a male sterile tomato strain that includes at least one parthenocarpy-related gene as a female. Further, Patent Literature 1 discloses the pat-2 as one of the parthenocarpy-related genes.

Patent Literature 2 discloses a method for creating a parthenocarpic tomato plant or a parthenocarpic and male sterile tomato plant, and the method includes introgressing of a genetic region from chromosome 4, 5 and/or 12 of *S*. *habrochaites.* Further, Patent Literature 2 discloses relation of the pat-2 to double recessive parthenocarpy.

### Citation List

### [Patent Literature]

### [Patent Literature 1]

Japanese Patent Application Publication Tokukai No. 2011-045373 (Publication date: March 10, 2011)

### [Patent Literature 2]

Japanese Translation of PCT International Application Tokuhyo No. 2010-527586 (Publication date: August 19, 2010)

### Summary of Invention

### Technical Problem

As disclosed in Patent Literatures 1 and 2, in the broad sense of tomato, the existence of a gene that relates to parthenocarpy is genetically recognized. However, details of a chromosomal location and of a structure of such a gene have not been reported yet.

Depending on cultivation conditions or cultivar lines, phenotypic expression of parthenocarpy can be unstable, and therefore it is sometimes difficult to evaluate parthenocarpy if the evaluation is based only on appearance of a plant. Moreover, in order to evaluate parthenocarpy, much manpower and time are required for emasculation before anthesis, evaluation of fruit set, and the like.

When the gene responsible for parthenocarpy is homozygous, the parthenocarpy trait can be evaluated based on appearance of tomato because parthenocarpy is a recessive trait. Moreover, in order to obtain a F1 cultivar that has parthenocarpy trait, it is necessary to introduce parthenocarpy trait into both parents, and therefore breeding of tomato cultivar cannot be carried out efficiently.

The present invention is accomplished in view of the problems, and its object is (i) to identify a new gene that is responsible for parthenocarpy and (ii) to provide use of the new gene.

### Solution to Problem

The inventors of the present invention diligently studied for attaining the object and, as a result, the inventors of the present invention have succeeded to identify and isolate a new gene that is responsible for parthenocarpy, and the present invention has been thus accomplished.

That is, the present invention encompasses any of the following 1) through 6):
1) A method for evaluating parthenocarpy of a plant, the method including the step of: checking whether or not expression of a parthenocarpy regulatory gene, which includes the polynucleotide described in any of (1) through (4) below, is inhibited in the plant; or checking whether or not a function of the polypeptide, which is encoded by a parthenocarpy regulatory gene, is inhibited in the plant.
(1) A polynucleotide that encodes a polypeptide having an amino acid sequence represented by SEQ ID NO: 1, (2) a polynucleotide that encodes a polypeptide (i) having a sequence identity of 70% or higher relative to the amino acid sequence represented by SEQ ID NO: 1 and (ii) having a parthenocarpy regulation activity, (3) a polynucleotide that encodes a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity, and (4) a polynucleotide that (i) is hybridized with a polynucleotide, which has a sequence complementary to the polynucleotide described in the above (1), under a stringent condition and (ii) encodes a polypeptide having a parthenocarpy regulation activity.
2) A method for producing a parthenocarpy-regulated plant, the method including the step of: selecting a parthenocarpic plant by carrying out the method described in the above 1).
3) A method for producing a parthenocarpy-regulated plant, the method including the step of: inhibiting, in a plant, gene expression of a parthenocarpy regulatory gene that includes a polynucleotide described in any of (1) through (4) below; or inhibiting, in a plant, a function of a polypeptide encoded by a parthenocarpy regulatory gene.
(1) A polynucleotide that encodes a polypeptide having an amino acid sequence represented by SEQ ID NO: 1, (2) a polynucleotide that encodes a polypeptide (i) having a sequence identity of 70% or higher relative to the amino acid sequence represented by SEQ ID NO: 1 and (ii) having a parthenocarpy regulation activity, (3) a polynucleotide that encodes a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity, and (4) a polynucleotide that (i) is hybridized with a polynucleotide, which has a sequence complementary to the polynucleotide described in the above (1), under a stringent condition and (ii) encodes a polypeptide having a parthenocarpy regulation activity.
4) A parthenocarpy-regulated plant produced by the method described in the above 2) or 3).
5) A parthenocarpy regulatory gene that includes a polynucleotide described in any of (1) through (4) below.
(1) A polynucleotide that encodes a polypeptide having an amino acid sequence represented by SEQ ID NO: 1, (2) a polynucleotide that encodes a polypeptide (i) having a sequence identity of 70% or higher relative to the amino acid sequence represented by SEQ ID NO: 1 and (ii) having a parthenocarpy regulation activity, (3) a polynucleotide that encodes a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity, and (4) a polynucleotide that (i) is hybridized with a polynucleotide, which has a sequence complementary to the polynucleotide described in the above (1), under a stringent condition and (ii) encodes a polypeptide having a parthenocarpy regulation activity.
6) A polypeptide described in any of (1) through (4) below.
(1) A polypeptide having an amino acid sequence represented by SEQ ID NO: 1, (2) a polypeptide (i) having a sequence identity of 70% or higher relative to the amino acid sequence represented by SEQ ID NO: 1 and (ii) having a parthenocarpy regulation activity, (3) a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity, and (4) a polypeptide that (i) is encoded by a polynucleotide which is hybridized, under a stringent condition, with a polynucleotide that has a sequence complementary to a polynucleotide for encoding the polypeptide described in the above (1) and (ii) has a parthenocarpy regulation activity.

### Advantageous Effects of Invention

It is possible to evaluate parthenocarpy trait of a plant or to produce a parthenocarpic plant by using, as an indicator or a target, a gene or a polypeptide encoded by the gene.

### Brief Description of Drawings

Fig. 1 is a view relating to an Example of the present invention and illustrating a linkage map of a region in which a parthenocarpy responsible gene A is located.
Fig. 2 is a schematic view illustrating a vector used to produce RNAi transgenic plant in an Example of the present invention.
Fig. 3 is a view relating to an Example of the present invention and illustrating, in regard to each of RNAi transgenic plants and non-transgenic plants, comparison between stigma-removed fruits and pollinated fruits in terms of mature fruit weight.
Fig. 4 is a view relating to an Example of the present invention and illustrating ratios of mature stigma-removed fruit weights of respective RNAi transgenic plants and non-transgenic plants, where a fruit weight of a pollinated fruit is assumed to 1.
Fig. 5 is a view relating to an Example of the present invention and illustrating a result of analyzing stage-specific and tissue-specific gene expressions of the parthenocarpy responsible gene A.
Fig. 6 is a view relating to an Example of the present invention and illustrating a structure of a cloning vector including a full length of the parthenocarpy responsible gene A.
Fig. 7 is a view relating to an Example of the present invention and illustrating a structure of a binary vector including a full length of the parthenocarpy responsible gene A.
Fig. 8 is a view relating to an Example of the present invention and illustrating a result of measuring mature fruit weights of stigma-removed fruits and pollinated fruits of transgenic tomatoes in each of which the parthenocarpy responsible gene A is expressed.
Fig. 9 is a view relating to an Example of the present invention and illustrating ratios of mature fruit weights of respective of stigma-removed fruits and pollinated fruits of the transgenic tomatoes in each of which the parthenocarpy responsible gene A is expressed, where a fruit weight of a pollinated fruit is assumed to 1.
Fig. 10 is a view relating to an Example of the present invention and illustrating a schematic configuration of an RNAi-induction vector of a parthenocarpy responsible gene SmA of eggplant.
Fig. 11 is a view relating to an Example of the present invention and illustrating a schematic configuration of an RNAi-induction binary vector of a parthenocarpy responsible gene SmA of eggplant.
Fig. 12 is a view relating to an Example of the present invention and illustrating a result of inhibiting expression of a parthenocarpy responsible gene SmA in eggplant by RNAi.
Fig. 13 is a view relating to an Example of the present invention and illustrating comparison in terms of expression pattern between the parthenocarpy responsible gene SmA of eggplant and the parthenocarpy responsible gene A of tomato.
Fig. 14 is a view relating to an Example of the present invention and illustrating a result of segregating traits of parthenocarpy and non-parthenocarpy in regard to tomato with the use of marker sequences.

### Description of Embodiments

The following description will discuss an embodiment of the present invention. Note that the present invention is not limited to this.

### [Definitions of Terms]

In this specification, "polynucleotide" can be reworded as "nucleic acid" or "nucleic acid molecule", and is intended to be a polymer of nucleotides. Moreover, "base sequence" can be reworded as "nucleic acid sequence" or "nucleotide sequence", and is intended to be a sequence of deoxyribonucleotide or a sequence of ribonucleotide, unless otherwise particularly noted.

In this specification, "polypeptide" can be reworded as "protein".

In this specification, "parthenocarpy (parthenocarpic)" is intended to be a trait of a plant whose fruit is enlarged without pollination and/or fertilization.

In this specification, "heterozygote (heterozygous)" is intended to be a state in which different allelic genes are at corresponding gene loci on respective homologous chromosomes, and "homozygote (homozygous)" is intended to be a state in which identical allelic genes are at corresponding gene loci on respective homologous chromosomes.

In this specification, "tomato" is a concept that encompasses, in the broad sense, cultivated species "S. *lycopersicum"* and wild species "*S*. *cheesmaniae*, *S*. *chilense, S. chmielewskii, S*. *galapagense, S*. *habrochaites, S*. *lycopersicoides, S. neorickii, S. pennellii, S*. *peruvianum,* and *S*. *pimpinellifolium",* and is intended to be *"S. lycopersicum"* in the narrow sense.

In this specification, "A and/or B" is a concept that includes both "A and B" and "A or B", and can be reworded as "at least one of A and B".

In this specification, a wording "polynucleotides are complementary to each other" is synonymous with "base sequences of respective polynucleotides are complementary to each other".

### [1. Parthenocarpy regulatory gene]

A parthenocarpy regulatory gene in accordance with the present invention encodes a polypeptide that has an activity (i.e., a parthenocarpy regulation activity) to regulate at least parthenocarpy. The polypeptide "that has an activity to regulate parthenocarpy" indicates that expression of a trait, i.e., parthenocarpy is inhibited by the presence of the polypeptide, that is, parthenocarpy is negatively regulated. Moreover, in a case where expression of the parthenocarpy regulatory gene of the present invention is inhibited or an activity of a polypeptide encoded by the parthenocarpy regulatory gene is inhibited, a plant shows a parthenocarpy trait or has a predisposition of the trait even though parthenocarpy is not shown.

The parthenocarpy regulatory gene of the present invention includes any of polynucleotides described in the following (1) through (4):
(1) A polynucleotide that encodes a polypeptide having an amino acid sequence represented by SEQ ID NO: 1.
(2) A polynucleotide that encodes a polypeptide (i) having a sequence identity of 70% or higher relative to the amino acid sequence represented by SEQ ID NO: 1 and (ii) having a parthenocarpy regulation activity. Note that the sequence identity of the amino acid sequence is preferably 75% or higher or 80% or higher, more preferably 85% or higher, more preferably 90% or higher, further preferably 95% or higher, particularly preferably 96% or higher, 97% or higher, 98% or higher, or 99% or higher. For example, a mutant gene derived from tomato and a homologous gene derived from a plant other than tomato are encompassed in this range.
(3) A polynucleotide that encodes a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity. Note that the number of amino acids which are substituted, deleted, inserted, and/or added is more preferably 1 to 72; or 1 to 58, more preferably 1 to 43, more preferably 1 to 29, further preferably 1 to 14; or 1 to 10, particularly preferably 1 to 5 or 6.

Note that the deletion, substitution, and/or addition of amino acids can be, for example, (i) carried out by artificially introducing a mutation(s) by the use of site-directed mutagenesis such as a Kunkel method (Kunkel et al. (1985): Proc.Natl.Acad.Sci.USA, vol.82. p488-) or (ii) derived from a naturally occurring similar mutant polypeptide.

Moreover, a region in which amino acids are deleted, substituted, and/or added is preferably, in the amino acid sequence represented by SEQ ID NO: 1, a region except for (i) a part from 56th valine to 112th asparagine which is predicted as a Zn finger domain and (ii) a part from 219th lysine to 276th asparagine which is predicted as a homeo domain.
(4) A polynucleotide that (i) is hybridized with a polynucleotide, which has a sequence complementary to the polynucleotide described in the above (1), under a stringent condition and (ii) encodes a polypeptide having a parthenocarpy regulation activity. Note that the stringent condition is, for example, a condition described in Reference Literature [Molecular cloning-a Laboratory manual 2nd edition (Sambrook et al., 1989)]. Specifically, the stringent condition is, for example, a condition in which the polynucleotide is hybridized by being constantly heated, at 65°C for 8 to 16 hours together with a probe, in a solution that contains 6 × SSC (composition of 1 × SSC: 0.15 M of sodium chloride, 0.015 M of sodium citrate, pH 7.0), 0.5% of SDS, 5 × Denhardt's solution, and 100 mg/mL of herring sperm DNA. Note that the polynucleotide preferably has a sequence identity of 70% or higher, more preferably has a sequence identity of 75% or higher or 80% or higher, more preferably has a sequence identity of 85% or higher, further preferably has a sequence identity of 90% or higher, particularly preferably has a sequence identity of 95% or higher, 96% or higher, 97% or higher, 98% or higher, or 99% or higher, relative to the base sequence of the polynucleotide described in the above (1).

The parthenocarpy regulatory gene of the present invention can exist in a form of RNA (e.g., mRNA) or a form of DNA (e.g., cDNA or genomic DNA). The DNA can be double-stranded or single-stranded. The base sequence represented by SEQ ID NO: 2, which is an example of the polynucleotide of the present invention, is a structural gene part of cDNA that encodes the polypeptide represented by SEQ ID NO: 1. The parthenocarpy regulatory gene of the present invention can include an additional sequence such as a sequence of an untranslated region (UTR).

A method for obtaining (isolating) the parthenocarpy regulatory gene of the present invention is not limited to a particular one, and can be a method which includes, for example, (i) preparing a probe which is specifically hybridized with a part of the base sequence of the parthenocarpy regulatory gene and (ii) screening a genomic DNA library or a cDNA library.

Alternatively, the method for obtaining the parthenocarpy regulatory gene of the present invention can be a method which uses amplifying means such as PCR. For example, a large amount of DNA fragments each containing the parthenocarpy regulatory gene of the present invention can be obtained by (i) preparing primers from respective 5' end and 3' end sequences (or complementary sequences thereof) in cDNA of the parthenocarpy regulatory gene, (ii) carrying out PCR or the like with the use of the primers and genomic DNA (or cDNA) or the like as a template, and (iii) amplifying a DNA region between the primers.

An origin of the parthenocarpy regulatory gene of the present invention is not limited to a particular one, provided that the origin is a plant. Note, however, that the origin is preferably a solanaceous plant such as tomato, eggplant, pimiento, paprika, pepper, or potato, more preferably a solanum plant such as tomato, eggplant, or potato, further preferably tomato or eggplant, particularly preferably tomato.

Note that whether or not a candidate gene of the isolated parthenocarpy regulatory gene has an intended parthenocarpy regulation activity can be evaluated by observing whether or not parthenocarpy of the original plant is induced by inhibiting expression of the candidate gene in the original plant.

The parthenocarpy regulatory gene of the present invention can be used to elucidate a mechanism of parthenocarpy of a plant.

Examples of the parthenocarpy regulatory gene of the present invention encompass a gene derived from tomato (SEQ ID NOs: 2 and 6 (ORF), and SEQ ID NO: 11 (full-length cDNA), SEQ ID NO: 16 (a gene on genomic DNA containing an intron)), a gene derived from *S*. *pimpinellifolium* (wild relative of tomato, SEQ ID NO: 18) or from *S*. *peruvianum* (wild relative of tomato, SEQ ID NO: 20), a gene derived from *S*. *tuberosum phureja* (potato, SEQ ID NO: 22), and a gene derived from *S*. *melongena* (eggplant, SEQ ID NO: 24). Moreover, the parthenocarpy regulatory gene of the present invention encompasses a gene including nucleotides which has a parthenocarpy regulation activity and has a sequence identity of 70% or higher, 75% or higher, 80% or higher, 85% or higher, 90% or higher, 95% or higher, 96% or higher, 97% or higher, 98% or higher, or 99% or higher, relative to a base sequence of the above exemplified polynucleotide.

### [2. Polypeptide as parthenocarpy regulation protein]

The polypeptide of the present invention is a product obtained by translating the parthenocarpy regulatory gene described in the part [1. Parthenocarpy regulatory gene] above and has at least an activity to regulate parthenocarpy of a plant. As above described, the polypeptide of the present invention inhibits appearance of the trait of parthenocarpy, i.e., negatively regulated.

The polypeptide of the present invention can be isolated from a natural resource or can be chemically synthesized. Specifically, the polypeptide encompasses a product obtained by purifying a substance isolated from a natural resource, a product obtained in chemical synthesis process, and a translated product obtained from a procaryotic host or a eucaryotic host (e.g., bacterial cell, yeast cell, higher plant cell, insect cell, or mammalian cell) by a recombination technique.

Specifically, the polypeptide of the present invention is a polypeptide described in any of (1) through (4) below. Note that the polypeptide is a translated product of the parthenocarpy regulatory gene. Therefore, a meaning of the wordings such as "hybridize under a stringent condition" can be understood with reference to the descriptions in [1. Parthenocarpy regulatory gene] above.
(1) A polypeptide having an amino acid sequence represented by SEQ ID NO: 1.
(2) A polypeptide (i) having a sequence identity of 70% or higher relative to the amino acid sequence represented by SEQ ID NO: 1 and (ii) having a parthenocarpy regulation activity. Note that the sequence identity is preferably 75% or higher or 80% or higher, more preferably 85% or higher, more preferably 90% or higher, further preferably 95% or higher, particularly preferably 96% or higher, 97% or higher, 98% or higher, or 99% or higher.
(3) A polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity. Note that the number of amino acids which are substituted, deleted, inserted, and/or added is preferably 1 to 72; or 1 to 58, more preferably 1 to 43, more preferably 1 to 29, further preferably 1 to 14, particularly preferably 1 to 5 or 6. (4) A polypeptide that (i) is encoded by a polynucleotide which is hybridized, under a stringent condition, with a polynucleotide that has a sequence complementary to a polynucleotide for encoding the polypeptide described in the above (1) and (ii) has a parthenocarpy regulation activity.

The polypeptide may be a polypeptide constituted by bonding of amino acids. Note, however, that the polypeptide is not limited to this and can contain a structure other than a polypeptide structure. The structure other than polypeptide can be a sugar chain, an isoprenoid group, and the like, but is not limited to these in particular.

Note that examples of the polypeptide of the present invention as a parthenocarpy regulation protein encompass a polypeptide derived from tomato (SEQ ID NO: 1), a polypeptide derived from *S*. *pimpinellifolium* (wild relative of tomato, SEQ ID NO: 19), a polypeptide derived from *S*. *peruvianum* (wild relative of tomato, SEQ ID NO: 21), a polypeptide derived from *S*. *tuberosum phureja* (potato, SEQ ID NO: 23), and a polypeptide derived from *S*. *melongena* (eggplant, SEQ ID NO: 25).

### [3. Recombinant expression vector and transformant]

The present invention also provides (i) a recombinant expression vector in which the parthenocarpy regulatory gene of the present invention is incorporated such that the parthenocarpy regulatory gene can be expressed and (ii) a transformant in which the recombinant expression vector or the parthenocarpy regulatory gene is introduced such that the recombinant expression vector or the parthenocarpy regulatory gene can be expressed.

A type of a vector for constituting the recombinant expression vector is not limited to a particular one, and a vector which can be expressed in a host cell can be selected as appropriate. That is, a promoter sequence is appropriately selected depending on a type of a host cell, and a vector, in which the promoter sequence and the parthenocarpy regulatory gene of the present invention are incorporated in, for example, a plasmid, a phagemid, a cosmid, or the like, can be used as an expression vector.

The transformant encompasses an organism, as well as a cell, a tissue, and an organ in which the recombinant expression vector or the parthenocarpy regulatory gene is introduced such that the recombinant expression vector or the parthenocarpy regulatory gene can be expressed. A species of the transformant is not limited to a particular one and can be, for example, a microorganism such as *E*. *coli*, a plant, an animal, or the like.

### [4. Method for evaluating parthenocarpy of plant]

A method of the present invention for evaluating parthenocarpy includes a step of checking (i) whether or not expression of the parthenocarpy regulatory gene is inhibited in a plant or (ii) whether or not a function of a polypeptide encoded by the parthenocarpy regulatory gene is inhibited in the plant. Here, the "parthenocarpy regulatory gene" indicates the parthenocarpy regulatory gene described in [1. Parthenocarpy regulatory gene] above, and the "polypeptide encoded by the parthenocarpy regulatory gene" indicates the polypeptide described in [2. Polypeptide as parthenocarpy regulation protein] above. Note that the inhibition of expression of the parthenocarpy regulatory gene encompasses inhibition of gene transcription and inhibition of translation into a protein.

Moreover, in the checking step, it is preferable to select, as a candidate plant whose parthenocarpy is regulated, (i) a plant in which expression of the parthenocarpy regulatory gene is inhibited or (ii) a plant in which the function of the polypeptide encoded by the parthenocarpy regulatory gene is inhibited.

A method for carrying out the checking step is not limited to a particular one and can be, for example, any of methods described in the following 1) through 3). Among these methods, the method of 1) or 2) is preferable in view of easiness in working.
1) An expression level of the parthenocarpy regulatory gene in a target plant is measured and, if necessary, compared with a standard gene expression level, and thus whether or not expression of the parthenocarpy regulatory gene is inhibited is examined. The expression level of the gene can be measured by, for example, (i) measuring an amount of a transcript with a method such as quantitative RT-PCR or quantitative real time PCR or (ii) measuring an amount of a translated product with a method such as quantitative Western blotting. The standard gene expression level indicates, for example, an expression level of the parthenocarpy regulatory gene in a conspecific plant that does not show parthenocarpy (e.g., an individual having the parthenocarpy regulatory gene as a homozygote). Note that, in a case where transcription or translation of the parthenocarpy regulatory gene is substantially completely inhibited with the use of a method such as gene disruption or RNAi, the comparison with the standard gene expression level may be unnecessary.
2) A base sequence of a parthenocarpy regulatory gene (genomic DNA, mRNA, or cDNA) of a target plant is analyzed. Then, in a case where a mutation(s) that influences a function of the polypeptide encoded by the parthenocarpy regulatory gene is found as a result of the analysis, it is determined that the function of the polypeptide is inhibited. The mutation(s) that influences the function of the polypeptide can be, for example, defect of several tens to several thousands of base pairs of nucleotides including a coding region of the parthenocarpy regulatory gene, preferably defect of at least 100 bp to 1100 bp of nucleotides, or defect of at least approximately 270 bp to 1100 bp nucleotides. Note that such a mutation(s) may occur in at least one of two parthenocarpy regulatory genes (i.e., a pair of parthenocarpy regulatory genes) in a homologous chromosome, and it is preferable that the mutation(s) occurs in both the two parthenocarpy regulatory genes (i.e., recessive homozygote).

Moreover, in a case where a marker sequence that shows a linkage with the mutation(s) on the parthenocarpy regulatory gene is utilized, the base sequence of the parthenocarpy regulatory gene is to be directly or indirectly checked, and it is thus possible to carry out the checking step. The marker sequence is, for example, located within approximately 5.5 cM (i.e., approximately 5.5 cM to 0 cM), preferably located within approximately 5.3 cM, more preferably located within approximately 1.2 cM, further preferably located within approximately 1.1 cM, from a location of the parthenocarpy regulatory gene. Note that it is of course possible to use the mutation(s) on the parthenocarpy regulatory gene as a marker sequence.
3) A polypeptide encoded by the parthenocarpy regulatory gene is isolated from a target plant, and an amino acid sequence of the polypeptide is analyzed. Then, in a case where a mutation(s) (e.g., generation of a truncated body that causes deletion of several tens or more of amino acids), which influences a function of the polypeptide, is found as a result of the analysis, it is determined that the function of the polypeptide is inhibited.

A type of a plant, to which the evaluating method of the present invention is applied, is not limited to a particular one, and is preferably solanaceous plants such as tomato, eggplant, pimiento, paprika, pepper, and potato, more preferably solanum plants such as tomato, eggplant, and potato, further preferably tomato or eggplant, particularly preferably tomato. Moreover, examples of a plant to which the evaluating method is applied encompass a breeding material (parental plant, i.e., pollen parent or seed parent) and offspring obtained by breeding. Note that the term "breeding" indicates a concept that encompasses (i) a method utilizing crossing and (ii) a genetic engineering method.

The phenotypic expression of parthenocarpy sometimes becomes unstable depending on a cultivation condition or a cultivar or line and therefore it is often difficult to evaluate based only on appearance of a plant. Moreover, a plant in which the parthenocarpy regulatory gene is heterozygous is worth using as a breeding material or the like. In particular, when the parthenocarpy regulatory gene is heterozygous, it is sometimes impossible to clearly only from appearance of fruit phenotype whether or not the parthenocarpy can be shown. The method of the present invention for evaluating the parthenocarpy is carried out at a gene level, and therefore trait evaluation can be carried out without making genes homozygous. For example, this makes it possible to significantly improve efficiency in breeding selection. Further, the parthenocarpy regulatory gene has been identified, and it is therefore possible to segregate, with the use of a gene engineering techniques or the like, linkage with an unfavorable trait which has naturally cosegregated with the parthenocarpy regulatory gene.

### [5. Method for producing parthenocarpy-regulated plant, and produced plant]

A form (hereinafter, referred to as "form 1") of a method of the present invention for producing a parthenocarpy-regulated plant is a method that includes a step of selecting a parthenocarpy-regulated plant by performing the evaluating method described in [4. Method for evaluating parthenocarpy of plant] above.

Alternatively, another form (hereinafter, referred to as "form 2") of the method of the present invention for producing a parthenocarpy-regulated plant is a method that includes a step of inhibiting expression of the parthenocarpy regulatory gene in the plant or a step of inhibiting a function of a polypeptide encoded by the parthenocarpy regulatory gene in the plant. Here, the "parthenocarpy regulatory gene" is the one described in [1. Parthenocarpy regulatory gene] above, and the "polypeptide encoded by the parthenocarpy regulatory gene" is the one described in [2. Polypeptide as parthenocarpy regulation protein] above.

Note that, in the form 2, it is preferable to inhibit expression of the parthenocarpy regulatory gene by disrupting the parthenocarpy regulatory gene or introducing, to the plant, a polynucleotide that inhibits expression of the parthenocarpy regulatory gene. Note that the wording "inhibit expression of the parthenocarpy regulatory gene" encompasses (i) inhibition of transcription of a gene and (ii) inhibition of translation into protein.

A method for disrupting the parthenocarpy regulatory gene is not limited to a particular one. For example, it can be performed (i) by a specific gene disruption using homologous recombination, (ii) by introducing a termination codon to the parthenocarpy regulatory gene using site-directed mutagenesis, and (iii) by a partial physical gene disruption using irradiation of a heavy ion beam or the like.

Moreover, the method for introducing, to the plant, a polynucleotide that inhibits expression of the parthenocarpy regulatory gene is not limited to a particular one and can be, for example, a method such as RNA interference or antisense RNA. A method for introducing, to the plant, an expression cassette (e.g., vector) containing the polynucleotide is not limited to a particular one, and it is possible to employ, as appropriate, a method using polyethyleneglycol, electroporation, a method via Agrobacterium, or a method using a particle gun. Note that the expression cassette containing the polynucleotide can be introduced to a plant cell, a callus, a plant tissue, or a plant individual.

Note that expression of the parthenocarpy regulatory gene can be inhibited by introducing a mutation(s), which inhibits gene expression, to an expression adjusting sequence (e.g., a promoter sequence or an enhancer sequence) that regulates expression of the parthenocarpy regulatory gene.

The present invention also provides a parthenocarpy-regulated plant, which has been produced by the above described production method. The term "parthenocarpy-regulated plant" indicates a concept that encompasses (i) a plant in which the trait of parthenocarpy is shown and (ii) a plant in which the trait of parthenocarpy is not shown but which has a predisposition of the parthenocarpy.

A type of a plant which is to be produced by the method of the present invention is not limited to a particular one and is preferably solanaceous plants such as tomato, eggplant, pimiento, paprika, pepper, and potato, more preferably solanum plants such as tomato, eggplant, and potato, further preferably tomato or eggplant, particularly preferably tomato.

### [6. Example of concrete aspect of present invention]

That is, the present invention encompasses any of the following 1) through 11) :
1) A method for evaluating parthenocarpy of a plant, the method including the step of: checking whether or not expression of a parthenocarpy regulatory gene, which includes a polynucleotide described in any of (1) through (4) below, is inhibited in the plant; or checking whether or not a function of a polypeptide, which is encoded by the parthenocarpy regulatory gene, is inhibited in the plant. According to a form of the checking step, for example, it is checked, in at least a bud of the plant, (i) whether or not expression of the parthenocarpy regulatory gene is inhibited or (ii) whether or not the function of the polypeptide encoded by the parthenocarpy regulatory gene is inhibited.
(1) A polynucleotide that encodes a polypeptide having an amino acid sequence represented by SEQ ID NO: 1, (2) a polynucleotide that encodes a polypeptide (i) having a sequence identity of 70% or higher relative to the amino acid sequence represented by SEQ ID NO: 1 and (ii) having a parthenocarpy regulation activity, (3) a polynucleotide that encodes a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity, and (4) a polynucleotide that (i) is hybridized with a polynucleotide, which has a sequence complementary to the polynucleotide described in the above (1), under a stringent condition and (ii) encodes a polypeptide having a parthenocarpy regulation activity.
2) The method described in the above 1) in which, in the step of checking, a base sequence of the parthenocarpy regulatory gene or an expression level of the parthenocarpy regulatory gene is checked.
3) The method described in the above 1) or 2) in which, in the step of checking, a plant in which the expression of the parthenocarpy regulatory gene is inhibited or a plant in which the function of the polypeptide encoded by the parthenocarpy regulatory gene is inhibited is selected as a parthenocarpic plant.
4) A method for producing a parthenocarpy-regulated plant, the method including the step of selecting a parthenocarpic plant by carrying out the method described in any one of the above 1) through 3).
5) A method for producing a parthenocarpy-regulated plant, the method including the step of: inhibiting, in a plant, gene expression of a parthenocarpy regulatory gene that includes a polynucleotide described in any of (1) through (4) below; or inhibiting, in a plant, a function of a polypeptide encoded by the parthenocarpy regulatory gene. According to an aspect of the step, for example, in at least a bud of the plant, gene expression of the parthenocarpy regulatory gene is inhibited or the function of the polypeptide encoded by the parthenocarpy regulatory gene is inhibited.
(1) A polynucleotide that encodes a polypeptide having an amino acid sequence represented by SEQ ID NO: 1, (2) a polynucleotide that encodes a polypeptide (i) having a sequence identity of 70% or higher relative to the amino acid sequence represented by SEQ ID NO: 1 and (ii) having a parthenocarpy regulation activity, (3) a polynucleotide that encodes a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity, and (4) a polynucleotide that (i) is hybridized with a polynucleotide, which has a sequence complementary to the polynucleotide recited in the above (1), under a stringent condition and (ii) encodes a polypeptide having a parthenocarpy regulation activity.
6) The method described in the above 5) in which, in the step, (i) a polynucleotide, which inhibits the gene expression of the parthenocarpy regulatory gene, is introduced into the plant or (ii) the parthenocarpy regulatory gene is disrupted.
7) The method described in any of the above 1) through 6) in which the plant is a solanaceous plant.
8) The method described in the above 7) in which the plant is a tomato.
9) A parthenocarpy-regulated plant produced by the method described in any of the above 4) through 6).
10) A parthenocarpy regulatory gene that includes a polynucleotide described in any of (1) through (4) below. According to a form of the parthenocarpy regulatory gene, for example, the plant shows parthenocarpy by inhibiting gene expression of the parthenocarpy regulatory gene in at least a bud of the plant. (1) A polynucleotide that encodes a polypeptide having an amino acid sequence represented by SEQ ID NO: 1, (2) a polynucleotide that encodes a polypeptide (i) having a sequence identity of 70% or higher relative to the amino acid sequence represented by SEQ ID NO: 1 and (ii) having a parthenocarpy regulation activity, (3) a polynucleotide that encodes a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity, and (4) a polynucleotide that (i) is hybridized with a polynucleotide, which has a sequence complementary to the polynucleotide described in the above (1), under a stringent condition and (ii) encodes a polypeptide having a parthenocarpy regulation activity.
11) A polypeptide described in any of (1) through (4) below. According to a form of the polypeptide, for example, the plant shows parthenocarpy by inhibiting a function of the polypeptide in at least a bud of the plant.
(1) A polypeptide having an amino acid sequence represented by SEQ ID NO: 1, (2) a polypeptide (i) having a sequence identity of 70% or higher relative to the amino acid sequence represented by SEQ ID NO: 1 and (ii) having a parthenocarpy regulation activity, (3) a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity, and (4) a polypeptide that (i) is encoded by a polynucleotide which is hybridized, under a stringent condition, with a polynucleotide that has a sequence complementary to a polynucleotide for encoding the polypeptide described in the above (1) and (ii) has a parthenocarpy regulation activity.

According to another aspect, the present invention can be any of the following 12) through 17):
12) A method for evaluating parthenocarpy of a plant, the method including the step of: checking whether or not gene expression of a parthenocarpy regulatory gene, which includes a polynucleotide described in any of (S1) through (S4) below, is inhibited in the plant; or checking whether or not a function of a polypeptide, which is encoded by the parthenocarpy regulatory gene, is inhibited in the plant. According to a form of the checking step, for example, it is checked, in at least a bud of the plant, (i) whether or not gene expression of the parthenocarpy regulatory gene is inhibited or (ii) whether or not the function of the polypeptide encoded by the parthenocarpy regulatory gene is inhibited.
(S1) A polynucleotide that encodes a polypeptide having an amino acid sequence represented by SEQ ID NO: 25, (S2) a polynucleotide that encodes a polypeptide (i) having a sequence identity of 70% or higher relative to the amino acid sequence represented by SEQ ID NO: 25 and (ii) having a parthenocarpy regulation activity, (S3) a polynucleotide that encodes a polypeptide (i) having an amino acid sequence in which 1 to 87 amino acids are substituted in, deleted from, inserted into, and/or added to the amino acid sequence represented by SEQ ID NO: 25 and (ii) having a parthenocarpy regulation activity, and (S4) a polynucleotide that (i) is hybridized with a polynucleotide, which has a sequence complementary to the polynucleotide described in the above (S1) under a stringent condition and (ii) encodes a polypeptide having a parthenocarpy regulation activity.
13) A method for producing a parthenocarpy-regulated plant, the method including the step of selecting a parthenocarpic plant by carrying out the method described in the above 12).
14) A method for producing a parthenocarpy-regulated plant, the method including the step of: inhibiting, in a plant, gene expression of a parthenocarpy regulatory gene that includes a polynucleotide described in any of (S1) through (S4) below; or inhibiting, in a plant, a function of a polypeptide encoded by the parthenocarpy regulatory gene. According to a form of the step, for example, in at least a bud of the plant, gene expression of the parthenocarpy regulatory gene is inhibited or the function of the polypeptide encoded by the parthenocarpy regulatory gene is inhibited.
(S1) A polynucleotide that encodes a polypeptide having an amino acid sequence represented by SEQ ID NO: 25, (S2) a polynucleotide that encodes a polypeptide (i) having a sequence identity of 70% or higher relative to the amino acid sequence represented by SEQ ID NO: 25 and (ii) having a parthenocarpy regulation activity, (S3) a polynucleotide that encodes a polypeptide (i) having an amino acid sequence in which 1 to 87 amino acids are substituted in, deleted from, inserted into, and/or added to the amino acid sequence represented by SEQ ID NO: 25 and (ii) having a parthenocarpy regulation activity, and (S4) a polynucleotide that (i) is hybridized with a polynucleotide, which has a sequence complementary to the polynucleotide described in the above (S1) under a stringent condition and (ii) encodes a polypeptide having a parthenocarpy regulation activity.
15) A parthenocarpy-regulated plant produced by the method described in the above 13) or 14).
16) A parthenocarpy regulatory gene that includes a polynucleotide described in any of (S1) through (S4) below. According to a form of the parthenocarpy regulatory gene, for example, the plant shows parthenocarpy by inhibiting gene expression of the parthenocarpy regulatory gene in at least a bud of the plant. (S1) A polynucleotide that encodes a polypeptide having an amino acid sequence represented by SEQ ID NO: 25, (S2) a polynucleotide that encodes a polypeptide (i) having a sequence identity of 70% or higher relative to the amino acid sequence represented by SEQ ID NO: 25 and (ii) having a parthenocarpy regulation activity, (S3) a polynucleotide that encodes a polypeptide (i) having an amino acid sequence in which 1 to 87 amino acids are substituted in, deleted from, inserted into, and/or added to the amino acid sequence represented by SEQ ID NO: 25 and (ii) having a parthenocarpy regulation activity, and (S4) a polynucleotide that (i) is hybridized with a polynucleotide, which has a sequence complementary to the polynucleotide described in the above (S1) under a stringent condition and (ii) encodes a polypeptide having a parthenocarpy regulation activity.
17) A polypeptide described in any of (S1) through (S4) below. According to a form of the polypeptide, for example, the plant shows parthenocarpy by inhibiting a function of the polypeptide in at least a bud of the plant.
(S1) A polypeptide having an amino acid sequence represented by SEQ ID NO: 25, (S2) a polypeptide (i) having a sequence identity of 70% or higher relative to the amino acid sequence represented by SEQ ID NO: 25 and (ii) having a parthenocarpy regulation activity, (S3) a polypeptide (i) having an amino acid sequence in which 1 to 87 amino acids are substituted in, deleted from, inserted into, and/or added to the amino acid sequence represented by SEQ ID NO: 25 and (ii) having a parthenocarpy regulation activity, (S4) a polypeptide that (i) is encoded by a polynucleotide which is hybridized, under a stringent condition, with a polynucleotide that has a sequence complementary to a polynucleotide for encoding the polypeptide described in the above (S1) and (ii) has a parthenocarpy regulation activity.

Note that, in the above 12) through 17), the sequence identity of the amino acid sequence is preferably 75% or higher or 80% or higher, more preferably 85% or higher, more preferably 90% or higher, further preferably 95% or higher, particularly preferably 96% or higher, 97% or higher, 98% or higher, or 99% or higher.

The present invention is not limited to the embodiments, but can be altered by a skilled person in the art within the scope of the claims. That is, an embodiment derived from a proper combination of technical means appropriately modified within the scope of the claims is also encompassed in the technical scope of the present invention.

### [Examples]

### [1. Identification of tomato-derived parthenocarpy responsible gene A]

### [Breeding of genetic analysis population]

By crossing a parthenocarpic tomato line LS935 (obtained from National Institute of Vegetable and Tea Science) and a non-parthenocarpic tomato line Saladette (LA2662) (obtained from Tomato Genetics Resource Center in US), a population of F₂ individuals was obtained. With the use of the F₂ population, a tomato linkage map in which mapped parthenocarpy responsible gene A was constructed and genetic analysis of parthenocarpy was carried out as described below. Further, a selfed progeny F₃ population, which was a line selected from the F₂ population, was used in the detailed genetic analysis of parthenocarpy described below.

Note that the tomato line LS935 is a genetic resource obtained by introducing, as a backcrossed line, Moneymaker (which is a large fruit tomato cultivar) into Severianin which is a cultivar that reportedly shows parthenocarpy. Saladette is a genetic resource introduced as a large fruit tomato line and shows non-parthenocarpy (i.e., does not show parthenocarpy).

### [Method for evaluating parthenocarpy]

A stigma was removed from flower before anthesis, and mature fruit weight of stigma-removed fruit was measured, and presence or absence of a seed in stigma-removed fruit was confirmed. A weight of each of the mature stigma-removed fruits, which had 3 or more flower clusters per plant, was measured, and a weight of each of mature open-pollinated fruits, which had 1 or more flower cluster(s) per plant, was measured. The weights of the stigma-removed fruits were compared with the weights of the open-pollinated fruits, and a line, from which a mature fruit having a fruit weight similar to that of the open-pollinated fruits was obtained, was determined to have parthenocarpy. Note that, with regard to lines other than the RNAi transgenic tomato, it is difficult to evaluate parthenocarpy when the open-pollinated fruit is small and a difference from the stigma-removed fruit is small. In such a case, parthenocarpy was evaluated again using propagated seedlings.

### [Construction of linkage map]

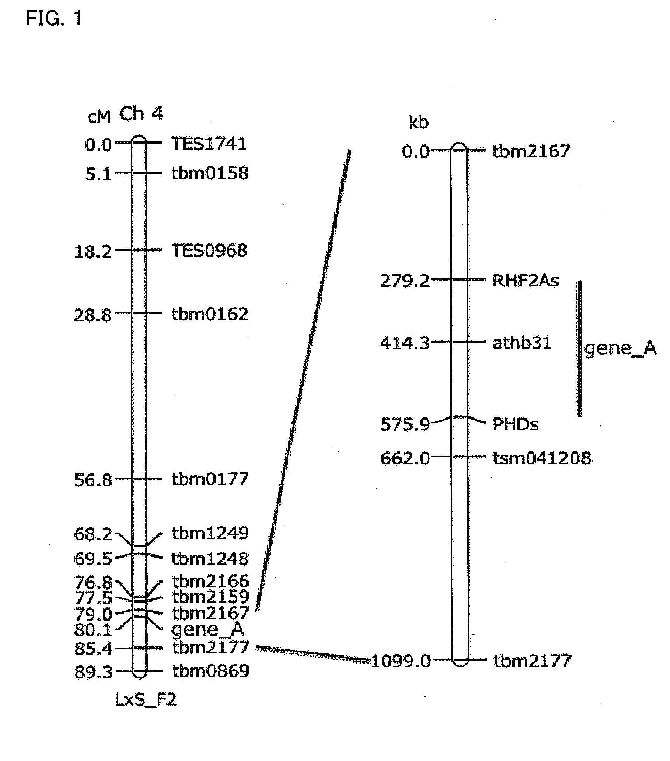

With the use of reported SSR (simple sequence repeat) markers (Reference Literature: Ohyama A. et al. (2009) Mol. Breed. 23: 685-691, Shirasawa L. et al. (2010) Theor. Appl. Genet. 121: 731-739.) and markers (which is being developed by the applicant) of SSR in the neighboring of reported SNP markers, a linkage map illustrated in Fig. 1 was constructed, and parthenocarpy/non-parthenocarpy traits were evaluated using F₂ population including 100 individuals and mapped on the linkage map. In the construction of the linkage map, Mapmaker exp 3.0 was used. Note that a left part of Fig. 1 is a linkage map of chromosome 4 of tomato. In Fig. 1, names of markers that begin with "TES" indicate reported SSR markers, and names of markers that begin with "tbm" indicate markers originally developed by the applicant.

### [Detailed genetic analysis of parthenocarpy]

From the genetic analysis with the use of the F₂ population, it has been found that the parthenocarpy responsible gene A is located at chromosome 4 of tomato and is located in a region of approximately 5 cM between two SSR markers (see Fig. 1). As a result of comparative analysis between nucleotide sequence of the SSR markers and tomato genome sequence (sol genomics network http://solgenomics.net/), a physical distance between the two SSR markers corresponded to approximately 1100 kb.

Next, with the use of the tomato genome sequence, several SSRs of the region in which the parthenocarpy responsible gene A is located were newly extracted and polymorphism was searched. Moreover, SNP polymorphisms in the neighboring of a candidate gene that was located in the region were newly searched. Some F₂ individuals, whose genotype in the region was heterozygous, were selected from the F₂ population, and genotype of progeny F₃ individuals was determined. Thus, genetic recombinated individuals were selected, and parthenocarpy of the individual was evaluated. As a result of the analysis, a locus of the parthenocarpy responsible gene A was narrowed down to approximately 300 kb region between two SNP markers. Note that genetic linkage markers located on neighboring of approximately 300 kb region are shown in a right part of Fig. 1. RHF2As and PHDs are newly developed SNP markers, tsm041208 is a newly developed SSR marker, and athb31 is a newly developed insertion-deletion mutation marker.

For the non-parthenocarpic line "Saladette" and the parthenocarpic line "LS935", a base sequence of the candidate region of approximately 300 kb was determined, and polymorphism between two lines was searched. As a result, by comparing with the genomic sequence (SEQ ID NO: 3) of "Saladette", a deletion mutation (SEQ ID NO: 5) of 1034 bp was found in the genomic sequence (SEQ ID NO: 4) of "LS935".

The deletion mutation existed on a putative gene that has open reading frame as represented by SEQ ID NO: 6, and it was estimated that a part of exon 1 and a part of intron 1 of the putative gene were lost from a genome of "LS935". Under the circumstances, it was predicted that the deletion mutation of the putative gene that had the open reading frame represented by SEQ ID NO: 6 induced parthenocarpy, and the following experiment was carried out. Note that a base sequence (including an intron) on the genome of the putative gene of "Saladette" is represented by SEQ ID NO: 16, and a corresponding base sequence of the genome of "LS935" is represented by SEQ ID NO: 17.

### [Obtain full-length cDNA sequence of parthenocarpy responsible gene A]

From aboveground tissues including leaves, stems, and flowers of a non-parthenocarpic tomato cultivar "Shugyoku", total RNA was extracted by a Trizol method, and cDNA was synthesized with the use of SMARTer RACE cDNA amplification Kit (manufactured by Takara Bio Inc.). By using the cDNA as a template, amplification of cDNA including a transcription start point and a translation start point of mRNA was attempted with the use of a 5'-RACE method in which the polynucleotide represented by SEQ ID NO: 7 was used as a gene-specific primer. An amplified product was cloned into an *E*. *coli* vector, and nucleotide sequence thereof was determined by Sanger's method. As a result, a sequence of 842 bp at a 5' end (including exon 1 (i.e., 298 bp of 5'-UTR (untranslated region) and 541 bp of 5' end of ORF) and 3 bp of exon 2) of full-length cDNA was obtained.

Similarly, with a 3'-RACE method in which the polynucleotide represented by SEQ ID NO: 8 was used as a gene-specific primer, (i) amplification of cDNA including a translation end point and a polyadenylation region, (ii) cloning to an *E*. *coli* vector, and (iii) determination of a nucleotide sequence were attempted. As a result, a sequence of 539 bp (including 305 bp of exon 2 (i.e., 3' end of ORF (including a translation end point)) and 234 bp of 3'-UTR) at a 3' end of full-length cDNA was obtained.

Further, with the use of a pair of primers, i.e., a primer represented by SEQ ID NO: 9 and a primer represented by SEQ ID NO: 10, cDNA was amplified by PCR which was carried out using, as a template, cDNA identical with the above described one. Then, nucleotide sequence of the cDNA thus amplified was determined by direct sequencing. Then, a base sequence (represented by SEQ ID NO: 11) of 1405 bp was obtained by combining the 5' end region and the 3' end region (which were obtained above) and the other regions of the full-length cDNA based on overlaps of sequences, and thus the base sequence represented by SEQ ID NO: 11 was obtained as a full-length cDNA sequence of the parthenocarpy responsible gene A.

### [Analysis of amino acid sequence of parthenocarpy responsible gene A and search for homologue gene]

As a result of blast search (http://blast.ncbi.nlm.nih.gov/), it was found that the parthenocarpy responsible gene A belongs to a Zn finger homeodomain type homeobox gene in which (i) a part from 56th valine to 112th asparagine in an amino acid sequence to be encoded was predicted to be a Zn finger domain and (ii) a part from 219th lysine to 276th asparagine in the amino acid sequence to be encoded was predicted to be a homeo domain. The Zn finger homeodomain type homeobox gene is predicted to regulate morphogenesis as a transcription factor. Moreover, homologs of the parthenocarpic gene A were found in databases, that is, an S. *pimpinellifolium* (wild relative of tomato)-derived homolog, an S. *peruvianum* (wild relative of tomato)-derived homolog, an S. *tuberosum phureja* (potato)-derived homolog, and an S. *melongena* (eggplant)-derived homolog were found. Homologies of amino acid sequences of the respective homologs relative to the amino acid sequence which is encoded by the parthenocarpic gene A were as follows: that is, a homology of the *S. pimpinellifolium* (wild relative of tomato)-derived homologue (SEQ ID NO: 19) was 95%, a homology of the S. *peruvianum* (wild relative of tomato)-derived homologue (SEQ ID NO: 21) was 94%, a homology of the *S. tuberosum phureja* (potato)-derived homologue (SEQ ID NO: 23) was 84%, and a homology of the *S. melongena* (eggplant)-derived homologue (SEQ ID NO: 25) was 75%. Note that the database used to search the homolog gene of eggplant was prepared by the inventors on their own and is not yet open to the public.

### [Isolation of promoter region of parthenocarpy responsible gene A]

The full-length cDNA sequence of the parthenocarpy responsible gene A represented by SEQ ID NO: 11 corresponds to a part from nucleotide number 9421 to nucleotide number 11603 in the genomic sequence represented by SEQ ID NO: 3. Therefore, an upstream genomic sequence thereof is presumed to be a promoter region that relates to control of expression of the parthenocarpy responsible gene A. Under the circumstances, PCR primers for amplifying the region was synthesized, and PCR was carried out with the use of the PCR primers and genomic DNA of a tomato cultivar "Momotaro 8" as a template. An obtained PCR product was digested by restricted enzymes *Hin*dIII and *Sma*I*,* and cloned by being inserted into a *Hin*dIII*-Sma*I site of a cloning vector pHSG398.

Then, a nucleotide sequence of an obtained clone was confirmed by the Sanger's method and was compared with the sequence represented by SEQ ID NO: 3. Thus, a clone was selected which did not contain a mutation(s) caused by the PCR amplification. The clone thus obtained had 2572 bp and had a genome-derived *Hin*dIII recognition site at a 5' end, and a PCR primer-derived *Sma*I recognition site at a 3' end. Specifically, the clone included (i) 2239 bp in a part upstream to a transcription start point of the parthenocarpy responsible gene A, (ii) 327 bp from the transcription start point to a part of a protein coding region via 5'-UTR and a translation start point, and (iii) 6 bp of the PCR primer-derived SmaI recognition site. This DNA fragment (SEQ ID NO: 12) was used in the following experiment as a promoter of the parthenocarpy responsible gene A.

### [Construction of RNA interference (RNAi) induction vector]

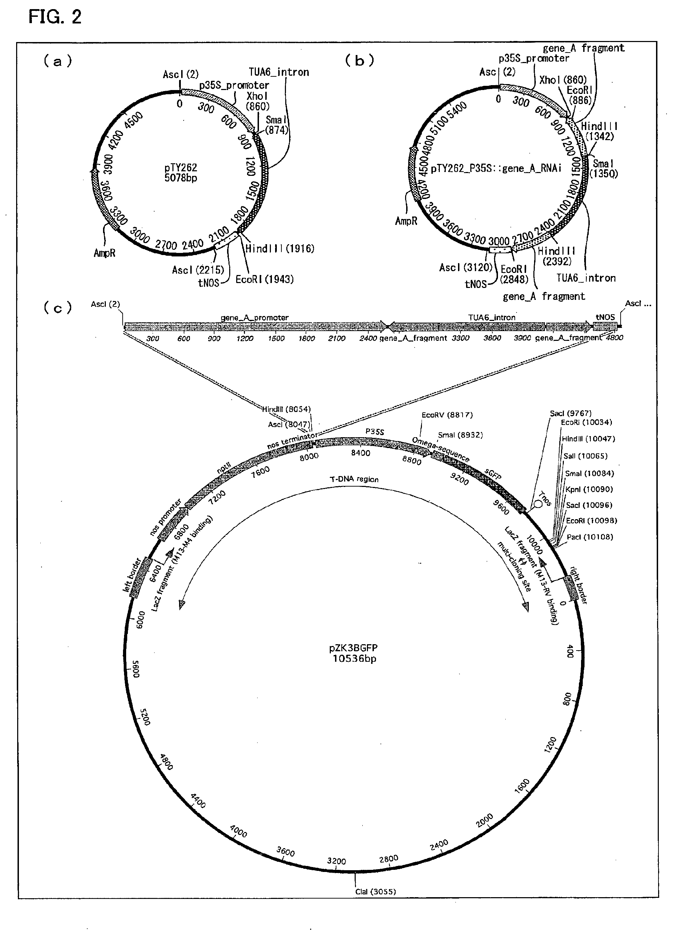

With the use of primers respectively represented by SEQ ID NO: 13 and SEQ ID NO: 14, PCR was carried out using cDNA of the tomato cultivar "Shugyoku" as a template, and thus 500 bp of DNA fragment represented by SEQ ID NO: 15 was obtained. The fragment had a *Sma*I recognition site and a *Hin*dIII recognition site at a 5' end, and an EcoRI recognition site and an *Xho*I recognition site at a 3' end. The fragment was inserted into a cloning vector pTY262 (AB736152 (DDBJ)), and RNAi induction chimeric gene with respect to the parthenocarpy responsible gene A was constructed. The pTY262 vector had an expression cassette including a CaMV35S promoter and a Nos terminator. In the pTY262 vector, cloning sites of exogenous sequences are arranged so that intron 1 of a tomato tubulin gene is located between the cloning sites of exogenous sequences (see (a) of Fig. 2).

First, the above described 500 bp fragment (SEQ ID NO: 15) was digested by *Hin*dIII and *Eco*RI and inserted ahead of the Nos terminator (tNos). Then, the above described 500 bp fragment was digested by *Sma*I and *Xho*I and inserted behind the CaMV35S promoter of the obtained vector. An RNAi induction chimeric gene thus obtained had a structure in which the substantially entire 500 bp fragment represented by SEQ ID NO: 15 was arranged in an inverted repeat sequence into which the intron 1 of the tomato tubulin gene was inserted. Expression of the RNAi induction chimeric gene was induced by the CaMV35S promoter which induces constitutive expression of general plants (see (b) of Fig. 2). Further, an RNAi induction chimeric gene was constructed by replacing the CaMV35S promoter with a promoter of the parthenocarpy responsible gene A represented by SEQ ID NO: 12. Next, a clone having the RNAi induction chimeric gene thus constructed was digested by *Asc*I*,* and pZK3BGFP_geneA-RNAi (see (c) of Fig. 2) was constructed by inserting a fragment including the obtained RNAi induction chimeric gene into an *Asc*I recognition site of a modified binary vector pZK3BGFP in which a reporter gene including a CaMV35S promoter, GFP, and a Nos terminator was inserted into a binary vector pZK3B (Reference Literature: Kuroda et al., Biosci Biotech Biochem, (2010) 74: 2348-2351).

### [Production of RNAi transgenic tomato]

Transformation of tomato was carried out with the use of the RNAi induction chimeric gene (vector) illustrated in (c) of Fig. 2. Specifically, with the use of an Agrobacterium method (Sun et al. 2006 Plant Cell Physiol 47: 426-431), cotyledon of the tomato cultivar "Shugyoku" was infected with Agrobacterium having the binary vector, and cultured on kanamycin-containing medium. Then, selection was carried out and thus regenerated plant was obtained. Insertion of the gene in the regenerated plant was confirmed by PCR, and RNAi transgenic tomato was obtained. Note that it was confirmed in the RNAi transgenic tomato that expression of the parthenocarpy responsible gene A was significantly inhibited.

### [Study of parthenocarpy of RNAi transgenic tomato]

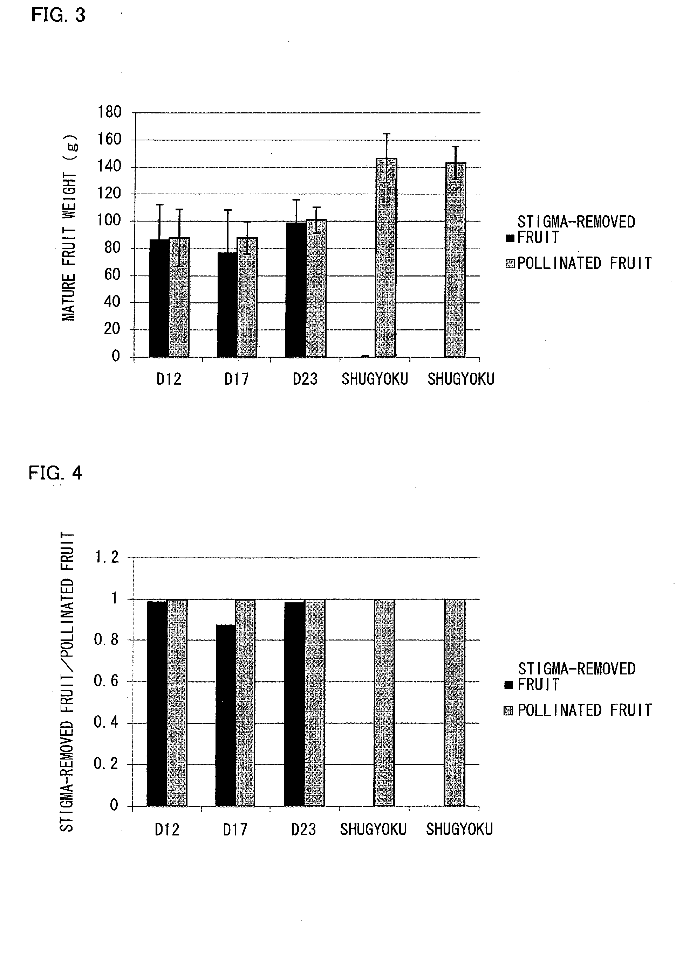

RNAi transgenic tomatoes were cultivated, and mature fruit weights of stigma-removed fruits, whose stigma had been removed before anthesis, were measured and mature fruit weights of pollinated fruits, which had been pollinated at anthesis, were measured. Moreover, non-transgenic plants were concurrently cultivated, and mature fruit weights of the non-transgenic plants were measured as controls through similar processes. Results are shown in Figs. 3 and 4.

As shown in Fig. 3, the stigma-removed fruit of the non-transgenic tomato cultivar "Shugyoku" was not fructified and enlarged, whereas the stigma-removed fruit of the RNAi transgenic tomato was fructified and enlarged to a size similar to that of the pollinated fruit. Moreover, as shown in Fig. 4, a ratio of the fruit weight of the stigma-removed fruit of the non-transgenic tomato (i.e., the control) was nearly 0 relative to the fruit weight of the pollinated fruit which was assumed to be 1. On the other hand, a ratio of the fruit weight of the RNAi transgenic tomato was not significantly different from the ratio of the fruit weight of the pollinated fruit.

From the above result, it has been concluded that inhibition of the function (i.e., deletion mutation) of the parthenocarpy responsible gene A induces parthenocarpy.

### [Analysis of expression of parthenocarpy responsible gene A]

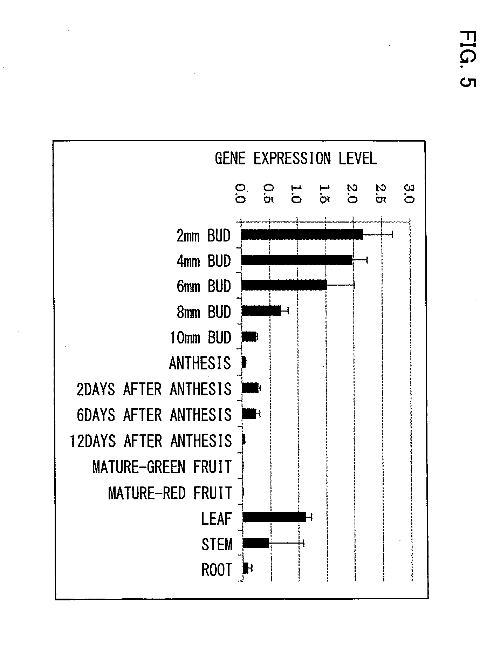

Stage-specific and tissue-specific expressions of the parthenocarpy responsible gene A were analyzed in detail by quantitative real time PCR with the use of a fluorescent real time PCR device (LC480, manufactured by Roche Diagnostics) and a LightCycler 480 SYBR Green master kit (manufactured by Roche Diagnostics). A tomato cultivar Moneymaker was used, and checked tissues are indicated in Fig. 5. Note that, in Fig. 5, "anthesis" indicates gene expression level of flowering, each of "mature-green fruit" and "mature-red fruit" indicates a gene expression level of a fruit. For amplification of the parthenocarpy responsible gene A, primers were used whose base sequences are represented by SEQ ID NO: 26 and SEQ ID NO: 27, respectively. As an endogenous standard gene for the quantitative real time PCR, a housekeeping gene Solyc04g049180.2.1 (ITAG2.30, SEQ ID NO: 28) was used which had been developed by the inventors and is constitutively-expressed in tomato tissues. For PCR amplification of the housekeeping gene, primers were used which had base sequences represented by SEQ ID NO: 29 and SEQ ID NO: 30, respectively.

As a result, it has been found that gene expression of the parthenocarpy responsible gene A shows at least 6 characteristics below (see also Fig. 5):
1. In all used tissues and stages of growth, gene expression level of the parthenocarpy responsible gene A is highest among immature buds with a total length of 2 mm.
2. The gene expression level of the parthenocarpy responsible gene A decreases as the bud grows, and decreases to 1/20 or less at anthesis.
3. Slight increase in gene expression of the parthenocarpy responsible gene A is seen during a period between the second day and the sixth day after the anthesis, but the increase level of gene expression of the gene is approximately 1/5 or less of that of the immature bud with a total length of 2 mm.
4. The expression of the parthenocarpy responsible gene A is approximately 0 in the mature-green fruit and the mature-red fruit.
5. Constant level of gene expression of the parthenocarpy responsible gene A is seen in the leaves and the stems but the gene expression level of the parthenocarpy responsible gene A is approximately 1/2 or less of that of the immature bud.
6. Gene expression of the parthenocarpy responsible gene A in roots is hardly seen.

### [Construction of for parthenocarpy responsible gene A expression vector]

With the use of primers having base sequences which are represented by SEQ ID NO: 31 and SEQ ID NO: 32, respectively, PCR amplification was carried out while using genomic DNA of the tomato cultivar "Shugyoku" as a template, and thus a gene fragment was obtained which had a base sequence represented by SEQ ID NO: 33. The gene fragment thus obtained corresponds to a part from the nucleotide number 7150 to the nucleotide number 12098 in the genomic sequence which is the base sequence represented by SEQ ID NO: 3. The gene fragment had primer-derived AscI recognition sites at its 5' end and 3' end. The gene fragment included (i) 2271 bp in a part upstream to a transcription start point of the parthenocarpy responsible gene A, (ii) 780 bp of exon 1 from the transcription start point to an intron region via 5'-UTR and a translation start point, (iii) 778 bp of an intron, (iv) 566 bp of exon 2 from immediately behind the intron to a polyA added location via a translation termination codon, and (v) 495 bp of a genomic sequence in a part downstream to the exon 2.

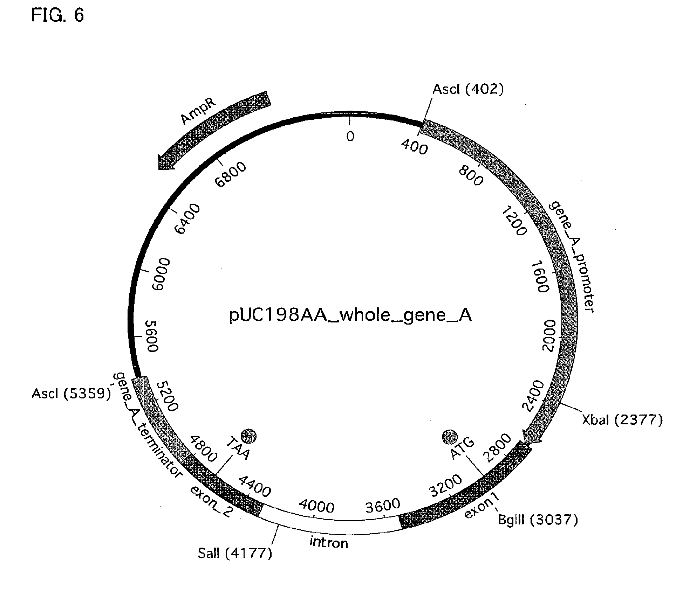

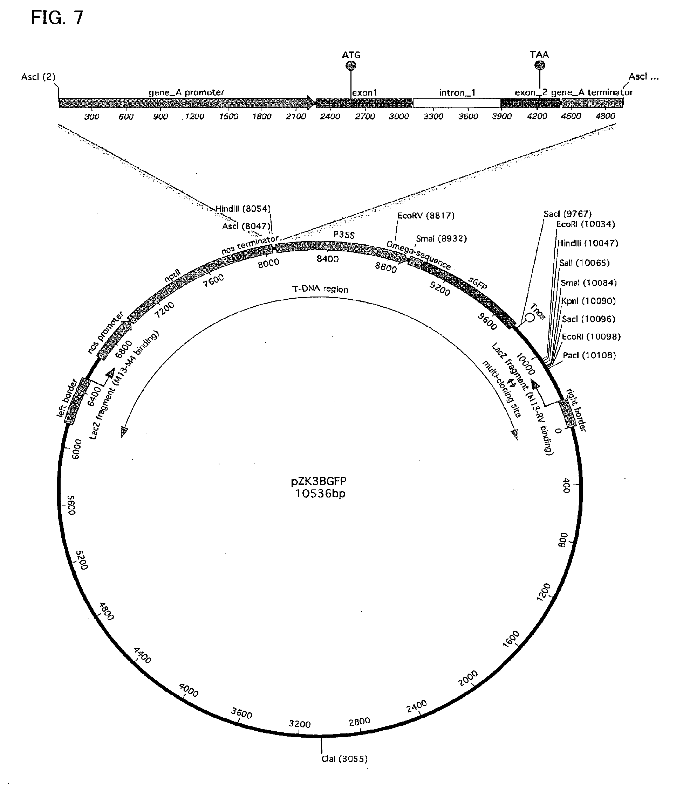

The gene fragment, which had been amplified, was digested by *Asc*I and was then inserted into an AscI recognition site of a cloning vector pUC198AA (Reference Literature: Kuroda et al., Biosci Biotech Biochem, (2010) 74: 2348-2351) for obtaining a clone (i.e., pUC 198AA_whole_gene_A in Fig. 6). A base sequence of the clone thus obtained was determined by the Sanger's method, and the base sequence was compared with the base sequence represented by SEQ ID NO: 3. Thus, a clone was selected which did not include a mutation(s) caused in PCR amplification. The clone thus selected was digested by AscI, and a binary vector pZK3BGFP_whole_Gene_A (see Fig. 7) was constructed by inserting a fragment thus obtained by digestion into an AscI recognition site of a modified binary vector pZK3BGFP in which a reporter gene including CaMV35S promoter, GFP, and a Nos terminator was inserted into a binary vector pZK3B (Kuroda et al., Biosci Biotech Biochem, (2010) 74: 2348-2351).

### [Production of transgenic tomato in which parthenocarpy responsible gene A is expressed]

In a manner similar to that described in [Production of RNAi transgenic tomato], with the use of the Agrobacterium method, a cotyledon of a parthenocarpic tomato line LS935 was infected with Agrobacterium having a binary vector pZK3BGFP_whole_gene_A, and cultured on a kanamycin-containing medium. Then, selection was carried out and thus a regenerated plant was obtained. Insertion of the gene in the regenerated plant was confirmed by the PCR, and a transgenic tomato in which the parthenocarpy responsible gene A is expressed was obtained.

### [Study of parthenocarpy of transgenic tomato in which parthenocarpy responsible gene A is expressed]

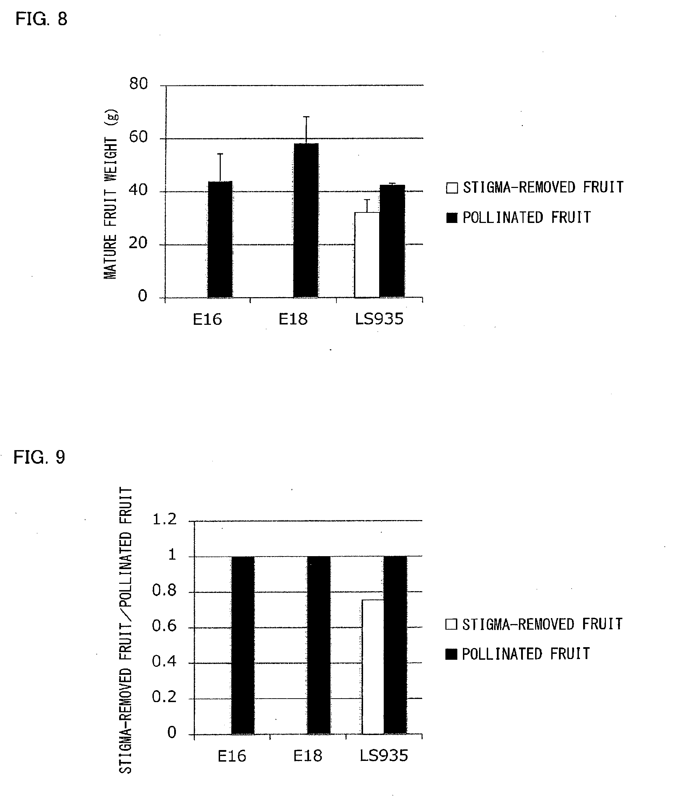

The transgenic tomatoes in each of which the parthenocarpy responsible gene A is expressed were cultivated, and mature fruit weights of stigma-removed fruits, whose stigma had been removed before anthesis, were measured and mature fruit weights of pollinated fruits, which had been pollinated at anthesis, were measured. Moreover, non-transgenic plants (LS935) were concurrently cultivated, and mature fruit weights were measured as controls through processes similar to those carried out on the transgenic tomatoes in each of which the parthenocarpy responsible gene A is expressed. Results are shown in Figs. 8 and 9. Note that, in Figs. 8 and 9, each of E16 and E18 is the transgenic tomato in which the parthenocarpy responsible gene A is expressed.

As shown in Fig. 8, the stigma-removed fruit of the non-transgenic tomato LS935, which is a parthenocarpic line, was fructified and enlarged to an extent similar to that of the pollinated fruit, whereas the stigma-removed fruit of the transgenic tomato in which the parthenocarpy responsible gene A is expressed was not fructified and enlarged. Moreover, as shown in Fig. 9, a ratio of the fruit weight of the stigma-removed fruit of the non-transgenic tomato LS935 was not significantly different from a ratio of the fruit weight of the pollinated fruit which was assumed to be 1. On the other hand, a ratio of the fruit weight of the stigma-removed fruit of the transgenic tomato in which the parthenocarpy responsible gene A is expressed was nearly 0.

### [2. Segregation analysis of tomato traits with use of DNA marker sequence]

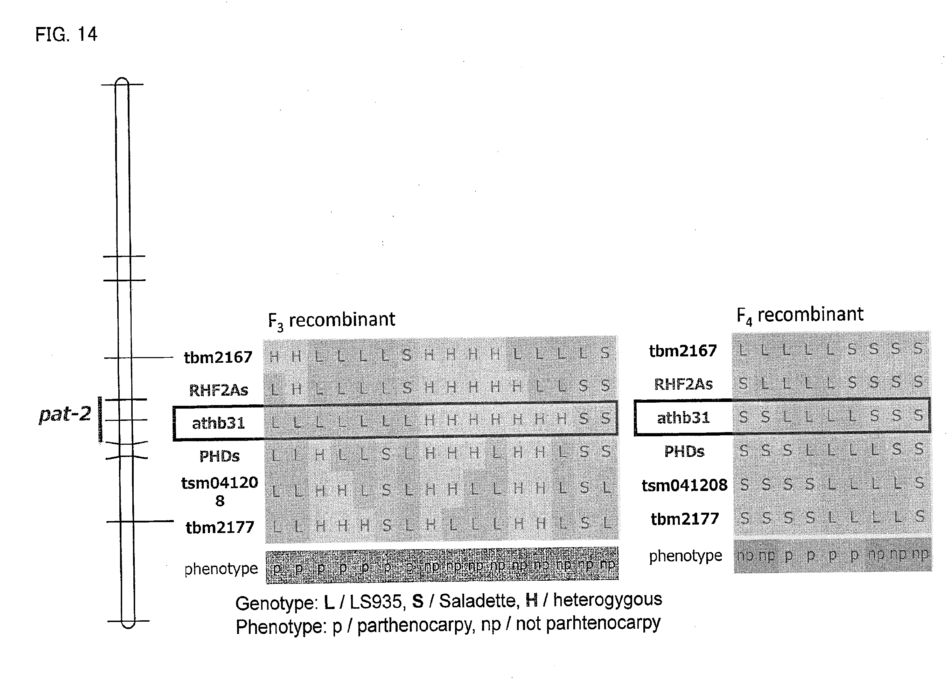

By crossing a parthenocarpic tomato line LS935 (obtained from National Institute of Vegetable and Tea Science) and a non-parthenocarpic tomato line Saladette (LA2662) (obtained from Tomato Genetics Resource Center in US), a population of F₂ individuals was obtained. Segregation of genotype and phenotype of tomato was analyzed with the use of (i) a selfed progeny F₃ population which was a line selected from the F₂ population, (ii) a selfed progeny F₄ population which was a line selected from the F₃ population, and (iii) marker sequences. Results are shown in Fig. 14.

In Fig. 14, a genotype indicated by "L" is a homozygote of LS935 (in which deletion occurs in the parthenocarpy responsible gene: parthenocarpy (p)), a genotype indicated by "S" is a homozygote of Saladette (in which no deletion occurs in the parthenocarpy responsible gene A: not parthenocarpy (np)), and a genotype indicated by "H" is a homozygote (not parthenocarpy (np)).

The marker sequences in Fig. 14 were developed by the inventors on their own and have characteristics below. Among these, RHF2As, athb31, and PHDs are high in evaluation accuracy, and particularly athb31 is a complete linkage marker that achieves evaluation accuracy of 100%.
· tbm2167: has a distance of approximately 1.1 cM from the parthenocarpy responsible gene A; is a SSR marker which is amplified by PCR that is carried out with the use of (i) primers respectively represented by SEQ ID NO: 48 and SEQ ID NO: 49 located on SEQ ID NO: 47 and (ii) genomic DNA as a template; and is a parthenocarpic line LS935 type if a fragment length is 241 base pairs or is a non-parthenocarpic line Saladette type if the fragment length is 243 base pairs.
· RHF2As: has a distance of approximately 135.1 kb from the parthenocarpy responsible gene A; is SNP marker whose 123th nucleotide is A or T in a base sequence represented by SEQ ID NO: 50; and has a genotype of the parthenocarpic line LS935 if the nucleotide is A or has a genotype of the non-parthenocarpic line Saladette if the nucleotide is T.
athb31: is an insertion-deletion mutation in the parthenocarpy responsible gene A; is a mutation in which 9977th through 11011th nucleotides are deleted from the base sequence represented by SEQ ID NO: 3; and has a genotype of the parthenocarpic line LS935 if the deletion has been caused or has a genotype of the non-parthenocarpic line Saladette if the deletion has not been caused.
· PHDs: has a distance of approximately 161.6 kb from the parthenocarpy responsible gene A; is SNP whose 119th nucleotide is A or G in a base sequence represented by SEQ ID NO: 51; and has a genotype of the parthenocarpic line LS935 if the nucleotide is A or has a genotype of the non-parthenocarpic line Saladette if the nucleotide is G.
· tsm041208: has a distance of approximately 247.7 kb from the parthenocarpy responsible gene A; is a polymorphism of a fragment which is amplified by PCR that is carried out with the use of (i) primers respectively represented by SEQ ID NO: 53 and SEQ ID NO: 54 located on SEQ ID NO: 52 and (ii) genomic DNA as a template; and is the parthenocarpic line LS935 type if a fragment length is 393 base pairs or is the non-parthenocarpic line Saladette type if the fragment length is 359 base pairs.
· tbm2177: has a distance of approximately 5.3 cM from the parthenocarpy responsible gene A; is a SSR marker which is amplified by PCR that is carried out with the use of (i) primers respectively represented by SEQ ID NO: 56 and SEQ ID NO: 57 located on SEQ ID NO: 55 and (ii) genomic DNA as a template; and is the parthenocarpic line LS935 type if a fragment length is 243 base pairs or is the non-parthenocarpic line Saladette type if the fragment length is 241 base pairs.

### [3. Identification of eggplant-derived parthenocarpy responsible gene SmA]

A sequencing library was prepared from genomic DNA of an eggplant cultivar "Nakateshinkuro", and pair end sequence of a fragment having an insert size of 200 pb to 300 pb and mate pair sequence of a fragment having an insert size of 2 kb were carried out with the use of a sequencer HiSeq2000 (manufactured by illumina Inc.) A total of 14.4 hundred-millions reads thus obtained (i.e., sequence data corresponding to approximately 140x of eggplant genome (approximately 1.1 Gb)) were assembled by an assembler program SOAPdenovo, and 1,321,157 sequences and fragment genomic sequences having a full length of 1,145 Mb was obtained. BLASTN analysis was carried out with respect to the sequence, and a genomic sequence (67,902 base pairs) was obtained which partially showed high homology to the tomato parthenocarpy responsible gene A. Gene prediction was carried out with the use of the genomic sequence thus obtained and a gene prediction program Augustus, and (i) a genomic DNA sequence (SEQ ID NO: 34) which includes a homologue gene (hereinafter, referred to as "parthenocarpy responsible gene SmA) candidate of eggplant and (ii) a protein code sequence (SEQ ID NO: 35) of the genomic DNA sequence were obtained. From the base sequences respectively represented by SEQ ID NO: 34 and SEQ ID NO: 35, it is clear that the parthenocarpy responsible gene SmA includes two exons and one intron. PCR was carried out with the use of (i) PCR primer pairs designed based on the predicted gene sequence and (ii) cDNA derived from a bud of eggplant as a template, a cDNA clone containing entire ORF of the parthenocarpy responsible gene SmA was obtained. A base sequence of the cDNA clone thus obtained was determined by the Sanger's method, and it was confirmed that (i) the base sequence of the cDNA clone was identical with the base sequence represented by SEQ ID NO: 35 predicted by the program and (ii) the cDNA clone was a gene expressed in the bud of eggplant. The protein encoded by the parthenocarpy responsible gene SmA has the amino acid sequence represented by SEQ ID NO: 25.

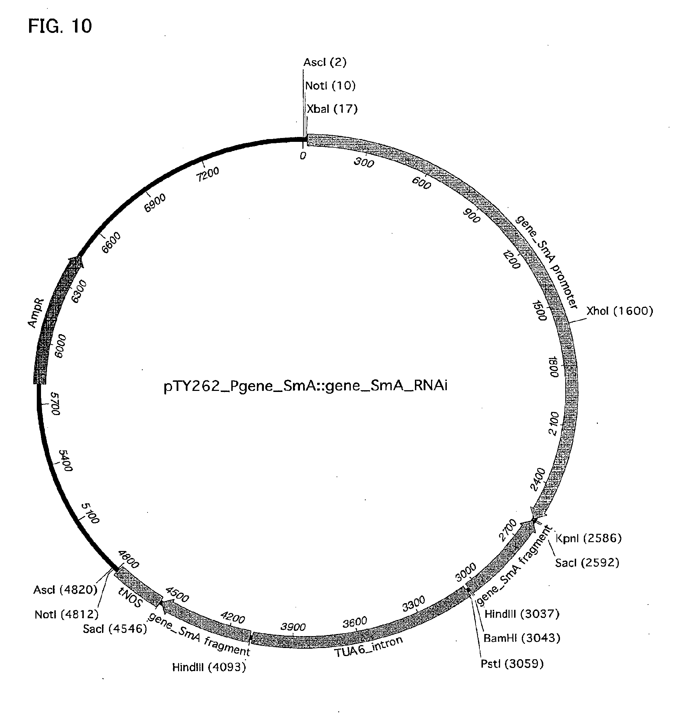

Primers having base sequences respectively represented by SEQ ID NO: 36 and SEQ ID NO: 37 were prepared, and PCR was carried out while using, as a template, a clone having a base sequence represented by SEQ ID NO: 35. A PCR product was obtained which had a base sequence of 473 base pairs represented by SEQ ID NO: 38. The PCR product had a BamHI recognition site and a HindIII recognition site at a 5' end, and a SacI recognition site and a KpnI recognition site at a 3' end. The PCR product was inserted to a cloning vector pTY262 (AB736152 (DDBJ)) so as to form an inverted repeat structure, and an RNAi-induction vector with respect to the parthenocarpy responsible gene SmA of eggplant was constructed. Further, a CaMV35S promoter of the vector was replaced with a SmA gene promoter sequence including 2561 base pairs represented by SEQ ID NO: 39 (see Fig. 10). The promoter sequence is predicted to include, at a 3' end, (i) 29 base pairs which are at a 5' end of the ORF of SmA having a base sequence represented by SEQ ID NO: 40 and (ii) a 5' untranslated region for encoding unidentified mRNA. However, RNAi induction does not require transcription of RNA for encoding protein having a function, and therefore a function as the RNAi-induction vector is not influenced.

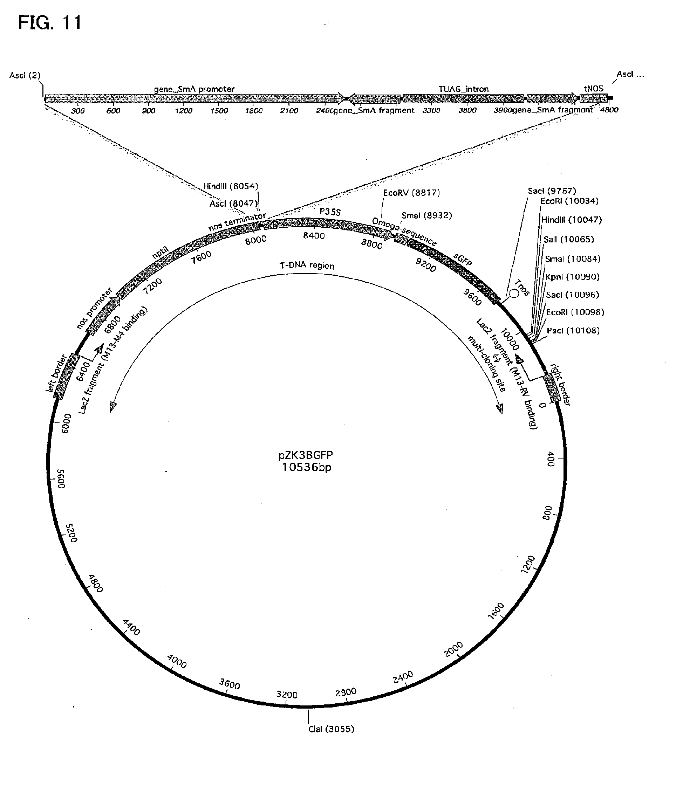

The RNAi-induction vector thus obtained was digested by AscI and then inserted into an *Asc*I recognition site of a modified binary vector pZK3BGFP in which a reporter gene including a CaMV35S promoter, GFP, and a Nos terminator was inserted into a binary vector pZK3B (Kuroda et al., Biosci Biotech Biochem, (2010) 74: 2348-2351). Thus, an RNAi-induction binary vector pZK3BGFP_SmA-RNAi was constructed (see Fig. 11).

Transformation of a non-parthenocarpic eggplant cultivar "Senryo-nigo" was carried out by the use of the RNAi induction chimeric gene (binary vector) illustrated in Fig. 11. Specifically, with the use of the Agrobacterium method (Billings et al. 1997 J. Amer. Hort. Sci. 122: 158-162), a cotyledon of the eggplant cultivar "Senryo-nigo" was infected, and culture on a kanamycin-containing medium and selection were carried out. Then, induction of an intended gene into the regenerated plant was confirmed by PCR, and thus an RNAi transgenic eggplant was obtained.

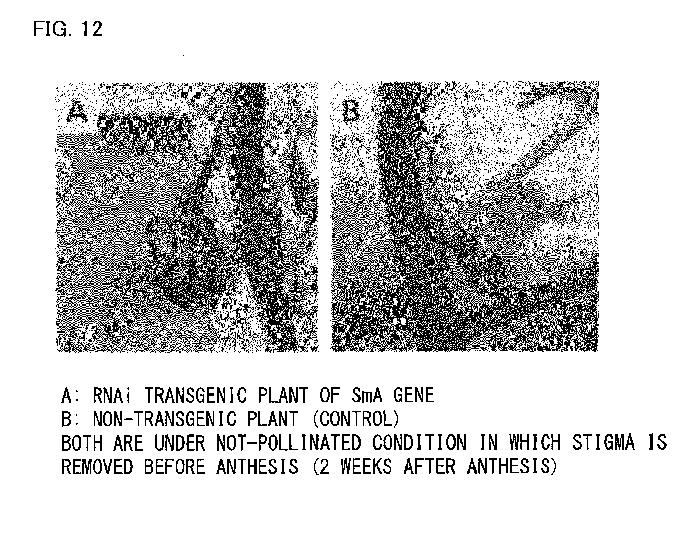

The RNAi transgenic eggplant was cultivated, and its flower whose stigma had been removed before anthesis clearly showed enlargement of an ovary (see Fig. 12). In a case where a stigma of a non-transgenic eggplant has been removed, enlargement of its ovary is hardly shown and its flower dies down in approximately 1 week after anthesis. From the above result, it has been concluded that prevention of falling and enlargement of fruit are also achieved in eggplant by inhibiting expression of the parthenocarpy responsible gene SmA, as observed in tomato.

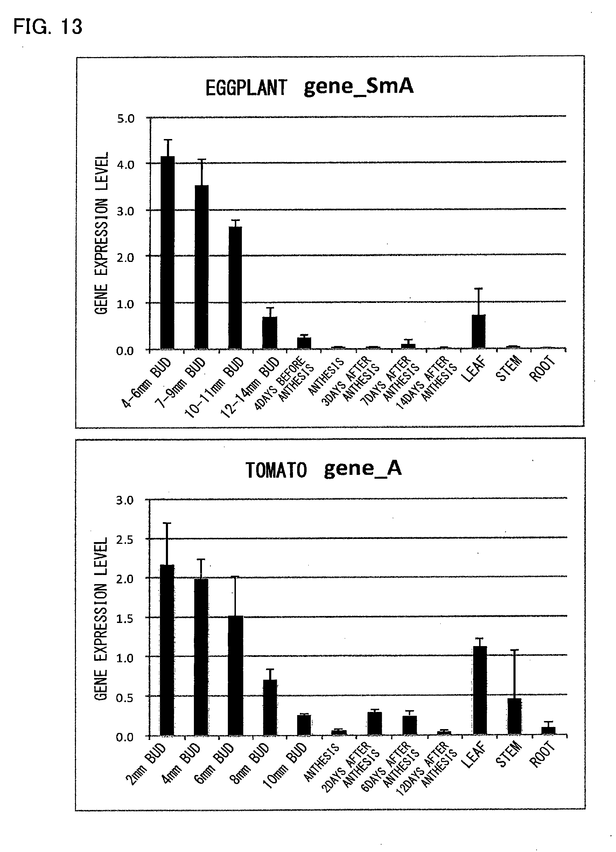

The parthenocarpy responsible gene SmA of eggplant has a sequence that is structurally most similar to that of the parthenocarpy responsible gene A of tomato. As shown in Fig. 13, it is clear that stage-specific and tissue-specific expressions are also similar to those of the parthenocarpy responsible gene A of tomato, from a result of quantitative real time PCR analysis. Note that, as an endogenous standard gene for the quantitative real time PCR, a housekeeping gene SmFL20F10 (SEQ ID NO: 41) was used which had been developed by the inventors and constitutively expressed. Moreover, for tomato, Solyc04g049180.2.1 (ITAG2.30, SEQ ID NO: 42) was used which was strongly supposed to be an orthologue of the SmFL20F10 from a result of BLAST search. The followings are sequences of the primers used to amplify the endogenous standard gene:
<For eggplant SmFL20F10 (SEQ ID NO: 41)>
   Forward primer: SEQ ID NO: 43
   Reverse primer: SEQ ID NO: 44
<For tomato Solyc04g049180.2.1 (SEQ ID NO: 42)>
   Forward primer: SEQ ID NO: 45
   Reverse primer: SEQ ID NO: 46

### Industrial Applicability

The present invention makes it possible to obtain a new cultivar plant having parthenocarpy. Moreover, the present invention can be used in a field such as agriculture or horticulture.

## Claims

1. A method for evaluating parthenocarpy of a plant, said method comprising the step of:
checking whether or not gene expression of a parthenocarpy regulatory gene, which includes a polynucleotide recited in any of (1) through (4) below, is inhibited in the plant; or
checking whether or not a function of a polypeptide, which is encoded by the parthenocarpy regulatory gene, is inhibited in the plant.
(1) A polynucleotide that encodes a polypeptide having an amino acid sequence represented by SEQ ID NO: 1,
(2) a polynucleotide that encodes a polypeptide (i) having a sequence identity of 70% or higher relative to the amino acid sequence represented by SEQ ID NO: 1 and (ii) having a parthenocarpy regulation activity,
(3) a polynucleotide that encodes a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity, and
(4) a polynucleotide that (i) is hybridized with a polynucleotide, which has a sequence complementary to the polynucleotide recited in the above (1), under a stringent condition and (ii) encodes a polypeptide having a parthenocarpy regulation activity.

2. The method as set forth in claim 1, wherein:
in the step of checking, a base sequence of the parthenocarpy regulatory gene or an expression level of the parthenocarpy regulatory gene is checked.

3. The method as set forth in claim 1 or 2, wherein:
in the step of checking, a plant in which the gene expression of the parthenocarpy regulatory gene is inhibited or a plant in which the function of the polypeptide encoded by the parthenocarpy regulatory gene is inhibited is selected as a parthenocarpic plant.

4. A method for producing a parthenocarpy-regulated plant, said method comprising the step of:
selecting a parthenocarpic plant by carrying out a method recited in any one of claims 1 through 3.

5. A method for producing a parthenocarpy-regulated plant, said method comprising the step of:
inhibiting, in a plant, gene expression of a parthenocarpy regulatory gene that includes a polynucleotide recited in any of (1) through (4) below; or
inhibiting, in a plant, a function of a polypeptide encoded by the parthenocarpy regulatory gene.
(1) A polynucleotide that encodes a polypeptide having an amino acid sequence represented by SEQ ID NO: 1,
(2) a polynucleotide that encodes a polypeptide (i) having a sequence identity of 70% or higher relative to the amino acid sequence represented by SEQ ID NO: 1 and (ii) having a parthenocarpy regulation activity,
(3) a polynucleotide that encodes a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity, and
(4) a polynucleotide that (i) is hybridized with a polynucleotide, which has a sequence complementary to the polynucleotide recited in the above (1), under a stringent condition and (ii) encodes a polypeptide having a parthenocarpy regulation activity.

6. The method as set forth in claim 5, wherein:
in the step, (i) a polynucleotide, which inhibits the gene expression of the parthenocarpy regulatory gene, is introduced into the plant or (ii) the parthenocarpy regulatory gene is disrupted.

7. The method as set forth in any one of claims 1 through 6, wherein the plant is a solanaceous plant.

8. The method as set forth in claim 7, wherein the plant is a tomato.

9. A parthenocarpy-regulated plant produced by a method recited in any one of claims 4 through 6.

10. A parthenocarpy regulatory gene that includes a polynucleotide recited in any of (1) through (4) below.
(1) A polynucleotide that encodes a polypeptide having an amino acid sequence represented by SEQ ID NO: 1,
(2) a polynucleotide that encodes a polypeptide (i) having a sequence identity of 70% or higher relative to the amino acid sequence represented by SEQ ID NO: 1 and (ii) having a parthenocarpy regulation activity,
(3) a polynucleotide that encodes a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity, and
(4) a polynucleotide that (i) is hybridized with a polynucleotide, which has a sequence complementary to the polynucleotide recited in the above (1), under a stringent condition and (ii) encodes a polypeptide having a parthenocarpy regulation activity.

11. A polypeptide recited in any of (1) through (4) below.
(1) A polypeptide having an amino acid sequence represented by SEQ ID NO: 1,
(2) a polypeptide (i) having a sequence identity of 70% or higher relative to the amino acid sequence represented by SEQ ID NO: 1 and (ii) having a parthenocarpy regulation activity,
(3) a polypeptide (i) having an amino acid sequence in which 1 to 87 amino acids are substituted in, deleted from, inserted into, and/or added to the amino acid sequence represented by SEQ ID NO: 1 and (ii) having a parthenocarpy regulation activity, and
(4) a polypeptide that (i) is encoded by a polynucleotide which is hybridized, under a stringent condition, with a polynucleotide that has a sequence complementary to a polynucleotide for encoding the polypeptide recited in the above (1) and (ii) has a parthenocarpy regulation activity.


SEQUENCE LISTING

<110>  INCORPORATED ADMINISTRATIVE AGENCY NATIONAL AGRICULTURE AND
        FOOD RESEARCH ORGANIZATION

<120>  Parthenocarpy regulation gene and method for developing
       parthenocarpy plant

<130>  AF13055

<150>  JP2012-170506
<151>  2012-07-31

<160>  57

<170>  PatentIn version 3.5

<210>  1
<211>  290
<212>  PRT
<213>  Solanum lycopersicum

<400>  1

Met Ala Leu Gly Gly Gly Asn Glu Glu Lys Glu Met Arg Ile Ser Gly
1               5                   10                  15

Phe Gln Asn Ile Asp Leu Val Ser Pro Gly Gly Ala Ala Pro Asp Asp
            20                  25                  30

Ile Val Ser Ala Ala Pro His Asn Asn Ser Thr Thr Gly Ser Asn Lys
        35                  40                  45

Leu Asn Lys Gly Thr Ser Thr Val Arg Gly Arg Tyr Arg Glu Cys Leu
    50                  55                  60

Lys Asn His Ala Ala Asn Ile Gly Gly Asn Val Thr Asp Gly Cys Gly
65                  70                  75                  80

Glu Phe Met Pro Ser Gly Glu Glu Gly Thr Leu Glu Ala Leu Lys Cys
                85                  90                  95

Ala Ala Cys Asn Cys His Arg Asn Phe His Arg Lys Glu Ile Pro Asn
            100                 105                 110

Asn Val Gly Ala Gly Val Gly Gly Gly Val Gly Gly Asp Asn Asn Asn
        115                 120                 125

Asn Ala Gly Ile Met Val Val His Pro Leu Gln Leu Pro Gln Pro Leu
    130                 135                 140

Pro Ser Pro Ile Pro Ser Leu Asn His His His His His His His Gln
145                 150                 155                 160

His Gly Gly Arg Ser Ile Trp Thr Thr Met Pro Pro Gln Pro Val Lys
                165                 170                 175

Met Ala Phe Gly Gly Ser Gly Gly Gly Ser Gly Ala Thr Asp Ser Ser
            180                 185                 190

Ser Glu Glu Leu Asn Phe Asn Thr Tyr His His Gln Gln Ala Thr Ser
        195                 200                 205

Val Pro Pro Gln Gln Pro Phe Met Leu Ala Lys Lys Arg Phe Arg Thr
    210                 215                 220

Lys Phe Ser Gln Glu Gln Lys Glu Lys Met Leu Glu Phe Ala Glu Lys
225                 230                 235                 240

Leu Gly Trp Arg Ile Pro Arg Glu Asp Asp Ala Glu Val Gln Arg Phe
                245                 250                 255

Cys Ser Gln Val Gly Val Lys Arg Gln Val Phe Lys Val Trp Met His
            260                 265                 270

Asn Asn Lys Asn Pro Ser Ser Ala Lys Lys Asn Ile Ile Gln Glu Asp
        275                 280                 285

Gln Pro
    290

<210>  2
<211>  873
<212>  DNA
<213>  Solanum lycopersicum

<400>  2
atggcattag gtggtggtaa tgaggaaaaa gagatgagaa tttcaggttt tcaaaatata      60

gatcttgtct cacctggtgg tgcagcgcca gacgacatcg tcagcgctgc accacacaat     120

aattccacta ctggatctaa taaattgaac aagggaacta gtacagtgag aggtagatat     180

cgtgagtgct tgaaaaatca tgctgctaat attggtggaa atgttacgga tggttgtggt     240

gagtttatgc ctagtggtga agaaggtaca ctcgaagctt tgaaatgtgc tgcttgtaat     300

tgtcatagga attttcatag aaaagaaata cctaacaacg ttggcgctgg tgttggtggt     360

ggtgttggtg gtgataataa taataatgcg gggattatgg ttgtgcatcc attacagctg     420

ccacaaccac taccttctcc gataccgtcg ttgaatcatc atcaccacca tcaccatcaa     480

cacggaggaa gatcaatttg gactactatg ccacctcagc ctgtcaaaat ggccttcgga     540

ggtagcggcg gaggaagtgg ggcaacagac tcatcaagtg aagaacttaa tttcaacaca     600

tatcatcatc aacaagcaac atcagtacct ccacaacaac catttatgtt ggctaagaaa 660

agatttagga caaaatttag tcaagaacag aaggaaaaaa tgttggaatt tgctgaaaaa 720

ttaggttgga gaattccaag agaagatgat gctgaagtac aaagattttg ctcacaagtt 780

ggagtcaaga gacaggtttt taaggtttgg atgcataata ataagaaccc ttcttctgct 840

aaaaaaaata taatacaaga agatcaacct taa 873

<210> 3
<211> 13050
<212> DNA
<213> Solanum lycopersicum

<400> 3
cctgaccgtg gtgttcaggt caacttctgc tcaactagtt cgacgagata cctgtcacgt 60

cccaccagca ataggtatca ggtaactctg tcgaccaagg ttaggacaac tcttcatgct 120

gaagaaacta caactcgtgc atctggagtc tagacaaata gatggttgat actagtttcg 180

aaaagagctt tagagtcaaa gacaagtcac tttcattagt tgtaacaaat aacaagactg 240

cagttatagt ataggagtac aagagaaaat aattacataa aaaacatata agttagctag 300

tgaagaggct acctatatat gttttacgcc atgataaacc tcatggtacc tacaccttat 360

cttgaaaaga caaaagcagg ggaaattgtg aaagacgcgg tattagaaat aagcataggg 420

ttagattttt ttcgatacaa ctataggcaa cctaatagtt aaaaccatcc aaatgtaagt 480

cgataaaaac ttaaaggaaa cagacgaaat tgagataatg cataacatta agtacttagt 540

ctatgcagtt tgttcacaat tcgttatcat cacaacatac ttgaagtcaa tatacagatt 600

gtcaaaaaga ttgttttttt ggcctaaaat actatagcgt tgacgcgtct atattagcca 660

tagaattgga attggaggat ctaatctctt ccagcagggt gaatttccta tctgatatta 720

tccacttgct gatgataata cttttcaaca aaaagtttgg tcacaaaata ctattttctt 780

attctccctc cacctattgt acctctcctt aaagaaaaat attcgacaac ttgctacaac 840

tcaacgcctt cttctccatc ttataaggac ctaacgtagc attatgttgc ccataccatt 900

cacttgagct agcactcggc tttaggcgaa gtagctctgc acacacgtgt acaacatggc 960

ctcgtggact gcaaggcaaa acaagaagtt cgaagaggct ttggcgttat atgacagaga 1020

cacacctgat cgttggcata acattgcaag gtgtgtaggt ggaaaatcag ctgcagaagt 1080

gaagaggcat tatgaagtac ttgtgaagga tatcatgcaa atagaaaatg gtcaagtacc 1140

tttaccaaat tacaaagccg cggctgaaac caataacaga tcaggttatg ccaatgaaca 1200

taggtattaa acatcaaaac taatctttta ttaattttgt acaagtcatt gatacgttct 1260

gtattcgatt cccatatggt tcgtgtcatt ttcctattca tggtactgtt tatgtttatc 1320

accatctatc catttttagt aactttacaa gccaaagact agtcacactc cttgttgaca 1380

cttttattct aaagaataaa tgcttatagt ttcaactctc tgcatccacg attgtttctt 1440

gttcctcgat aaaagaaaat gtacgcaaac aagtaactaa tgcttatgaa catgtctata 1500

tgttgttgaa caggcttctg aagaacctga agttacagtg agaacaaatt tcgcggccat 1560

gctgaataac atcacaagtt gtctgttata tttgcaaatg caaaaggcat aaattgtgaa 1620

tttatgtctt aagtttttga agcaagcaaa atgtattatg actattgttg ttcatctcta 1680

tccatttaac ttttgtttta ctgttgtaat gtttctcaaa cgcataatat aaagtctact 1740

tcactttcat gttatagtgt aatatgtttg ataaaataaa atcaaaatta cacgttttat 1800

agtctgaatt caatggatcg taattctcaa ttcatttgtt cttttttaga tacttgtaaa 1860

atattcaagt acactttcac aatgagattg tcacacggtt cagtatcata acaatcaaat 1920

ctcacaccgt acatgagttc atatgtgatt ctttaggtta ctattagaga ttacgaattc 1980

gagctatggg agctaacaac atcatgagtt catatgtgat tctttaggtt attattagag 2040

attacgaatt cgagctagga gctaacatct aaaaaatagt tcatatgtga ttctttaggt 2100

tattattaga gattacgaat tcgagctagg agctaacatc taaaaaataa aagtaggaac 2160

gtaaaaatgg agatgcgggt atcgatcccc gtacctctcg catgctaagc gagcgctcta 2220

ccatctgagc tacatcccct ttcacatgct gtgcatctta tattaaaata aatatatcaa 2280

ataaacatct agtaaacaga aatagttgta caaggaagga acatttagca cttttttttt 2340

ttaagaatca aaactgctta aatgacatat ttatgtatta taccattata aaactaaatc 2400

aattcagact cgctttaaaa aattgtatac aacgtctata tattaatttt tatctcatag 2460

gatcaatcac aaaattaatt ttgtaataca tcaacaaggt agattaaaat tttacaggaa 2520

cacctttaca tgtagaaaat tattattgcc attgaatacc aagaacaaaa aaaaatatac 2580

tattttctct tgtacatagt taggtaacta acaaactaat caaactcccc tcagcttttt 2640

tcgagcttct gtctgtcaaa ggttcgagaa tcaatgaaag atcatcaccg tctttgttat 2700

aggcgagcaa attaacagac gaaaataaaa aaggaaaaat cttgaacacc caacacatct 2760

cctcttcgtc ttcttcgtat gaccaaattt gtaccaattc ttgagtctca taactataag 2820

caacaatttt atgtgccaaa acttgtagat ataatacctg taaatcctta ttgaatgcac 2880

atggcttcac tgtgtaccct gtttcaaacc ccaatcttac aaaatgatca agcttgaagt 2940

catttcctct gaaactccat atgaaaactc cattgtcaac tggatcacag aattgtatta 3000

ctccgtcttc ttccgatgat tcgtataaaa actgagagtg gtaaacatct tttcttctgg 3060

aattgttttt cggcagtgaa aacagtgtaa atactttgaa atctatgtcg tataccaaca 3120

aacataaact ccatatccag tacaattttc ttttctcgcg agaaaaaaca ctcgaattat 3180

agcattgccc tcgaataaat ccatctgatt ggaggagatg ggaattcaaa atttgaattt 3240

gggtttcccg ccacttccat gaattcgatc cgaagataat acaattcacc atttgggtat 3300

cgaaatcaat ttcttcccag aagaaagcga taacttgaaa cgaatttaac tcaaaaacaa 3360

gacttgaaca agtgggtctt gaaatatgat gagaaattgg tagagaaatt aactggttaa 3420

gaacagggga tgaaacagag tatgtccaga acccattgtt gttattttct gaagcgtaaa 3480

gaagaacccc attacaagaa tcgatcaaat aagggaattt atgagtggaa gatgaatggg 3540

aagtacaaaa tttggggatt tttttctggg ttaagtcgtg taacttcatg aaaactgatt 3600

cttgataagt tttgtgtttg aggaagatca caagaccagt tgaagttgtg agagggtaac 3660

aatggcggat attggtaatc gaatttcgcc atgttttcga aacaatcttc aattggatga 3720

gagatttaaa gggaatttta gtgataattg attcaactat atgatttgat aaattcccaa 3780

gctctatcat ctcttcttct tcttcttgct ctgtttccat ggcaatcgcc attgctaatc 3840

ccctctgttt tttgaagtta aatttcatat tcaatcaatt aattaatact taaaaggtat 3900

ttatagaaac cctaataaac tagctatggc ggttaattat caattacagt taatttattt 3960

gtgtttgata attaattaat tgattaattg tacttggtat gcaagattat aagtaagtaa 4020

atgcttgatg agcaagcatt aaatttctat agttaaacgg gttgataact aatcacctat 4080

attttctcac ttaaatattt atttttggct atttatttac aatttttttg gctagttata 4140

tttttggtaa agtttaatca tgtaatatat gacatatgta tatatataat ttatagcgta 4200

actttcacat ataacaaata aaaaatttat atttgcatgc tatagtaaag tttgcgtaat 4260

tgcgctccat agcaaacata aaaattgtat aattcgctat acatatacag ttgaagcaaa 4320

ttgtataaaa cgaagtgtat aaaacaagaa agagaaagac acttgggcag agaactgtat 4380

aaaaacgaag tgtataaaac atattgtatt ataagtgtat agaacgatta tatacaattt 4440

gaatttgtat aaaatgagaa tgaaagaaaa caaaagagac ttgacaagga atatacaatt 4500

gaatcgaatt gtataaaacg agaaatggag aaattagata caatttaaaa attgtataaa 4560

acgagaaaga gtgaaaggca aaaaaaaacg ggatagggaa atatttttat tgtataatca 4620

taagtgtata ggacaaatag caaaactata gagcgctaat atgattttat atttattatt 4680

cctaaaattt actattctaa aaattatttt aaaatactca taagtttgta ttatcattct 4740

ataatgaaaa gtgtttcgtg aaaagtcatt acaagaacat aattcaaatt cattaaaatg 4800

aatttgaaaa aaaaggaagg aaataaaaga aaaatgtcat tgatttgtgt atgaaaaact 4860

actattatta cttgtaattg gaagtgactt cgaagaacaa gctaggataa aaatagtata 4920

taaagtattt atataaataa ataaatttta ttcaacaaat taataatgaa atttaatcaa 4980

aaattttgaa tgataggtgt ttttattcag ctactagtaa tagtgtgatt atttaagagt 5040

gtatttatat attattttat gcatgtatta attatataga gattgattat ttattcactc 5100

ccataaggta tctatttctt atttcatgca taaaaagata cataaattct ttcataactt 5160

ttacatattt taatagtcat gcaagtttct taaatatata catgaattag atatttttct 5220

atacctatcg aacataaatg tcgtacaaat tatataaaaa ttaatataaa aataacttat 5280

ctcttatcag ctatcaaaca acttgtaatt tcacaaataa aatatgaaaa tgttaatagg 5340

taaactataa atatgttata tttaatagaa tctaacacta ataaacaatt caaagtaaag 5400

aaaaaaaatt gctataacgt tgctctcaat taaatcagat gtcataattt tgacgtttca 5460

caaaaataga atttttttta aagaaaattg tcatatttgt tgaagtaaca tgtgtgccca 5520

tatgtggtcc tgttattatt agaaagtagg taaaagaagt atgaaaaata ctatattata 5580

attggatctc atgatataat aagtgaacat aatttggcac agattaagaa aagcatcctc 5640

aattaaggat attaatagaa tatttattca tgccataaaa ggaaagaata gtataattgc 5700

atgtgttttt ttaattacat gtttgcaaga ctcaaattgt tatgtttatt agtcggtgct 5760

taccttaaaa taataaatgc atatattatt tgcacatcaa ctttttctat ttaattatag 5820

ttgactaaca tgtactttta tattctaata aagtctacct aattatgata agacaattct 5880

ccctcattaa cttttgtttg ttttatgtta tataaatttt agtgattcaa atcttaatta 5940

ttagtgtata tttgttatat tactttataa ccatgcactt aacgatttta aatagatttg 6000

aataatttat aatattttta gttattcaga aaaatacaca agtatctttt taatctatgt 6060

tcaaaaaact tcaaagacac acttatacta tactaatgtt ctattacttc cctaaattta 6120

ttttatagta attttctacc tctttttgat ctacgtggta ttgggttgaa aaaaaaataa 6180

accagttttg atcccacaaa atagtgtcac gtagaccaaa agggggaagg aaaattacat 6240

ataaaataag ttcaggggaa taatatgacc ttggtatagt ataaatgtgt ctttagattt 6300

tggcataggt tgagaaagta tttgagcatt atccctattt ataatcattt acaatcatca 6360

ttacccataa taatcgttca tcataattat tagttatcac ttctggcttc aactaatcac 6420

aaaactttta aaaaatcatt aatatgtcat ataaaaatta tttataaaaa gtaataattt 6480

taccgttact ttgatatttc atcaaaaaca tacaaaaaaa cttttaatgg atgcatttcc 6540

caagttatca tataaattga taatgaccac aagtagcctt ctgattcctc ctatggtcca 6600

ttatcttcat gtttcaacat ttatagcttc tgcttcttgc atagcttatt taaataaaat 6660

tacattttaa tatatatata gaaaaagtga aacaaacttt catagaccca atttaataat 6720

ataccatttg tttccattaa ttttaaaagt gaatggaaat taaccacact cacaaacaaa 6780

tatgatgaaa taacatattt tattcatcac tatacctaaa tacaatcata tttttcactt 6840

atttttattt atgaattttc ttcgcgactt gatgatttta atatctaatg gatgtatgtt 6900

gatttgaata tccttgcaat gtgttttatt attacgtatg tcatgtattg ttatttaata 6960

tgttgatttt ttaataaaaa aaatatttat aaaaaattat tcgagaatat aataatacta 7020

gaaatattta aaataatcgc ttctttcaat aaaacaatat atcttgctac atgtacacct 7080

ttgagtttca cttgcttatt atattgttta tttttggcga tttatttttt tatatatatt 7140

attgaacatg atccaaagaa gatccaccat tgttcgtttc caagcttatt tattaattta 7200

tttattttcc tctattttaa tttgacggtg aaatatgatt atcaattctt tcttccattt 7260

taataatcaa aattacttaa aatatccaaa atgattgtaa ttttacaaaa aataaaatga 7320

aattaaataa tattaagttt tcactttgcc tttaatatta agtttttacg gaagcaacca 7380

ttatactaaa caataggaag aaaacaatat aaatccgaac aaataaataa ttatcggttg 7440

atatcatgat tttcaaatat aaaaaagatt aattcatatt cagtcatatt tcttaaaaat 7500

aaataaggac acaaagaaaa acatgagaat tattaataat atagaatgaa tgaagcatgt 7560

tgaatttata tattatataa aatatatttt ttaattagtc tcaattaata tttatatttt 7620

caattttaaa aattcataaa taaaaaatta aatttacata aaataaaata aattaataca 7680

tatatttaac actatataaa acaaatatat gtcacgtaaa ttttgtttac tgttacttta 7740

ttgacgtata ttaaatgtta gtaataggat aaaaactttc aatcattgca aggaaaagat 7800

ggtgtatgca ttgtatttag caattaattt tatgaataat attaatgcct aatttatgta 7860

tttttatatt tttttaagtg acaataaatt aatgtgacgc aattatgctg tctcaattgt 7920

cgagcccctg caatattact gataacgcaa atgccttgaa tgttgattaa tctgtaaaaa 7980

aagataaagc aagtaccaaa gcaaataaca gaaattagac atgacaagag acctcaccca 8040

gatgtaagtt tgtactaatt ttatttttta tcttaaatta tttatcatat cttgataagt 8100

tgattagcgt agacacttaa atttgtataa agttgaacaa ataggcacac ttgaccattt 8160

tatatggtaa tttatatctt acgtgtacta tgtcacatat ggctcatgtg tctacctgtt 8220

taattttata taagtttaag tttttacttg tgtgcattca aaattgagat gcataattgt 8280

gtaactaatt taaattaaat gagacattta ccatgtcttg tgtcatttaa aaataaaaaa 8340

tacttactat tagagaaaat ctctctcgtt aaaatttgaa tggtgacaaa taaattgaaa 8400

cgagaaaaaa gaatttgcta aggagaaaag catggagttt gcaggtagaa ggtgacatag 8460

aattaggagt actcataatt tgcatcattc attgggcaga agttgaataa taataataaa 8520

agctgatagc agagtcaaat cagattgcag aatcgcacta tttttgcgtc attttcgact 8580

ctatgtgtat gtaacacggt aaactaaaag attcaccaac tccacacccc aaaacatatg 8640

gtgaatcttt tgaagactag agtagggata tcgccaaata tgtcctaaga agctgaggac 8700

taagtttaat atatccataa tttaaattta tagatggagt actagataat tttatattaa 8760

actaattttt atcaaatcca acctaattca ttttttaagt agtattcaaa ctttgcccat 8820

ttctaagtca tacttatatt tacttttgac tttttttccc tatagtttgg agcttaagtt 8880

ctgagtcaga gttaaaactt gaaattaaag ttggattgct cactggttaa ggttggatct 8940

cagg tcgaga tcggtttatt gttcaggact cgagacaaag tcatctcatg ccccaaatcc 9000

ctgtcgggta tcgagtccca agtaagaatt gaatgtcgaa tcacgagtct tgaatttcaa 9060

attaagcccg aagctcaatt atcgagtcta aaatctcgag tcagaggcga ggtcgagtct 9120

agagtcctgg tcagggttag atttcaagta aggatcaaaa ttgagactca aaatctcaaa 9180

ttttataact ccattcaaaa tcaaatcaag tctgagatcc ggactcaagg atcgaattag 9240

agaaataaag ttgttcattc taacccccta atgggaaaat gaaggggggg gggttggtag 9300

ggcaataatg aataaatggg gtggtgggca tggggccaat gtttgcaggg cgtgggttag 9360

gactgaacat gacattgtcg gttgttttct tatgcacact tcatccagta tctctttgct 9420

gtttcccact ttctctctct tctctctctc tctcccttct tctttagctc cgtttgaagc 9480

aaccatgttg tctcttgttt cttacatccc atgtttcttc ctccccaaca acaccacttt 9540

ttattcctta aaatctctcc ttctttttta acaacatagt ccattgtttt tccttctcta 9600

cttctagggc cccccatcct cctttcattt cttcaatttc acaccccccc acataacatt 9660

agacataggg ttagggtttc tttttttttt tttttaaatt aacaaaaaaa aaaaaatcat 9720

ggcattaggt ggtggtaatg aggaaaaaga gatgagaatt tcaggttttc aaaatataga 9780

tcttgtctca cctggtggtg cagcgccaga cgacatcgtc agcgcgcacc acacaataat 9840

tccactactg gatctaataa attgaacaag ggaactagta cagtgagagg tagatatcgt 9900

gagtgcttga aaaatcatgc tgctaatatt ggtggaaatg ttacggatgg ttgtggtgag 9960

tttatgccta gtggtgaaga aggtacactc gaagctttga aatgtgctgc ttgtaattgt 10020

cataggaatt ttcatagaaa agaaatacct aacaacgttg gcgctggtgt tggtggtggt 10080

gttggtggtg ataataataa taatgcgggg attatggttg tgcatccatt acagctgcca 10140

caaccactac cttctccgat accgtcgttg aatcatcatc accaccatca ccatcaacac 10200

ggaggaagat caatttggac tactatgcca cctcagcctg tcaaaatggc cttcggaggt 10260

aatgtgttta gctcatctaa aaattttaat gaaatctcat ttgctcaatt tgaattacca 10320

tgccacctca gcctatcaaa atgggctttg gggggtttcg atttaacatt cgattatttc 10380

gtctaatagt ttaaattgtg agacatattt tttttattta cttgtactaa ttatttcatt 10440

ttatgtgtct ttgtttcaca ttgcactaaa tttagaaggt gaatatctaa tttgaatatt 10500

gtgattttaa accttattaa tcatttctta aaatagtatc attaagaata attagcatca 10560

tgagaatgtt agatttcaaa gcttacttaa atactcatat gataagatat aaacatacat 10620

tactagaaat aaataaaaaa aattcacctt gaattcgtga gaaatttgta ttatcgaact 10680

gatttttcca ctcatttttc acctaaccag ctcacggccg cgagaaacac atctaacttc 10740

ctaatttttt cgcgagaaaa tgttctggtc attccttact aattcaatca aaatatctct 10800

aaatatttct ccatgaaaga aataataatt ttttagtaat gatatttact gaagacacaa 10860

attaaaataa attattacgc ttaattatgt cttcaacacg ttgttagtac ctttttgtcg 10920

actcgaacct aagactcttg atatcatgat gaatcgtatg ataagttaaa atcattaaag 10980

tgaactatat acctacattt ttattttttt atttacttaa ttatatcttc aaacaggtag 11040

cggcggagga agtggggcaa cagactcatc aagtgaagaa cttaatttca acacatatca 11100

tcatcaacaa gcaacatcag tacctccaca acaaccattt atgttggcta agaaaagatt 11160

taggacaaaa tttagtcaag aacagaagga aaaaatgttg gaatttgctg aaaaattagg 11220

ttggagaatt ccaagagaag atgatgctga agtacaaaga ttttgctcac aagttggagt 11280

caagagacag gtttttaagg tttggatgca taataataag aacccttttt ttgctaaaaa 11340

aaatataata caagaagatc aaccttaatt aattttgttt aattaaggtt ttgagggaaa 11400

aaatgaaatt aaatttcttg ggatttggtt tatgcttatg taatcttact aggaatcgta 11460

cgtagctagt tttttttgtt ttttttttgg gggggtctat gtagtattat tgtaactata 11520

tggaaccact taattagatt tttattttta ttaattgttg tactttggaa cctttattca 11580

tctgaaaatt ttcagctatt tcttacactt ctcttgtcac tgaaatttag accactcatg 11640

atgccgcttt atcgaggtta gcgaggtaga gataaaattt acgtatgtaa actttaaaaa 11700

aaatgtgcta gatatgttgt tgtaatatga gccaagttgt atatcgaatt ctttagctct 11760

caagatagag taaagtctgt gtatgaagtt agttaattga tgatgttgag ggagaaacat 11820

gagttattta tttttttgaa atataaaaat gacaagtaaa agcgaaaaat aaatgttttt 11880

tttaggaatg gaaggatatt taattaatag agagccgttg gatgaaaaga aagaatgaat 11940

cttagtcgtc taatttgaat tatttgaaag agtagtagtt ttttttatcc ttagtcttgt 12000

cgaatataaa agcttttcct atttaaattt atttattagt ttgtgtattg tctatttcaa 12060

taaattatga tgcttcttgg agtttctgtc accattttaa atgtttgttt cctattttgg 12120

gaatagtttg ctgcttcttt ttctcactat ttgaggctta tacttataga gcatatcacc 12180

ccaatcttct ccatgtgatt atcaatgttg agtgtcataa acattagata atacaaattt 12240

tcgacattcg atttaagatt atgattttaa aattattttt agatatgtaa ttttattatc 12300

atgattttaa agtttaaatt attcaagaag cacgattgaa attttagttg atttatattt 12360

tataaaaaaa atatatcatt gaattttgaa aaaagaaaga cccatgctca tcgtgaattg 12420

acagtttatg ctatgtgaaa taattatact aagaaatttc tctttatatt ccttttagtt 12480

gttctgaaat tctttttctc aaagttaaaa aataagaatt ttgattaacg ttttacaata 12540

cattttatta ttatattgat aataataaga actttaacta attttattag atgttgcgat 12600

cagatacata gccctacgcg acttatcgag acgttgagtg atgtatctga aactgatcag 12660

atgcaatata ttgagacgta atgtattgag atattttgga aagcaattga attccactaa 12720

attaaaaaaa agtatatata taataaaaga gagtaagaat ataatatatt taactcttta 12780

acacgtatgc aatgttatgt taaattccct taataattca cttaaaaggt tatttgaact 12840

ttgattaaga tgttgtgaat aatagatcat agttcaagtc cattcatttc ttatcaaatt 12900

ttaatcatcc tcatttttgt ctctttttca tgtgctttga cgtttgattg cttctatcct 12960

tatcttcact tatttgatat aaaaaaacaa aaaatacatt aaaaatgtag gtcactaaat 13020

tctacataaa acacattatt caaagtaata 13050

<210> 4
<211> 12033
<212> DNA
<213> Solanum lycopersicum

<400> 4
cctgaccgtg gtgttcaggt caacttctgc tcaactagtt cgacgagata cctgtcacgt 60

cccaccagca ataggtatca ggtaactctg tcgaccaagg ttaggacaac tcttcatgct 120

gaagaaacta caactcgtgc atctggagtc tagacaaata gatggttgat actagtttcg 180

aaaagagctt tagagtcaaa gacaagtcac tttcattagt tgtaacaaat aacaagactg 240

cagttatagt ataggagtac aagagaaaat aattacataa aaaacatata agttagctag 300

tgaagaggct acctatatat gttttacgcc atgataaacc tcatggtacc tacaccttat 360

cttgaaaaga caaaagcagg ggaaattgtg aaagacgcgg tattagaaat aagcataggg 420

ttagattttt ttcgatacaa ctataggcaa cctaatagtt aaaaccatcc aaatgtaagt 480

cgataaaaac ttaaaggaaa cagacgaaat tgagataatg cataacatta agtacttagt 540

ctatgcagtt tgttcacaat tcgttatcat cacaacatac ttgaagtcaa tatacagatt 600

gtcaaaaaga ttgttttttt ggcctaaaat actatagcgt tgacgcgtct atattagcca 660

tagaattgga attggaggat ctaatctctt ccagcagggt gaatttccta tctgatatta 720

tccacttgct gatgataata cttttcaaca aaaagtttgg tcacaaaata ctattttctt 780

attctccctc cacctattgt acctctcctt aaagaaaaat attcgacaac ttgctacaac 840

tcaacgcctt cttctccatc ttataaggac ctaacgtagc attatgttgc ccataccatt 900

cacttgagct agcactcggc tttaggcgaa gtagctctgc acacacgtgt acaacatggc 960

ctcgtggact gcaaggcaaa acaagaagtt cgaagaggct ttggcgttat atgacagaga 1020

cacacctgat cgttggcata acattgcaag gtgtgtaggt ggaaaatcag ctgcagaagt 1080

gaagaggcat tatgaagtac ttgtgaagga tatcatgcaa atagaaaatg gtcaagtacc 1140

tttaccaaat tacaaagccg cggctgaaac caataacaga tcaggttatg ccaatgaaca 1200

taggtattaa acatcaaaac taatctttta ttaattttgt acaagtcatt gatacgttct 1260

gtattcgatt cccatatggt tcgtgtcatt ttcctattca tggtactgtt tatgtttatc 1320

accatctatc catttttagt aactttacaa gccaaagact agtcacactc cttgttgaca 1380

cttttattct aaagaataaa tgcttatagt ttcaactctc tgcatccacg attgtttctt 1440

gttcctcgat aaaagaaaat gtacgcaaac aagtaactaa tgcttatgaa catgtctata 1500

tgttgttgaa caggcttctg aagaacctga agttacagtg agaacaaatt tcgcggccat 1560

gctgaataac atcacaagtt gtctgttata tttgcaaatg caaaaggcat aaattgtgaa 1620

tttatgtctt aagtttttga agcaagcaaa atgtattatg actattgttg ttcatctcta 1680

tccatttaac ttttgtttta ctgttgtaat gtttctcaaa cgcataatat aaagtctact 1740

tcactttcat gttatagtgt aatatgtttg ataaaataaa atcaaaatta cacgttttat 1800

agtctgaatt caatggatcg taattctcaa ttcatttgtt cttttttaga tacttgtaaa 1860

atattcaagt acactttcac aatgagattg tcacacggtt cagtatcata acaatcaaat 1920

ctcacaccgt acatgagttc atatgtgatt ctttaggtta ctattagaga ttacgaattc 1980

gagctatggg agctaacaac atcatgagtt catatgtgat tctttaggtt attattagag 2040

attacgaatt cgagctagga gctaacatct aaaaaatagt tcatatgtga ttctttaggt 2100

tattattaga gattacgaat tcgagctagg agctaacatc taaaaaataa aagtaggaac 2160

gtaaaaatgg agatgcgggt atcgatcccc gtacctctcg catgctaagc gagcgctcta 2220

ccatctgagc tacatcccct ttcacatgct gtgcatctta tattaaaata aatatatcaa 2280

ataaacatct agtaaacaga aatagttgta caaggaagga acatttagca cttttttttt 2340

ttaagaatca aaactgctta aatgacatat ttatgtatta taccattata aaactaaatc 2400

aattcagact cgctttaaaa aattgtatac aacgtctata tattaatttt tatctcatag 2460

gatcaatcac aaaattaatt ttgtaataca tcaacaaggt agattaaaat tttacaggaa 2520

cacctttaca tgtagaaaat tattattgcc attgaatacc aagaacaaaa aaaaatatac 2580

tattttctct tgtacatagt taggtaacta acaaactaat caaactcccc tcagcttttt 2640

tcgagcttct gtctgtcaaa ggttcgagaa tcaatgaaag atcatcaccg tctttgttat 2700

aggcgagcaa attaacagac gaaaataaaa aaggaaaaat cttgaacacc caacacatct 2760

cctcttcgtc ttcttcgtat gaccaaattt gtaccaattc ttgagtctca taactataag 2820

caacaatttt atgtgccaaa acttgtagat ataatacctg taaatcctta ttgaatgcac 2880

atggcttcac tgtgtaccct gtttcaaacc ccaatcttac aaaatgatca agcttgaagt 2940

catttcctct gaaactccat atgaaaactc cattgtcaac tggatcacag aattgtatta 3000

ctccgtcttc ttccgatgat tcgtataaaa actgagagtg gtaaacatct tttcttctgg 3060

aattgttttt cggcagtgaa aacagtgtaa atactttgaa atctatgtcg tataccaaca 3120

aacataaact ccatatccag tacaattttc ttttctcgcg agaaaaaaca ctcgaattat 3180

agcattgccc tcgaataaat ccatctgatt ggaggagatg ggaattcaaa atttgaattt 3240

gggtttcccg ccacttccat gaattcgatc cgaagataat acaattcacc atttgggtat 3300

cgaaatcaat ttcttcccag aagaaagcga taacttgaaa cgaatttaac tcaaaaacaa 3360

gacttgaaca agtgggtctt gaaatatgat gagaaattgg tagagaaatt aactggttaa 3420

gaacagggga tgaaacagag tatgtccaga acccattgtt gttattttct gaagcgtaaa 3480

gaagaacccc attacaagaa tcgatcaaat aagggaattt atgagtggaa gatgaatggg 3540

aagtacaaaa tttggggatt tttttctggg ttaagtcgtg taacttcatg aaaactgatt 3600

cttgataagt tttgtgtttg aggaagatca caagaccagt tgaagttgtg agagggtaac 3660

aatggcggat attggtaatc gaatttcgcc atgttttcga aacaatcttc aattggatga 3720

gagatttaaa gggaatttta gtgataattg attcaactat atgatttgat aaattcccaa 3780

gctctatcat ctcttcttct tcttcttgct ctgtttccat ggcaatcgcc attgctaatc 3840

ccctctgttt tttgaagtta aatttcatat tcaatcaatt aattaatact taaaaggtat 3900

ttatagaaac cctaataaac tagctatggc ggttaattat caattacagt taatttattt 3960

gtgtttgata attaattaat tgattaattg tacttggtat gcaagattat aagtaagtaa 4020

atgcttgatg agcaagcatt aaatttctat agttaaacgg gttgataact aatcacctat 4080

attttctcac ttaaatattt atttttggct atttatttac aatttttttg gctagttata 4140

tttttggtaa agtttaatca tgtaatatat gacatatgta tatatataat ttatagcgta 4200

actttcacat ataacaaata aaaaatttat atttgcatgc tatagtaaag tttgcgtaat 4260

tgcgctccat agcaaacata aaaattgtat aattcgctat acatatacag ttgaagcaaa 4320

ttgtataaaa cgaagtgtat aaaacaagaa agagaaagac acttgggcag agaactgtat 4380

aaaaacgaag tgtataaaac atattgtatt ataagtgtat agaacgatta tatacaattt 4440

gaatttgtat aaaatgagaa tgaaagaaaa caaaagagac ttgacaagga atatacaatt 4500

gaatcgaatt gtataaaacg agaaatggag aaattagata caatttaaaa attgtataaa 4560

acgagaaaga gtgaaaggca aaaaaaaacg ggatagggaa atatttttat tgtataatca 4620

taagtgtata ggacaaatag caaaactata gagcgctaat atgattttat atttattatt 4680

cctaaaattt actattctaa aaattatttt aaaatactca taagtttgta ttatcattct 4740

ataatgaaaa gtgtttcgtg aaaagtcatt acaagaacat aattcaaatt cattaaaatg 4800

aatttgaaaa aaaaggaagg aaataaaaga aaaatgtcat tgatttgtgt atgaaaaact 4860

actattatta cttgtaattg gaagtgactt cgaagaacaa gctaggataa aaatagtata 4920

taaagtattt atataaataa ataaatttta ttcaacaaat taataatgaa atttaatcaa 4980

aaattttgaa tgataggtgt ttttattcag ctactagtaa tagtgtgatt atttaagagt 5040

gtatttatat attattttat gcatgtatta attatataga gattgattat ttattcactc 5100

ccataaggta tctatttctt atttcatgca taaaaagata cataaattct ttcataactt 5160

ttacatattt taatagtcat gcaagtttct taaatatata catgaattag atatttttct 5220

atacctatcg aacataaatg tcgtacaaat tatataaaaa ttaatataaa aataacttat 5280

ctcttatcag ctatcaaaca acttgtaatt tcacaaataa aatatgaaaa tgttaatagg 5340

taaactataa atatgttata tttaatagaa tctaacacta ataaacaatt caaagtaaag 5400

aaaaaaaatt gctataacgt tgctctcaat taaatcagat gtcataattt tgacgtttca 5460

caaaaataga atttttttta aagaaaattg tcatatttgt tgaagtaaca tgtgtgccca 5520

tatgtggtcc tgttattatt agaaagtagg taaaagaagt atgaaaaata ctatattata 5580

attggatctc atgatataat aagtgaacat aatttggcac agattaagaa aagcatcctc 5640

aattaaggat attaatagaa tatttattca tgccataaaa ggaaagaata gtataattgc 5700

atgtgttttt ttaattacat gtttgcaaga ctcaaattgt tatgtttatt agtcggtgct 5760

taccttaaaa taataaatgc atatattatt tgcacatcaa ctttttctat ttaattatag 5820

ttgactaaca tgtactttta tattctaata aagtctacct aattatgata agacaattct 5880

ccctcattaa cttttgtttg ttttatgtta tataaatttt agtgattcaa atcttaatta 5940

ttagtgtata tttgttatat tactttataa ccatgcactt aacgatttta aatagatttg 6000

aataatttat aatattttta gttattcaga aaaatacaca agtatctttt taatctatgt 6060

tcaaaaaact tcaaagacac acttatacta tactaatgtt ctattacttc cctaaattta 6120

ttttatagta attttctacc tctttttgat ctacgtggta ttgggttgaa aaaaaaataa 6180

accagttttg atcccacaaa atagtgtcac gtagaccaaa agggggaagg aaaattacat 6240

ataaaataag ttcaggggaa taatatgacc ttggtatagt ataaatgtgt ctttagattt 6300

tggcataggt tgagaaagta tttgagcatt atccctattt ataatcattt acaatcatca 6360

ttacccataa taatcgttca tcataattat tagttatcac ttctggcttc aactaatcac 6420

aaaactttta aaaaatcatt aatatgtcat ataaaaatta tttataaaaa gtaataattt 6480

taccgttact ttgatatttc atcaaaaaca tacaaaaaaa cttttaatgg atgcatttcc 6540

caagttatca tataaattga taatgaccac aagtagcctt ctgattcctc ctatggtcca 6600

ttatcttcat gtttcaacat ttatagcttc tgcttcttgc atagcttatt taaataaaat 6660

tacattttaa tatatatata gaaaaagtga aacaaacttt catagaccca atttaataat 6720

ataccatttg tttccattaa ttttaaaagt gaatggaaat taaccacact cacaaacaaa 6780

tatgatgaaa taacatattt tattcatcac tatacctaaa tacaatcata tttttcactt 6840

atttttattt atgaattttc ttcgcgactt gatgatttta atatctaatg gatgtatgtt 6900

gatttgaata tccttgcaat gtgttttatt attacgtatg tcatgtattg ttatttaata 6960

tgttgatttt ttaataaaaa aaatatttat aaaaaattat tcgagaatat aataatacta 7020

gaaatattta aaataatcgc ttctttcaat aaaacaatat atcttgctac atgtacacct 7080

ttgagtttca cttgcttatt atattgttta tttttggcga tttatttttt tatatatatt 7140

attgaacatg atccaaagaa gatccaccat tgttcgtttc caagcttatt tattaattta 7200

tttattttcc tctattttaa tttgacggtg aaatatgatt atcaattctt tcttccattt 7260

taataatcaa aattacttaa aatatccaaa atgattgtaa ttttacaaaa aataaaatga 7320

aattaaataa tattaagttt tcactttgcc tttaatatta agtttttacg gaagcaacca 7380

ttatactaaa caataggaag aaaacaatat aaatccgaac aaataaataa ttatcggttg 7440

atatcatgat tttcaaatat aaaaaagatt aattcatatt cagtcatatt tcttaaaaat 7500

aaataaggac acaaagaaaa acatgagaat tattaataat atagaatgaa tgaagcatgt 7560

tgaatttata tattatataa aatatatttt ttaattagtc tcaattaata tttatatttt 7620

caattttaaa aattcataaa taaaaaatta aatttacata aaataaaata aattaataca 7680

tatatttaac actatataaa acaaatatat gtcacgtaaa ttttgtttac tgttacttta 7740

ttgacgtata ttaaatgtta gtaataggat aaaaactttc aatcattgca aggaaaagat 7800

ggtgtatgca ttgtatttag caattaattt tatgaataat attaatgcct aatttatgta 7860

tttttatatt tttttaagtg acaataaatt aatgtgacgc aattatgctg tctcaattgt 7920

cgagcccctg caatattact gataacgcaa atgccttgaa tgttgattaa tctgtaaaaa 7980

aagataaagc aagtaccaaa gcaaataaca gaaattagac atgacaagag acctcaccca 8040

gatgtaagtt tgtactaatt ttatttttta tcttaaatta tttatcatat cttgataagt 8100

tgattagcgt agacacttaa atttgtataa agttgaacaa ataggcacac ttgaccattt 8160

tatatggtaa tttatatctt acgtgtacta tgtcacatat ggctcatgtg tctacctgtt 8220

taattttata taagtttaag tttttacttg tgtgcattca aaattgagat gcataattgt 8280

gtaactaatt taaattaaat gagacattta ccatgtcttg tgtcatttaa aaataaaaaa 8340

tacttactat tagagaaaat ctctctcgtt aaaatttgaa tggtgacaaa taaattgaaa 8400

cgagaaaaaa gaatttgcta aggagaaaag catggagttt gcaggtagaa ggtgacatag 8460

aattaggagt actcataatt tgcatcattc attgggcaga agttgaataa taataataaa 8520

agctgatagc agagtcaaat cagattgcag aatcgcacta tttttgcgtc attttcgact 8580

ctatgtgtat gtaacacggt aaactaaaag attcaccaac tccacacccc aaaacatatg 8640

gtgaatcttt tgaagactag agtagggata tcgccaaata tgtcctaaga agctgaggac 8700

taagtttaat atatccataa tttaaattta tagatggagt actagataat tttatattaa 8760

actaattttt atcaaatcca acctaattca ttttttaagt agtattcaaa ctttgcccat 8820

ttctaagtca tacttatatt tacttttgac tttttttccc tatagtttgg agcttaagtt 8880

ctgagtcaga gttaaaactt gaaattaaag ttggattgct cactggttaa ggttggatct 8940

caggtcgaga tcggtttatt gttcaggact cgagacaaag tcatctcatg ccccaaatcc 9000

ctgtcgggta tcgagtccca agtaagaatt gaatgtcgaa tcacgagtct tgaatttcaa 9060

attaagcccg aagctcaatt atcgagtcta aaatctcgag tcagaggcga ggtcgagtct 9120

agagtcctgg tcagggttag atttcaagta aggatcaaaa ttgagactca aaatctcaaa 9180

ttttataact ccattcaaaa tcaaatcaag tctgagatcc ggactcaagg atcgaattag 9240

agaaataaag ttgttcattc taacccccta atgggaaaat gaaggggggg gggttggtag 9300

ggcaataatg aataaatggg gtggtgggca tggggccaat gtttgcaggg cgtgggttag 9360

gactgaacat gacattgtcg gttgttttct tatgcacact tcatccagta tctctttgct 9420

gtttcccact ttctctctct tctctctctc tctcccttct tctttagctc cgtttgaagc 9480

aaccatgttg tctcttgttt cttacatccc atgtttcttc ctccccaaca acaccacttt 9540

ttattcctta aaatctctcc ttctttttta acaacatagt ccattgtttt tccttctcta 9600

cttctagggc cccccatcct cctttcattt cttcaatttc acaccccccc acataacatt 9660

agacataggg ttagggtttc tttttttttt ttttaaatta aaaaaaaaaa aaaaatcttt 9720

tcatttggtg gtgtaaagga ggaaaaaaag atgtgttttt caggttatca aaatatcgct 9780

cttgtctccc cgggggtggc cgcgccggga ggacttggtg agggctccac cacacaataa 9840

ttccactact ggatctaata aattgaacaa gggaactagt acagtgagag gtagatatcg 9900

tgagtgcttg aaaaatcatg ctgctaatat tggtggaaat gttacggatg gttgtggtga 9960

gtttatgcct agtggtgagt ttacttaatt atatcttcaa acaggtagcg gcggaggaag 10020

tggggcaaca gactcatcaa gtgaagaact taatttcaac acatatcatc atcaacaagc 10080

aacatcagta cctccacaac aaccatttat gttggctaag aaaagattta ggacaaaatt 10140

tagtcaagaa cagaaggaaa aaatgttgga atttgctgaa aaattaggtt ggagaattcc 10200

aagagaagat gatgctgaag tacaaagatt ttgctcacaa gttggagtca agagacaggt 10260

ttttaaggtt tggatgcata ataataagaa cccttttttt gctaaaaaaa atataataca 10320

agaagatcaa ccttaattaa ttttgtttaa ttaaggtttt gagggaaaaa atgaaattaa 10380

atttcttggg atttggttta tgcttatgta atcttactag gaatcgtacg tagctagttt 10440

tttttgtttt tttttggggg gggtctatgt agtattattg taactatatg gaaccactta 10500

attagatttt tatttttatt aattgttgta ctttggaacc tttattcatc tgaaattttt 10560

cagctatttc ttacacttct cttgtcactg aaatttagac cactcatgat gccgctttat 10620

cgaggttagc gaggtagaga taaaatttac gtatgtaaac tttaaaaaaa atgtgctaga 10680

tatgttgttg taatatgagc caagttgtat atcgaattct ttagctctca agatagagta 10740

aagtctgtgt atgaagttag ttaattgatg atgttgaggg agaaacatga gttatttatt 10800

tttttgaaat ataaaaatga caagtaaaag cgaaaaataa atgttttttt taggaatgga 10860

aggatattta attaatagag agccgttgga tgaaaagaaa gaatgaatct tagtcgtcta 10920

atttgaatta tttgaaagag tagtagtttt ttttatcctt agtcttgtcg aatataaaag 10980

cttttcctat ttaaatttat ttattagttt gtgtattgtc tatttcaata aattatgatg 11040

cttcttggag tttctgtcac cattttaaat gtttgtttcc tattttggga atagtttgct 11100

gcttcttttt ctcactattt gaggcttata cttatagagc atatcacccc aatcttctcc 11160

atgtgattat caatgttgag tgtcataaac attagataat acaaattttc gacattcgat 11220

ttaagattat gattttaaaa ttatttttag atatgtaatt ttattatcat gattttaaag 11280

tttaaattat tcaagaagca cgattgaaat tttagttgat ttatatttta taaaaaaaat 11340

atatcattga attttgaaaa aagaaagacc catgctcatc gtgaattgac agtttatgct 11400

atgtgaaata attatactaa gaaatttctc tttatattcc ttttagttgt tctgaaattc 11460

tttttctcaa agttaaaaaa taagaatttt gattaacgtt ttacaataca ttttattatt 11520

atattgataa taataagaac tttaactaat tttattagat gttgcgatca gatacatagc 11580

cctacgcgac ttatcgagac gttgagtgat gtatctgaaa ctgatcagat gcaatatatt 11640

gagacgtaat gtattgagat attttggaaa gcaattgaat tccactaaat taaaaaaaag 11700

tatatatata ataaaagaga gtaagaatat aatatattta actctttaac acgtatgcaa 11760

tgttatgtta aattccctta ataattcact taaaaggtta tttgaacttt gattaagatg 11820

ttgtgaataa tagatcatag ttcaagtcca ttcatttctt atcaaatttt aatcatcctc 11880

atttttgtct ctttttcatg tgctttgacg tttgattgct tctatcctta tcttcactta 11940

tttgatataa aaaaacaaaa aatacattaa aaatgtaggt cactaaattc tacataaaac 12000

acatattcaa agtaatacta caaaaaacta aag 12033

<210> 5
<211> 1034
<212> DNA
<213> Solanum lycopersicum

<400> 5
agaaggtaca ctcgaagctt tgaaatgtgc tgcttgtaat tgtcatagga attttcatag 60

aaaagaaata cctaacaacg ttggcgctgg tgttggtggt ggtgttggtg gtgataataa 120

taataatgcg gggattatgg ttgtgcatcc attacagctg ccacaaccac taccttctcc 180

gataccgtcg ttgaatcatc atcaccacca tcaccatcaa cacggaggaa gatcaatttg 240

gactactatg ccacctcagc ctgtcaaaat ggccttcgga ggtaatgtgt ttagctcatc 300

taaaaatttt aatgaaatct catttgctca atttgaatta ccatgccacc tcagcctatc 360

aaaatgggct ttggggggtt tcgatttaac attcgattat ttcgtctaat agtttaaatt 420

gtgagacata ttttttttat ttacttgtac taattatttc attttatgtg tctttgtttc 480

acattgcact aaatttagaa ggtgaatatc taatttgaat attgtgattt taaaccttat 540

taatcatttc ttaaaatagt atcattaaga ataattagca tcatgagaat gttagatttc 600

aaagcttact taaatactca tatgataaga tataaacata cattactaga aataaataaa 660

aaaaattcac cttgaattcg tgagaaattt gtattatcga actgattttt ccactcattt 720

ttcacctaac cagctcacgg ccgcgagaaa cacatctaac ttcctaattt tttcgcgaga 780

aaatgttctg gtcattcctt actaattcaa tcaaaatatc tctaaatatt tctccatgaa 840

agaaataata attttttagt aatgatattt actgaagaca caaattaaaa taaattatta 900

cgcttaatta tgtcttcaac acgttgttag tacctttttg tcgactcgaa cctaagactc 960

ttgatatcat gatgaatcgt atgataagtt aaaatcatta aagtgaacta tatacctaca 1020

tttttatttt ttta 1034

<210> 6
<211> 873
<212> DNA
<213> Solanum lycopersicum

<400> 6
atggcattag gtggtggtaa tgaggaaaaa gagatgagaa tttcaggttt tcaaaatata 60

gatcttgtct cacctggtgg tgcagcgcca gacgacatcg tcagcgctgc accacacaat 120

aattccacta ctggatctaa taaattgaac aagggaacta gtacagtgag aggtagatat 180

cgtgagtgct tgaaaaatca tgctgctaat attggtggaa atgttacgga tggttgtggt 240

gagtttatgc ctagtggtga agaaggtaca ctcgaagctt tgaaatgtgc tgcttgtaat 300

tgtcatagga attttcatag aaaagaaata cctaacaacg ttggcgctgg tgttggtggt 360

ggtgttggtg gtgataataa taataatgcg gggattatgg ttgtgcatcc attacagctg 420

ccacaaccac taccttctcc gataccgtcg ttgaatcatc atcaccacca tcaccatcaa 480

cacggaggaa gatcaatttg gactactatg ccacctcagc ctgtcaaaat ggccttcgga 540

ggtagcggcg gaggaagtgg ggcaacagac tcatcaagtg aagaacttaa tttcaacaca 600

tatcatcatc aacaagcaac atcagtacct ccacaacaac catttatgtt ggctaagaaa 660

agatttagga caaaatttag tcaagaacag aaggaaaaaa tgttggaatt tgctgaaaaa 720

ttaggttgga gaattccaag agaagatgat gctgaagtac aaagattttg ctcacaagtt 780

ggagtcaaga gacaggtttt taaggtttgg atgcataata ataagaaccc ttcttctgct 840

aaaaaaaata taatacaaga agatcaacct taa 873

<210> 7
<211> 23

<212>  DNA
<213>  Artificial Sequence

<220>
<223>  primer sequence

<400>  7
tgttgcccca cttcctccgc cgc                                              23


<210>  8
<211>  23
<212>  DNA
<213>  Artificial Sequence

<220>
<223>  primer sequence

<400>  8
cggcggagga agtggggcaa cag                                              23


<210>  9
<211>  20
<212>  DNA
<213>  Artificial Sequence

<220>
<223>  primer sequence

<400>  9
gctttgaaat gtgctgcttg                                                  20


<210>  10
<211>  22
<212>  DNA
<213>  Artificial Sequence

<220>
<223>  primer sequence

<400>  10
ttcagcatca tcttctcttg ga                                               22


<210>  11
<211>  1405
<212>  DNA
<213>  Solanum lycopersicum

<400>  11
gtttcccact ttctctctct tctctctctc tctcccttct tctttagctc cgtttgaagc      60

aaccatgttg tctcttgttt cttacatccc atgtttcttc ctccccaaca acaccacttt     120

ttattcctta aaatctctcc ttctttttta acaacatagt ccattgtttt tccttctcta     180

cttctagggc cccccatcct cctttcattt cttcaatttc acaccccccc acataacatt     240

agacataggg ttagggtttc tttttttttt tttttaaatt aacaaaaaaa aaaaaatcat     300

ggcattaggt ggtggtaatg aggaaaaaga gatgagaatt tcaggttttc aaaatataga     360

tcttgtctca cctggtggtg cagcgccaga cgacatcgtc agcgctgcac cacacaataa 420

ttccactact ggatctaata aattgaacaa gggaactagt acagtgagag gtagatatcg 480

tgagtgcttg aaaaatcatg ctgctaatat tggtggaaat gttacggatg gttgtggtga 540

gtttatgcct agtggtgaag aaggtacact cgaagctttg aaatgtgctg cttgtaattg 600

tcataggaat tttcatagaa aagaaatacc taacaacgtt ggcgctggtg ttggtggtgg 660

tgttggtggt gataataata ataatgcggg gattatggtt gtgcatccat tacagctgcc 720

acaaccacta ccttctccga taccgtcgtt gaatcatcat caccaccatc accatcaaca 780

cggaggaaga tcaatttgga ctactatgcc acctcagcct gtcaaaatgg ccttcggagg 840

tagcggcgga ggaagtgggg caacagactc atcaagtgaa gaacttaatt tcaacacata 900

tcatcatcaa caagcaacat cagtacctcc acaacaacca tttatgttgg ctaagaaaag 960

atttaggaca aaatttagtc aagaacagaa ggaaaaaatg ttggaatttg ctgaaaaatt 1020

aggttggaga attccaagag aagatgatgc tgaagtacaa agattttgct cacaagttgg 1080

agtcaagaga caggttttta aggtttggat gcataataat aagaaccctt cttctgctaa 1140

aaaaaatata atacaagaag atcaacctta attaattttg tttaattaag gttttgaggg 1200

aaaaaatgaa attaaatttc ttgggatttg gtttatgctt atgtaatctt actaggaatc 1260

gtacgtagct agtttttttt gttttttttt tgggggggtc tatgtagtat tattgtaact 1320

atatggaacc acttaattag atttttattt ttattaattg ttgtactttg gaacctttat 1380

tcatctgaaa attttcagct atttc 1405

<210> 12
<211> 2572
<212> DNA
<213> Solanum lycopersicum

<400> 12
aagcttattt attaatttat ttattttcct ctattttaat ttgacggtga aatatgatta 60

tcaattcttt cttccatttt aataatcaaa attacttaaa atatccaaaa tgattgtaat 120

tttacaaaaa ataaaatgaa attaaataat attaagtttt cactttgcct ttaatattaa 180

gtttttacgg aagcaaccat tatactaaac aataggaaga aaacaatata aatccgaaca 240

aataaataat tatcggttga tatcatgatt ttcaaatata aaaaagatta attcatattc 300

agtcatattt cttaaaaata aataaggaca caaagaaaaa catgagaatt attaataata 360

tagaatgaat gaagcatgtt gaatttatat attatataaa atatattttt taattagtct 420

caattaatat ttatattttc aattttaaaa attcataaat aaaaaattaa atttacataa 480

aataaaataa attaatacat atatttaaca ctatataaaa caaatatatg tcacgtaaat 540

tttgtttact gttactttat tgacgtatat taaatgttag taataggata aaaactttca 600

atcattgcaa ggaaaagatg gtgtatgcat tgtatttagc aattaatttt atgaataata 660
ttaatgccta atttatgtat ttttatattt ttttaagtga caataaatta atgtgacgca 720
attatgctgt ctcaattgtc gagcccctgc aatattactg ataacgcaaa tgccttgaat 780
gttgattaat ctgtaaaaaa agataaagca agtaccaaag caaataacag aaattagaca 840
tgacaagaga cctcacccag atgtaagttt gtactaattt tattttttat cttaaattat 900
ttatcatatc ttgataagtt gattagcgta gacacttaaa tttgtataaa gttgaacaaa 960
taggcacact tgaccatttt atatggtaat ttatatctta cgtgtactat gtcacatatg 1020
gctcatgtgt ctacctgttt aattttatat aagtttaagt ttttacttgt gtgcattcaa 1080
aattgagatg cataattgtg taactaattt aaattaaatg agacatttac catgtcttgt 1140
gtcatttaaa aataaaaaat acttactatt agagaaaatc tctctcgtta aaatttgaat 1200
ggtgacaaat aaattgaaac gagaaaaaag aatttgctaa ggagaaaagc atggagtttg 1260
caggtagaag gtgacataga attaggagta ctcataattt gcatcattca ttgggcagaa 1320
gttgaataat aataataaaa gctgatagca gagtcaaatc agattgcaga atcgcactat 1380
ttttgcgtca ttttcgactc tatgtgtatg taacacggta aactaaaaga ttcaccaact 1440
ccacacccca aaacatatgg tgaatctttt gaagactaga gtagggatat cgccaaatat 1500
gtcctaagaa gctgaggact aagtttaata tatccataat ttaaatttat agatggagta 1560
ctagataatt ttatattaaa ctaattttta tcaaatccaa cctaattcat tttttaagta 1620
gtattcaaac tttgcccatt tctaagtcat acttatattt acttttgact ttttttccct 1680
atagtttgga gcttaagttc tgagtcagag ttaaaacttg aaattaaagt tggattgctc 1740
actggttaag gttggatctc aggtcgagat cggtttattg ttcaggactc gagacaaagt 1800
catctcatgc cccaaatccc tgtcgggtat cgagtcccaa gtaagaattg aatgtcgaat 1860
cacgagtctt gaatttcaaa ttaagcccga agctcaatta tcgagtctaa aatctcgagt 1920
cagaggcgag gtcgagtcta gagtcctggt cagggttaga tttcaagtaa ggatcaaaat 1980
tgagactcaa aatctcaaat tttataactc cattcaaaat caaatcaagt ctgagatccg 2040
gactcaagga tcgaattaga gaaataaagt tgttcattct aaccccctaa tgggaaaatg 2100
aagggggggg ggttggtagg gcaataatga ataaatgggg tggtgggcat ggggccaatg 2160
tttgcagggc gtgggttagg actgaacatg acattgtcgg ttgttttctt atgcacactt 2220
catccagtat ctctttgctg tttcccactt tctctctctt ctctctctct ctcccttctt 2280
ctttagctcc gtttgaagca accatgttgt ctcttgtttc ttacatccca tgtttcttcc 2340
tccccaacaa caccactttt tattccttaa aatctctcct tcttttttaa caacatagtc 2400
cattgttttt ccttctctac ttctagggcc ccccatcctc ctttcatttc ttcaatttca 2460

caccccccca cataacatta gacatagggt tagggtttct tttttttttt ttttaaatta 2520

acaaaaaaaa aaaaatcatg gcattaggtg gtggtaatga ggaaaacccg gg 2572

<210> 13
<211> 31
<212> DNA
<213> Artificial Sequence

<220>
<223> primer sequence

<400> 13
ctccccggga agctttgaaa tgtgctgctt g 31

<210> 14
<211> 31
<212> DNA
<213> Artificial Sequence

<220>
<223> primer sequence

<400> 14
ctcctcgagt tcagcatcat cttctcttgg a 31

<210> 15
<211> 500
<212> DNA
<213> Solanum lycopersicum

<400> 15
ctccccggga agctttgaaa tgtgctgctt gtaattgtca taggaatttt catagaaaag 60

aaatacctaa caacgttggc gctggtgttg gtggtggtgt tggtggtgat aataataata 120

atgcggggat tatggttgtg catccattac agctgccaca accactacct tctccgatac 180

cgtcgttgaa tcatcatcac caccatcacc atcaacacgg aggaagatca atttggacta 240

ctatgccacc tcagcctgtc aaaatggcct tcggaggtag cggcggagga agtggggcaa 300

cagactcatc aagtgaagaa cttaatttca acacatatca tcatcaacaa gcaacatcag 360

tacctccaca acaaccattt atgttggcta agaaaagatt taggacaaaa tttagtcaag 420

aacagaagga aaaaatgttg gaatttgctg aaaaattagg ttggagaatt ccaagagaag 480

atgatgctga actcgaggag 500

<210> 16
<211> 1651
<212> DNA
<213> Solanum lycopersicum

<400> 16
atggcattag gtggtggtaa tgaggaaaaa gagatgagaa tttcaggttt tcaaaatata 60

gatcttgtct cacctggtgg tgcagcgcca gacgacatcg tcagcgctgc accacacaat 120

aattccacta ctggatctaa taaattgaac aagggaacta gtacagtgag aggtagatat 180

cgtgagtgct tgaaaaatca tgctgctaat attggtggaa atgttacgga tggttgtggt 240

gagtttatgc ctagtggtga agaaggtaca ctcgaagctt tgaaatgtgc tgcttgtaat 300

tgtcatagga attttcatag aaaagaaata cctaacaacg ttggcgctgg tgttggtggt 360

ggtgttggtg gtgataataa taataatgcg gggattatgg ttgtgcatcc attacagctg 420

ccacaaccac taccttctcc gataccgtcg ttgaatcatc atcaccacca tcaccatcaa 480

cacggaggaa gatcaatttg gactactatg ccacctcagc ctgtcaaaat ggccttcgga 540

ggtaatgtgt ttagctcatc taaaaatttt aatgaaatct catttgctca atttgaatta 600

ccatgccacc tcagcctatc aaaatgggct ttggggggtt tcgatttaac attcgattat 660

ttcgtctaat agtttaaatt gtgagacata ttttttttat ttacttgtac taattatttc 720

attttatgtg tctttgtttc acattgcact aaatttagaa ggtgaatatc taatttgaat 780

attgtgattt taaaccttat taatcatttc ttaaaatagt atcattaaga ataattagca 840

tcatgagaat gttagatttc aaagcttact taaatactca tatgataaga tataaacata 900

cattactaga aataaataaa aaaaattcac cttgaattcg tgagaaattt gtattatcga 960

actgattttt ccactcattt ttcacctaac cagctcacgg ccgcgagaaa cacatctaac 1020

ttcctaattt tttcgcgaga aaatgttctg gtcattcctt actaattcaa tcaaaatatc 1080

tctaaatatt tctccatgaa agaaataata attttttagt aatgatattt actgaagaca 1140

caaattaaaa taaattatta cgcttaatta tgtcttcaac acgttgttag tacctttttg 1200

tcgactcgaa cctaagactc ttgatatcat gatgaatcgt atgataagtt aaaatcatta 1260

aagtgaacta tatacctaca tttttatttt tttatttact taattatatc ttcaaacagg 1320

tagcggcgga ggaagtgggg caacagactc atcaagtgaa gaacttaatt tcaacacata 1380

tcatcatcaa caagcaacat cagtacctcc acaacaacca tttatgttgg ctaagaaaag 1440

atttaggaca aaatttagtc aagaacagaa ggaaaaaatg ttggaatttg ctgaaaaatt 1500

aggttggaga attccaagag aagatgatgc tgaagtacaa agattttgct cacaagttgg 1560

agtcaagaga caggttttta aggtttggat gcataataat aagaaccctt cttctgctaa 1620

aaaaaatata atacaagaag atcaacctta a 1651

<210> 17
<211> 617
<212> DNA
<213> Solanum lycopersicum

<400> 17
atggcattag gtggtggtaa tgaggaaaaa gagatgagaa tttcaggttt tcaaaatata 60

gatcttgtct cacctggtgg tgcagcgcca gacgacatcg tcagcgctgc accacacaat 120

aattccacta ctggatctaa taaattgaac aagggaacta gtacagtgag aggtagatat 180

cgtgagtgct tgaaaaatca tgctgctaat attggtggaa atgttacgga tggttgtggt 240

gagtttatgc ctagtggtga tttacttaat tatatcttca aacaggtagc ggcggaggaa 300

gtggggcaac agactcatca agtgaagaac ttaatttcaa cacatatcat catcaacaag 360

caacatcagt acctccacaa caaccattta tgttggctaa gaaaagattt aggacaaaat 420

ttagtcaaga acagaaggaa aaaatgttgg aatttgctga aaaattaggt tggagaattc 480

caagagaaga tgatgctgaa gtacaaagat tttgctcaca agttggagtc aagagacagg 540

tttttaaggt ttggatgcat aataataaga acccttcttc tgctaaaaaa aatataatac 600

aagaagatca accttaa 617

<210> 18
<211> 717
<212> DNA
<213> Solanum pimpinellifolium

<400> 18
ccacacaata attccactac tggatctaat aaattgaaca agggaactag tacagtgaga 60

ggtagatatc gtgagtgctt gaaaaatcat gctgctaata ttggtggaaa tgttacggat 120

ggttgtggtg agtttatgcc tagtggtgaa gaaggtacac tcgaagcttt gaaatgtgct 180

gcttgtaatt gtcataggaa ttttcataga aaagaaatac ctaacaacgt tggcgttggc 240

attggtgttg gtggtgataa taataataat gcggggatta tggttgtgca tccattacag 300

ctgccacaac cactaccttc tccgataccg tcgttgaatc atcatcacca ccatcaccat 360

caacacggag gaagatcaat ttggactact atgccacctc agcctgtcaa aatggccttc 420

ggaggtagcg gcggaggaag tggggcaaca gactcatcaa gtgaagaact taatttcaac 480

acatatcatc atcaacaagc aacatcagta cctccacaac aaccatttat gttggctaag 540

aaaagattta ggacaaaatt tagtcaagaa cagaaggaaa aaatgttgga atttgctgaa 600

aaattaggtt ggagaattcc aagagaagat gatgctgaag tacaaagatt ttgctcacaa 660

gttggagtca agagacagac cactcatgat gccgctttat cgaggttagc gaggtag 717

<210> 19
<211> 238
<212> PRT
<213> Solanum pimpinellifolium

<400> 19

Pro His Asn Asn Ser Thr Thr Gly Ser Asn Lys Leu Asn Lys Gly Thr
1               5                   10                  15

Ser Thr Val Arg Gly Arg Tyr Arg Glu Cys Leu Lys Asn His Ala Ala

            20                  25                  30

Asn Ile Gly Gly Asn Val Thr Asp Gly Cys Gly Glu Phe Met Pro Ser
        35                  40                  45

Gly Glu Glu Gly Thr Leu Glu Ala Leu Lys Cys Ala Ala Cys Asn Cys
    50                  55                  60

His Arg Asn Phe His Arg Lys Glu Ile Pro Asn Asn Val Gly Val Gly
65                  70                  75                  80

Ile Gly Val Gly Gly Asp Asn Asn Asn Asn Ala Gly Ile Met Val Val
                85                  90                  95

His Pro Leu Gln Leu Pro Gln Pro Leu Pro Ser Pro Ile Pro Ser Leu
            100                 105                 110

Asn His His His His His His His Gln His Gly Gly Arg Ser Ile Trp
        115                 120                 125

Thr Thr Met Pro Pro Gln Pro Val Lys Met Ala Phe Gly Gly Ser Gly
    130                 135                 140

Gly Gly Ser Gly Ala Thr Asp Ser Ser Ser Glu Glu Leu Asn Phe Asn
145                 150                 155                 160

Thr Tyr His His Gln Gln Ala Thr Ser Val Pro Pro Gln Gln Pro Phe
                165                 170                 175

Met Leu Ala Lys Lys Arg Phe Arg Thr Lys Phe Ser Gln Glu Gln Lys
            180                 185                 190

Glu Lys Met Leu Glu Phe Ala Glu Lys Leu Gly Trp Arg Ile Pro Arg
        195                 200                 205

Glu Asp Asp Ala Glu Val Gln Arg Phe Cys Ser Gln Val Gly Val Lys
    210                 215                 220

Arg Gln Thr Thr His Asp Ala Ala Leu Ser Arg Leu Ala Arg
225                 230                 235

<210> 20
<211> 1014
<212> DNA
<213> Solanum peruvianum

<400> 20
aaaaaagaaa aaaaaaatag ctacgtacga ttcctagtaa gattacataa gcataaacca 60

aatcccaaga aatttaattt cattttttcc cctcaaaacc ttaattaaac aaaattaatt 120

aaggttgatc ttcttgtatt atattttttt tagcagaaga agggttctta ttattatgca 180

tccaaacctt aaaaacctgt ctcttgactc caacttgtga gcaaaatctt tgtacttcag 240

catcatcttc tcttggaatt ctccaaccta atttttcagc aaattccaac attttttcct 300

tttgttcttg actaaatttt gtcctaaatc ttttcttagc caacataaat ggttgttgtg 360

gaggtactga tgttgcttgt tgatgatgat atgtgttgaa attaagttct tcacttgatg 420

agtctgttgc cccacttcct ccgccgctac ctccgaaggc cattttgaca ggctgaggtg 480

gcatagtagt ccaaattgat cttcctccgt gttgatggtg atggtggtgg tgatgattca 540

acgatggtat cggagaaggt agtggttgtg gtagttgtaa tggatgcaca accataatcc 600

ccgcattatt attattatca ccaccaacac caacaccacc accaacgcca acaacgttgt 660

taggtatttc ttttctatga aaattcctat gacaattaca agcagcacat ttcaaagctt 720

cgagtgtacc ttcttcacca ctaggcataa actcaccaca tccatccgta acatttccac 780

caatattagc agcatgattt ttcaagcact cacgatatct acctctcact gtactagttg 840

ccttgttcaa tttattagat ccagtagtgg aattattgtg tggtgcagcg ccaacgacga 900

cgtcgtctgg cgctgcacca ccaccaggtg agacaagatc tatattttga aaacctgaaa 960

ttctcatctc tttttcctca ttaccaccac ctaatgccat gatttttatt tttt 1014

<210> 21
<211> 282
<212> PRT
<213> Solanum peruvianum

<400> 21

Met Arg Ile Ser Gly Phe Gln Asn Ile Asp Leu Val Ser Pro Gly Gly
1               5                   10                  15

Gly Ala Ala Pro Asp Asp Val Val Val Gly Ala Ala Pro His Asn Asn
            20                  25                  30

Ser Thr Thr Gly Ser Asn Lys Leu Asn Lys Ala Thr Ser Thr Val Arg
        35                  40                  45

Gly Arg Tyr Arg Glu Cys Leu Lys Asn His Ala Ala Asn Ile Gly Gly
    50                  55                  60

Asn Val Thr Asp Gly Cys Gly Glu Phe Met Pro Ser Gly Glu Glu Gly
65                  70                  75                  80

Thr Leu Glu Ala Leu Lys Cys Ala Ala Cys Asn Cys His Arg Asn Phe
                85                  90                  95

His Arg Lys Glu Ile Pro Asn Asn Val Val Gly Val Gly Gly Gly Val
            100                 105                 110

Gly Val Gly Gly Asp Asn Asn Asn Asn Ala Gly Ile Met Val Val His
        115                 120                 125

Pro Leu Gln Leu Pro Gln Pro Leu Pro Ser Pro Ile Pro Ser Leu Asn
    130                 135                 140

His His His His His His His Gln His Gly Gly Arg Ser Ile Trp Thr
145                 150                 155                 160

Thr Met Pro Pro Gln Pro Val Lys Met Ala Phe Gly Gly Ser Gly Gly
                165                 170                 175

Gly Ser Gly Ala Thr Asp Ser Ser Ser Glu Glu Leu Asn Phe Asn Thr
            180                 185                 190

Tyr His His Gln Gln Ala Thr Ser Val Pro Pro Gln Gln Pro Phe Met
        195                 200                 205

Leu Ala Lys Lys Arg Phe Arg Thr Lys Phe Ser Gln Glu Gln Lys Glu
    210                 215                 220

Lys Met Leu Glu Phe Ala Glu Lys Leu Gly Trp Arg Ile Pro Arg Glu
225                 230                 235                 240

Asp Asp Ala Glu Val Gln Arg Phe Cys Ser Gln Val Gly Val Lys Arg
                245                 250                 255

Gln Val Phe Lys Val Trp Met His Asn Asn Lys Asn Pro Ser Ser Ala
            260                 265                 270

Lys Lys Asn Ile Ile Gln Glu Asp Gln Pro
        275                 280

<210>  22
<211>  855
<212>  DNA
<213>  Solanum tuberosum phureja

<400>  22
atggaattag ctggtggtga ggaaaaggag atgagaatgt caggttatca ttcacttgat      60

caaaatatag atcttctctc acccaccggc ggtggaggtg gaggtggtgg tgcagcgcca     120

ggtgatggcg ctgcaccaca cactaattcc accgctggat ctaataaatt taacaaggga     180

actactacag caagagcaag gtaccgtgag tgtctgaaaa atcatgctgc taatattggt     240

ggaaatgtta ctgatggatg tggtgagttt atgcctagtg gtggagaagg tacacttgaa 300

gctttgaaat gtgctgcttg taattgtcat cggaattttc atagaaaaga acagcctaat 360

aataatgccg ggattatggt tgtgcatcca ttacagctac cacaatcact gccttctccg 420

ataccatcgt tgaatcacca ccaccaccat caccatcaac acggaggagg aagatcaatt 480

tggactacta tgccacctca gcctgtcaaa atggcctttg gaggtagcgg gggaggaagt 540

ggggcaacag actcatcaag tgaagaactt aatttcaata catatcatca tcaacaagca 600

acatcggtac atccacaaca accatttatg ttggctaaga aaagatttag gacaaaattt 660

agtcaagaac aaaaggaaaa aatgttggaa tttgctgaaa aattaggttg gagaattcca 720

agagaagatg atactgaagt acaaagattc tgctcacaag ttggagtcaa gagacaggtt 780

tttaaggttt ggatgcataa taataagaat ccttcttctg ctaaaaaaaa cataatacaa 840

gaagatcaac cttaa 855

<210> 23
<211> 284
<212> PRT
<213> Solanum tuberosum phureja

<400> 23

Met Glu Leu Ala Gly Gly Glu Glu Lys Glu Met Arg Met Ser Gly Tyr
1               5                   10                  15

His Ser Leu Asp Gln Asn Ile Asp Leu Leu Ser Pro Thr Gly Gly Gly
            20                  25                  30

Gly Gly Gly Gly Gly Ala Ala Pro Gly Asp Gly Ala Ala Pro His Thr
        35                  40                  45

Asn Ser Thr Ala Gly Ser Asn Lys Phe Asn Lys Gly Thr Thr Thr Ala
    50                  55                  60

Arg Ala Arg Tyr Arg Glu Cys Leu Lys Asn His Ala Ala Asn Ile Gly
65                  70                  75                  80

Gly Asn Val Thr Asp Gly Cys Gly Glu Phe Met Pro Ser Gly Gly Glu
                85                  90                  95

Gly Thr Leu Glu Ala Leu Lys Cys Ala Ala Cys Asn Cys His Arg Asn
            100                 105                 110

Phe His Arg Lys Glu Gln Pro Asn Asn Asn Ala Gly Ile Met Val Val
        115                 120                 125

His Pro Leu Gln Leu Pro Gln Ser Leu Pro Ser Pro Ile Pro Ser Leu

130                 135                 140

Asn His His His His His His His Gln His Gly Gly Gly Arg Ser Ile
145                 150                 155                 160

Trp Thr Thr Met Pro Pro Gln Pro Val Lys Met Ala Phe Gly Gly Ser
                165                 170                 175

Gly Gly Gly Ser Gly Ala Thr Asp Ser Ser Ser Glu Glu Leu Asn Phe
            180                 185                 190

Asn Thr Tyr His His Gln Gln Ala Thr Ser Val His Pro Gln Gln Pro
        195                 200                 205

Phe Met Leu Ala Lys Lys Arg Phe Arg Thr Lys Phe Ser Gln Glu Gln
    210                 215                 220

Lys Glu Lys Met Leu Glu Phe Ala Glu Lys Leu Gly Trp Arg Ile Pro
225                 230                 235                 240

Arg Glu Asp Asp Thr Glu Val Gln Arg Phe Cys Ser Gln Val Gly Val
                245                 250                 255

Lys Arg Gln Val Phe Lys Val Trp Met His Asn Asn Lys Asn Pro Ser
            260                 265                 270

Ser Ala Lys Lys Asn Ile Ile Gln Glu Asp Gln Pro
        275                 280

<210>  24
<211>  879
<212>  DNA
<213>  Solanum melongena

<400>  24
atggcattaa ccggtgagga aaaggaaatg agaatgtcag gttccttagg atatcattca      60

cttgatcaaa acatagatca cactgtccca ccaaacattc aagatcaatc taccggcgga     120

ggtagtggtg cagcgccagt tgctggcgtt gcaccacata attcctccgc cggatctaat     180

aaattcaata aggtaacttc atcaagagct aggtaccgtg agtgtctgaa aaatcatgct     240

gctaacattg gtggaaatgt taccgatgga tgtggtgaat tcatgcctag tggagaagaa     300

ggaactcttg aggctttgaa atgtgctgcg tgtaattgtc accgtaattt tcacagaaaa     360

gaactgccca acaatggtga taataataat aacaacccgg ggattatggt tgtccatcca     420

ctgcaacttc ctcaaccact tcctactcca ataccgtcga tgaatcacca tcatcaccat     480

caccaccaac acagaggtcg atcaatctgg actaccatgc cacctcagcc tgtcaaaatg     540

gccttcggag ccagcggggg aagtggggca acagactcat caagtgaaga acttaatttc 600

aacacttatc atcatcaaca ggcaacctca gttcctccac aacaaccatt tttgttggct 660

aagaaaaggt ttaggacaaa attcagtcaa gaacaaaagg aaaaaatgtt ggaatttgca 720

gagaaattag gatggagaat tccaagagaa gatgatactg aagtacaaag tttctgctca 780

caagttggag tgaagagaca ggtatttaag gtttggatgc ataataataa gaacccttct 840

gctaaaaaga acataaatca agaagatcaa caaccttaa 879

<210> 25
<211> 292
<212> PRT
<213> Solanum melongena

<400> 25

Met Ala Leu Thr Gly Glu Glu Lys Glu Met Arg Met Ser Gly Ser Leu
1               5                   10                  15

Gly Tyr His Ser Leu Asp Gln Asn Ile Asp His Thr Val Pro Pro Asn
            20                  25                  30

Ile Gln Asp Gln Ser Thr Gly Gly Gly Ser Gly Ala Ala Pro Val Ala
        35                  40                  45

Gly Val Ala Pro His Asn Ser Ser Ala Gly Ser Asn Lys Phe Asn Lys
    50                  55                  60

Val Thr Ser Ser Arg Ala Arg Tyr Arg Glu Cys Leu Lys Asn His Ala
65                  70                  75                  80

Ala Asn Ile Gly Gly Asn Val Thr Asp Gly Cys Gly Glu Phe Met Pro
                85                  90                  95

Ser Gly Glu Glu Gly Thr Leu Glu Ala Leu Lys Cys Ala Ala Cys Asn
            100                 105                 110

Cys His Arg Asn Phe His Arg Lys Glu Leu Pro Asn Asn Gly Asp Asn
        115                 120                 125

Asn Asn Asn Asn Pro Gly Ile Met Val Val His Pro Leu Gln Leu Pro
    130                 135                 140

Gln Pro Leu Pro Thr Pro Ile Pro Ser Met Asn His His His His His
145                 150                 155                 160

His His Gln His Arg Gly Arg Ser Ile Trp Thr Thr Met Pro Pro Gln
                165                 170                 175

Pro Val Lys Met Ala Phe Gly Ala Ser Gly Gly Ser Gly Ala Thr Asp
            180                 185                 190

Ser Ser Ser Glu Glu Leu Asn Phe Asn Thr Tyr His His Gln Gln Ala
        195                 200                 205

Thr Ser Val Pro Pro Gln Gln Pro Phe Leu Leu Ala Lys Lys Arg Phe
    210                 215                 220

Arg Thr Lys Phe Ser Gln Glu Gln Lys Glu Lys Met Leu Glu Phe Ala
225                 230                 235                 240

Glu Lys Leu Gly Trp Arg Ile Pro Arg Glu Asp Asp Thr Glu Val Gln
                245                 250                 255

Ser Phe Cys Ser Gln Val Gly Val Lys Arg Gln Val Phe Lys Val Trp
            260                 265                 270

Met His Asn Asn Lys Asn Pro Ser Ala Lys Lys Asn Ile Asn Gln Glu
        275                 280                 285

Asp Gln Gln Pro
    290

<210>  26
<211>  20
<212>  DNA
<213>  Artificial Sequence

<220>
<223>  primer sequence

<400>  26
ggggattatg gttgtgcatc                                                   20

<210>  27
<211>  20
<212>  DNA
<213>  Artificial Sequence

<220>
<223>  primer sequence

<400>  27
gatgagtctg ttgccccact                                                   20

<210>  28
<211>  729
<212>  DNA
<213>  Solanum lycopersicum

<400>  28

atggggtatg caatcatcag cactattgga cttcttttct tggggaagct gcttgagccc 60

atctggggtt ctagggaatt cttaaagttc atctttattg tcaacttttt gacatctgtc 120

tttgttttca tcacagccat atcattgtac tacgtaacaa gactggaaat ttatctctat 180

atgcccattt ccggcttcca aggagttctt tctggtttct tagttggtgt caagcaaatc 240

atgcccgacc aggagttgtc tattttgaaa ctgaaagcaa agtggttgcc ttcccttgca 300

ttgttgttct caattgctat aagctttttt acagcagatt cagtatctta ccttccaaca 360

atagtatttg ggacatattt aggctggatt tacctaagat attggcagaa gaaaccagag 420

acaaaactaa agggtgaccc aagtgatgaa ttttccttct cttctttctt ccctgaattt 480

ttgagaccag taattgatcc aattgcaaca atatttgaac ggatgctttg tggtagaaga 540

tctgagactt ctaatgagga aaggggctat actctaggtg gttccacatt gcctggttct 600

gatcccattg aagcatcgag gaggagggaa agaggtgcga gagcacttga agaaagattg 660

gcagctgagc ttttggcttc tggaaagaag ggtgaagaat cagaacgagg tgtgaatgat 720

aatgtgtag 729

<210> 29
<211> 20
<212> DNA
<213> Artificial Sequence

<220>
<223> primer sequence

<400> 29
aagtggttgc cttcccttgc 20

<210> 30
<211> 24
<212> DNA
<213> Artificial Sequence

<220>
<223> primer sequence

<400> 30
gttttgtctc tggtttcttc tgcc 24

<210> 31
<211> 41
<212> DNA
<213> Artificial Sequence

<220>
<223> primer sequence

<400> 31
ctcggcgcgc cgatccaaag aagatccacc attgttcgtt t 41

<210> 32
<211> 41
<212> DNA
<213> Artificial Sequence

<220>
<223> primer sequence

<400> 32
ctcggcgcgc caaatggtga cagaaactcc aagaagcatc a 41


<210> 33
<211> 4965
<212> DNA
<213> Solanum lycopersicum

<400> 33
ggcgcgccga tccaaagaag atccaccatt gttcgtttcc aagcttattt attaatttat 60

ttattttcct ctattttaat ttgacggtga aatatgatta tcaattcttt cttccatttt 120

aataatcaaa attacttaaa atatccaaaa tgattgtaat tttacaaaaa ataaaatgaa 180

attaaataat attaagtttt cactttgcct ttaatattaa gtttttacgg aagcaaccat 240

tatactaaac aataggaaga aaacaatata aatccgaaca aataaataat tatcggttga 300

tatcatgatt ttcaaatata aaaaagatta attcatattc agtcatattt cttaaaaata 360

aataaggaca caaagaaaaa catgagaatt attaataata tagaatgaat gaagcatgtt 420

gaatttatat attatataaa atatattttt taattagtct caattaatat ttatattttc 480

aattttaaaa attcataaat aaaaaattaa atttacataa aataaaataa attaatacat 540

atatttaaca ctatataaaa caaatatatg tcacgtaaat tttgtttact gttactttat 600

tgacgtatat taaatgttag taataggata aaaactttca atcattgcaa ggaaaagatg 660

gtgtatgcat tgtatttagc aattaatttt atgaataata ttaatgccta atttatgtat 720

ttttatattt ttttaagtga caataaatta atgtgacgca attatgctgt ctcaattgtc 780

gagcccctgc aatattactg ataacgcaaa tgccttgaat gttgattaat ctgtaaaaaa 840

agataaagca agtaccaaag caaataacag aaattagaca tgacaagaga cctcacccag 900

atgtaagttt gtactaattt tattttttat cttaaattat ttatcatatc ttgataagtt 960

gattagcgta gacacttaaa tttgtataaa gttgaacaaa taggcacact tgaccatttt 1020

atatggtaat ttatatctta cgtgtactat gtcacatatg gctcatgtgt ctacctgttt 1080

aattttatat aagtttaagt ttttacttgt gtgcattcaa aattgagatg cataattgtg 1140

taactaattt aaattaaatg agacatttac catgtcttgt gtcatttaaa aataaaaaat 1200

acttactatt agagaaaatc tctctcgtta aaatttgaat ggtgacaaat aaattgaaac 1260

gagaaaaaag aatttgctaa ggagaaaagc atggagtttg caggtagaag gtgacataga 1320

attaggagta ctcataattt gcatcattca ttgggcagaa gttgaataat aataataaaa 1380

gctgatagca gagtcaaatc agattgcaga atcgcactat ttttgcgtca ttttcgactc 1440

tatgtgtatg taacacggta aactaaaaga ttcaccaact ccacacccca aaacatatgg 1500

tgaatctttt gaagactaga gtagggatat cgccaaatat gtcctaagaa gctgaggact 1560

aagtttaata tatccataat ttaaatttat agatggagta ctagataatt ttatattaaa 1620

ctaattttta tcaaatccaa cctaattcat tttttaagta gtattcaaac tttgcccatt 1680

tctaagtcat acttatattt acttttgact ttttttccct atagtttgga gcttaagttc 1740

tgagtcagag ttaaaacttg aaattaaagt tggattgctc actggttaag gttggatctc 1800

aggtcgagat cggtttattg ttcaggactc gagacaaagt catctcatgc cccaaatccc 1860

tgtcgggtat cgagtcccaa gtaagaattg aatgtcgaat cacgagtctt gaatttcaaa 1920

ttaagcccga agctcaatta tcgagtctaa aatctcgagt cagaggcgag gtcgagtcta 1980

gagtcctggt cagggttaga tttcaagtaa ggatcaaaat tgagactcaa aatctcaaat 2040

tttataactc cattcaaaat caaatcaagt ctgagatccg gactcaagga tcgaattaga 2100

gaaataaagt tgttcattct aaccccctaa tgggaaaatg aagggggggg ggttggtagg 2160

gcaataatga ataaatgggg tggtgggcat ggggccaatg tttgcagggc gtgggttagg 2220

actgaacatg acattgtcgg ttgttttctt atgcacactt catccagtat ctctttgctg 2280

tttcccactt tctctctctt ctctctctct ctcccttctt ctttagctcc gtttgaagca 2340

accatgttgt ctcttgtttc ttacatccca tgtttcttcc tccccaacaa caccactttt 2400

tattccttaa aatctctcct tcttttttaa caacatagtc cattgttttt ccttctctac 2460

ttctagggcc ccccatcctc ctttcatttc ttcaatttca caccccccca cataacatta 2520

gacatagggt tagggtttct tttttttttt ttttaaatta acaaaaaaaa aaaaatcatg 2580

gcattaggtg gtggtaatga ggaaaaagag atgagaattt caggttttca aaatatagat 2640

cttgtctcac ctggtggtgc agcgccagac gacatcgtca gcgctgcacc acacaataat 2700

tccactactg gatctaataa attgaacaag ggaactagta cagtgagagg tagatatcgt 2760

gagtgcttga aaaatcatgc tgctaatatt ggtggaaatg ttacggatgg ttgtggtgag 2820

tttatgccta gtggtgaaga aggtacactc gaagctttga aatgtgctgc ttgtaattgt 2880

cataggaatt ttcatagaaa agaaatacct aacaacgttg gcgctggtgt tggtggtggt 2940

gttggtggtg ataataataa taatgcgggg attatggttg tgcatccatt acagctgcca 3000

caaccactac cttctccgat accgtcgttg aatcatcatc accaccatca ccatcaacac 3060

ggaggaagat caatttggac tactatgcca cctcagcctg tcaaaatggc cttcggaggt 3120

aatgtgttta gctcatctaa aaattttaat gaaatctcat ttgctcaatt tgaattacca 3180

tgccacctca gcctatcaaa atgggctttg gggggtttcg atttaacatt cgattatttc 3240

gtctaatagt ttaaattgtg agacatattt tttttattta cttgtactaa ttatttcatt 3300

ttatgtgtct ttgtttcaca ttgcactaaa tttagaaggt gaatatctaa tttgaatatt 3360

gtgattttaa accttattaa tcatttctta aaatagtatc attaagaata attagcatca 3420

tgagaatgtt agatttcaaa gcttacttaa atactcatat gataagatat aaacatacat 3480

tactagaaat aaataaaaaa aattcacctt gaattcgtga gaaatttgta ttatcgaact 3540

gatttttcca ctcatttttc acctaaccag ctcacggccg cgagaaacac atctaacttc 3600

ctaatttttt cgcgagaaaa tgttctggtc attccttact aattcaatca aaatatctct 3660

aaatatttct ccatgaaaga aataataatt ttttagtaat gatatttact gaagacacaa 3720

attaaaataa attattacgc ttaattatgt cttcaacacg ttgttagtac ctttttgtcg 3780

actcgaacct aagactcttg atatcatgat gaatcgtatg ataagttaaa atcattaaag 3840

tgaactatat acctacattt ttattttttt atttacttaa ttatatcttc aaacaggtag 3900

cggcggagga agtggggcaa cagactcatc aagtgaagaa cttaatttca acacatatca 3960

tcatcaacaa gcaacatcag tacctccaca acaaccattt atgttggcta agaaaagatt 4020

taggacaaaa tttagtcaag aacagaagga aaaaatgttg gaatttgctg aaaaattagg 4080

ttggagaatt ccaagagaag atgatgctga agtacaaaga ttttgctcac aagttggagt 4140

caagagacag gtttttaagg tttggatgca taataataag aacccttctt ctgctaaaaa 4200

aaatataata caagaagatc aaccttaatt aatttgtttt aattaaggtt ttgagggaaa 4260

aaatgaaatt aaatttcttg ggatttggtt tatgcttatg taatcttact aggaatcgta 4320

cgtagctagt tttttttgtt ttttttttgg gggggtctat gtagtattat tgtaactata 4380

tggaaccact taattagatt tttattttta ttaattgttg tactttggaa cctttattca 4440

tctgaaaatt ttcagctatt tcttacactt ctcttgtcac tgaaatttag accactcatg 4500

atgccgcttt atcgaggtta gcgaggtaga gataaaattt acgtatgtaa actttaaaaa 4560

aaatgtgcta gatatgttgt tgtaatatga gccaagttgt atatcgaatt ctttagctct 4620

caagatagag taaagtctgt gtatgaagtt agttaattga tgatgttgag ggagaaacat 4680

gagttattta tttttttgaa atataaaaat gacaagtaaa agcgaaaaat aaatgttttt 4740

tttaggaatg gaaggatatt taattaatag agagccgttg gatgaaaaga aagaatgaat 4800

cttagtcgtc taatttgaat tatttgaaag agtagtagtt ttttttatcc ttagtcttgt 4860

cgaatataaa agcttttcct atttaaattt atttattagt ttgtgtattg tctatttcaa 4920

taaattatga tgcttcttgg agtttctgtc accatttggc gcgcc 4965

<210> 34
<211> 1600
<212> DNA
<213> Solanum melongena

<400>  34
gaaatagggt tagggtttct taaatcttaa ttagggtttt aatctggtaa ttagaaaaaa      60

ataatggcat taaccggtga ggaaaaggaa atgagaatgt caggttcctt aggatatcat     120

tcacttgatc aaaacataga tcacactgtc ccaccaaaca ttcaagatca atctaccggc     180

ggaggtagtg gtgcagcgcc agttgctggc gttgcaccac ataattcctc cgccggatct     240

aataaattca ataaggtaac ttcatcaaga gctaggtacc gtgagtgtct gaaaaatcat     300

gctgctaaca ttggtggaaa tgttaccgat ggatgtggtg aattcatgcc tagtggagaa     360

gaaggaactc ttgaggcttt gaaatgtgct gcgtgtaatt gtcaccgtaa ttttcacaga     420

aaagaactgc ccaacaatgg tgataataat aataacaacc cggggattat ggttgtccat     480

ccactgcaac ttcctcaacc acttcctact ccaataccgt cgatgaatca ccatcatcac     540

catcaccacc aacacagagg tcgatcaatc tggactacca tgccacctca gcctgtcaaa     600

atggccttcg gaggtaatgt gattatttca tctaaaagtt taaattgtta gatgaaatac     660

atttttattt acttccatta tttcatttta tgtgtctata tttcacatga cgctaaattt     720

agaagcataa aaatgtgtat atatagtgaa tattgtgatt ctagacctta cttatcattt     780

ctatgaagta tcattaagaa caattagcat gaaaatctta gatttaaatg cttaaatact     840

cataaactac tagaaacaaa tttggtcgtg aattagttag aaatttgtgc tatgtaactt     900

attttttcca ctcattttca accaaattag ttcacggaaa tcaaaaaatg catgatgaga     960

aacaattgtc gattcttaat ctttttcata tgtaaaacat tactagtttc ttcctcaccg    1020

attccactaa aatataacat ttttagtgtg aaattccatg attttttagt agtgagcttt    1080

tacctttttt tagtgaacaa aacatgtcac ctcaaacgta agacgttagt ctgttttgat    1140

atgatgatta ctgtacaata atgtcaccta gaattgaaaa aatcattacc atatatacat    1200

acatacattt tttttcaatt atttacttaa ttatgtcttc aacagccagc gggggaagtg    1260

gggcaacaga ctcatcaagt gaagaactta atttcaacac ttatcatcat caacaggcaa    1320

cctcagttcc tccacaacaa ccatttttgt tggctaagaa aaggtttagg acaaaattca    1380

gtcaagaaca aaaggaaaaa atgttggaat ttgcagagaa attaggatgg agaattccaa    1440

gagaagatga tactgaagta caaagtttct gctcacaagt tggagtgaag agacaggtat    1500

ttaaggtttg gatgcataat aataagaacc cttctgctaa aaagaacata aatcaagaag    1560

atcaacaacc ttaattaatt aaattaaggt caaatgaaat                          1600

<210>  35
<211>  879
<212>  DNA
<213>  Solanum melongena

<400>  35

atggcattaa ccggtgagga aaaggaaatg agaatgtcag gttccttagg atatcattca 60

cttgatcaaa acatagatca cactgtccca ccaaacattc aagatcaatc taccggcgga 120

ggtagtggtg cagcgccagt tgctggcgtt gcaccacata attcctccgc cggatctaat 180

aaattcaata aggtaacttc atcaagagct aggtaccgtg agtgtctgaa aaatcatgct 240

gctaacattg gtggaaatgt taccgatgga tgtggtgaat tcatgcctag tggagaagaa 300

ggaactcttg aggctttgaa atgtgctgcg tgtaattgtc accgtaattt tcacagaaaa 360

gaactgccca acaatggtga taataataat aacaacccgg ggattatggt tgtccatcca 420

ctgcaacttc ctcaaccact tcctactcca ataccgtcga tgaatcacca tcatcaccat 480

caccaccaac acagaggtcg atcaatctgg actaccatgc cacctcagcc tgtcaaaatg 540

gccttcggag ccagcggggg aagtggggca acagactcat caagtgaaga acttaatttc 600

aacacttatc atcatcaaca ggcaacctca gttcctccac aacaaccatt tttgttggct 660

aagaaaaggt ttaggacaaa attcagtcaa gaacaaaagg aaaaaatgtt ggaatttgca 720

gagaaattag gatggagaat tccaagagaa gatgatactg aagtacaaag tttctgctca 780

caagttggag tgaagagaca ggtatttaag gtttggatgc ataataataa gaacccttct 840

gctaaaaaga acataaatca agaagatcaa caaccttaa 879

<210> 36
<211> 31
<212> DNA
<213> Artificial Sequence

<220>
<223> primer sequence

<400> 36
ctcggatcca agctttgaaa tgtgctgcgt g 31

<210> 37
<211> 34
<212> DNA
<213> Artificial Sequence

<220>
<223> primer sequence

<400> 37
ctcggtaccg agctcagtat catcttctct tgga 34

<210> 38
<211> 473
<212> DNA
<213> Solanum melongena

<400> 38
ctcggatcca agctttgaaa tgtgctgcgt gtaattgtca ccgtaatttt cacagaaaag 60

aactgcccaa caatggtgat aataataata acaacccggg gattatggtt gtccatccac 120

tgcaacttcc tcaaccactt cctactccaa taccgtcgat gaatcaccat catcaccatc 180

accaccaaca cagaggtcga tcaatctgga ctaccatgcc acctcagcct gtcaaaatgg 240

ccttcggagc cagcggggga agtggggcaa cagactcatc aagtgaagaa cttaatttca 300

acacttatca tcatcaacag gcaacctcag ttcctccaca acaaccattt ttgttggcta 360

agaaaaggtt taggacaaaa ttcagtcaag aacaaaagga aaaaatgttg gaatttgcag 420

agaaattagg atggagaatt ccaagagaag atgatactga gctcggtacc gag 473

<210> 39
<211> 2561
<212> DNA
<213> Solanum melongena

<400> 39
gattctcatt taatgtacca aattatcttt ttagtctcat gatttgaaac atgtcacgtg 60

aaaaataata attaaaactt ttcaaaaaat attaattatt tttctaaaca gcgcaaaaga 120

aaaagtagat tactcatttt gaaatgaagg gagtaaaatt tttcgtttta tctctaatgt 180

gtaaatcatt ttttatgatt tattttttgg attgcaaagt tcaaaatgtt ttcttcaaac 240

tcctttgcat atatagattt gagactagaa aaaaagatca caaaatttaa taatatttat 300

tatattttac acacctcttt aatttaaaat tataaaatta aaataaaact ccatactcaa 360

tcagcctaag aatttataaa atgaaatgaa gaaaacatat ctaagaattt ataaaaataa 420

aacgaagaaa acatctttta gactaagcag aaatgcatac taaaataaac cctctcgatc 480

tctctggtgt caatttatgt gacatctttt gaaatataaa agttaaacga gtttttcttt 540

actacaacct ttacataatc ttgtttgtac ctttaagaaa tttaattttg gaatttaaat 600

tgcgccacaa aaattaaact cataattata aaaaaattaa tatttttgct tttgagattg 660

atatatttga gttgagggtc ttccagaaac agtatcttca tctgtataat atagtggtaa 720

gatctgtata aattttacca tctctacact tcacttatag aatttcacta gatatattat 780

tgtcgttttt cttagaatag aaagaaaggg agtggttacc attgattgta tgaaagaaaa 840

ggttgcttgt gggaaagcat ggagtttgca gagttttgca ggtagaaggt gacatatata 900

caatacaatt actcataatt ttcataattc atttggtaga acgtgataat aaaagctgag 960

cagagtcaaa tcagattgca ggacagaaga atcgcactat ttttgcgtca ttttcatcta 1020

acacgctaaa ataaaagatt caccaaaatt aacaagttac tgcttattac aaccaagtaa 1080

cttatatgat ggaaaaagtt tttacctcag agagtgagta aaattttcca tgaactcaaa 1140

atataacaat caccaaattg gaaactgaaa aaagtgatcg tatgatctaa aatttgatgg 1200

ctcattcaat tttttcaaaa ctttatggtt ttaagttgat ttctaaaatg atctttaatt 1260

taactcaatt taagttattt tcttagattt acttttatct aggctttact acttaagatt 1320

tactttcatt ttttatatgg gttggggctc aagtctgaag ttagatcttg agcatagtcg 1380

aagttggatc gctagtcagg attgagtctc aagtcaagtt tggttcggtg cttgggacaa 1440

agtctaagtt agggtctcat gtcctgaact agggtcgagt ttcaagtgaa gatcaggtcc 1500

taggttagag tcgatagttg aaatcttatt cttataagat cccaagttag ggtcaaggct 1560

caggcctcaa tactcaaatc tcgagttagg gggtgggaga aagggagttt cgaggctgaa 1620

tctgaagtca gatcaggtct caagtgatta cgaaccaagt tttgggttag gtacaagctc 1680

gaattccaag tttttaaact caagatcaag atagagcatt gagtcgaaga tcaaatcgag 1740

gtcgagtgat ctcaagaaat actacaagag aattttagat acatatatat taaattttaa 1800

tttaaaataa ggagtgccta attaggaaaa atataaagta agtcattcta atttgtaccc 1860

acctccccaa agagggtggt gggggtgggg caataatgaa ggagggtggt gggaatgggg 1920

ccaatgttgg cagggcgtgg gagaggactg aacatgacat tgtcggttgt tttcttatgc 1980

tcagtccttt tgtttcttaa caatccataa tctcaccaca gtttatcttt gctgttccgc 2040

actactctct ccctttctta tctcctctct cttttccttt tgaaattatc tctctttttc 2100

ttttagctcc gtttcaagca accatgttgt ctcttgtttc ttacattcta tgttgcctcc 2160

tccccaacac accacaacct taaaacaaac ctcatcactt taaatttctc tttcctccat 2220

ctctaactca ctactgtttc ttaatctcaa tttcatgcat ttttaaggtg gtggtccgcc 2280

ggcttttgct tcttctaagg tagtagttct agggccccat cttctttatt tccagcttaa 2340

tttcttcttc tgttttaccc tttttccttt tctctttctc tctcacccca cataccatta 2400

agtagaatca gaaggtttca tcatttttgt tagttacatc tatttttctt cagtgttttg 2460

ttagaataag aaatagggtt agggtttctt aaatcttaat tagggtttta atctggtaat 2520

tagaaaaaaa taatggcatt aaccggtgag gaaaaggaaa t 2561

<210> 40
<211> 29
<212> DNA
<213> Solanum melongena

<400> 40
atggcattaa ccggtgagga aaaggaaat 29

<210> 41
<211> 1153
<212> DNA
<213> Solanum melongena

<400> 41
gagaggcaac cccatctctc actcgtctga aacacgcatc accattaaag ctctctgctt 60

tctgtgatca cttggcacta tgagtatgag tatgtatacg gggttcacca ggctttgcaa 120

gggtcttgcg gttgtgcttg tgggctgtca cattgttgtt cagattcttc cttctgctct 180

ttcgtatctt gctcttatcc ctgccaagac tattccattt gcatggaatc ttataacagc 240

tggttacact gaacaaacag tgtatggggc aatcatcagc actatcggac ttctttacct 300

ggggaagctg cttgagccca tttggggttc tagggaattc ttgaagttca tctttattgt 360

caactttttg acatctgtct gtgtttttat cacagccata tcattgtact acataacaag 420

actggaaatt tatctctata tgcccatttc cggcttccaa ggagttcttt ctggtttctt 480

agttggcgtc aagcaaatca tgcctgacca ggagttgtct attttgaaac tgaaagcaaa 540

gtggttgcct tcctttgcat tgttgttctc aattgctata agctttttta cagcagattt 600

agtatcatat cttccaacag tagtatttgg gacatattta ggctggattt acctgagata 660

ctggcagaag aaaccagaga caaaattaaa gggtgaccca agtgatgaat tttccttctc 720

ttctttcttc cctgaatttt tgagaccagt aattgatcca attgcaacaa tatttgagcg 780

gatgctttgt ggtagaagat ctgagacttc taatgaggaa aggggatata ctctaggtgg 840

ttccacattg cctggttctg atcccattga agcatcgagg aggagggaaa gaggtgccag 900

agcacttgaa gaaagattgg cagctgagcg tttggctgct ggaaagaagg ctgaagaatc 960

agaaagagat gtgaatgata atgtgtaaaa gctggtcaat atatatgtaa tgcgctttct 1020

ttgcagaaag aacgtttgtc cgctagaagc tagaaattgt gcactttttg gcagtgtact 1080

cttttggatt cttgacggaa gttgtcgtct gttgtttatc atccctatgg taaatgtaca 1140

tttttctcct tcc 1153

<210> 42
<211> 729
<212> DNA
<213> Solanum lycopersicum

<400> 42
atggggtatg caatcatcag cactattgga cttcttttct tggggaagct gcttgagccc 60

atctggggtt ctagggaatt cttaaagttc atctttattg tcaacttttt gacatctgtc 120

tttgttttca tcacagccat atcattgtac tacgtaacaa gactggaaat ttatctctat 180

atgcccattt ccggcttcca aggagttctt tctggtttct tagttggtgt caagcaaatc 240

atgcccgacc aggagttgtc tattttgaaa ctgaaagcaa agtggttgcc ttcccttgca 300

ttgttgttct caattgctat aagctttttt acagcagatt cagtatctta ccttccaaca 360

atagtatttg ggacatattt aggctggatt tacctaagat attggcagaa gaaaccagag 420

acaaaactaa agggtgaccc aagtgatgaa ttttccttct cttctttctt ccctgaattt 480

ttgagaccag taattgatcc aattgcaaca atatttgaac ggatgctttg tggtagaaga 540

tctgagactt ctaatgagga aaggggctat actctaggtg gttccacatt gcctggttct 600

gatcccattg aagcatcgag gaggagggaa agaggtgcga gagcacttga agaaagattg     660

gcagctgagc ttttggcttc tggaaagaag ggtgaagaat cagaacgagg tgtgaatgat     720

aatgtgtag                                                             729


<210>  43
<211>  20
<212>  DNA
<213>  Artificial Sequence

<220>
<223>  primer sequence

<400>  43
aaagaactgc ccaacaatgg                                                  20


<210>  44
<211>  20
<212>  DNA
<213>  Artificial Sequence

<220>
<223>  primer sequence

<400>  44
gatgagtctg ttgccccact                                                  20


<210>  45
<211>  20
<212>  DNA
<213>  Artificial Sequence

<220>
<223>  primer sequence

<400>  45
aagtggttgc cttcccttgc                                                  20


<210>  46
<211>  24
<212>  DNA
<213>  Artificial Sequence

<220>
<223>  primer sequence

<400>  46
gttttgtctc tggtttcttc tgcc                                             24


<210>  47
<211>  1039
<212>  DNA
<213>  Solanum lycopersicum

<400>  47
tataataaaa tatcttatga aaaaaatatc acaaaaaatc agttttcaaa aatatttaca      60

aaaataattt aaagttagat taaaatttaa ataatctcgc taaaaataat ttacgtgtaa 120

atcaaactct aaaatcatcc tcaacgctca aagtggtcat attggacagt ggatggagtg 180

ggaaataata tttctgacat ataattattc taaaaaaata atattatgtg tgtaaattgt 240

aaattaacta aaatttttat gatatatttt aaatttataa tttcattaaa agaacttatt 300

ttattaaaaa tatatctata gtaaataaac ttaattaaaa tttcactcat cattaattaa 360

aattttaaat tcgcatctca gacaaaataa aatatactcc acaaactttg ttacactttc 420

gacaagaata tttatgggaa tttctcgtct cctttttcaa ttaggattta gttgagttgg 480

gtagaatcaa ataaaaaaat tatatatata tatatatata tatatatata tatatatatt 540

cctaaatgag aagaagatgt agaaattccc aaattaatct atatcacttt ctatatgttt 600

atcctttaat cattttcaat tttacgtagt aatatcgacc attacataag ttattaatgg 660

ctgagagaaa gcgagtagat gatcataata cagtatataa gacttgtgat tcagattatt 720

caatcttcca tgcaaattgt ccaatatatt gttcacttgt ggttccaatt attccacttc 780

ctctattaaa ttagctttga ttatagcaag cagtggtgga gtcaggattt ttaataaggg 840

agttcaaaat atgaaaaata aatatatgaa ttagtggaag ggggtttaaa catttatcat 900

acatatctaa aaaataattt taatcatata taaatttcct aagtatatgt tacctccgct 960

cctgataata gatattcttt aaaattaatc gagatgccaa tatattaatc gagatatcat 1020

gctatattta ttggaaaaa 1039

<210> 48
<211> 21
<212> DNA
<213> Solanum lycopersicum

<400> 48
ggaatttctc gtctcctttt t 21

<210> 49
<211> 21
<212> DNA
<213> Solanum lycopersicum

<400> 49
tactcgcttt ctctcagcca t 21

<210> 50
<211> 249
<212> DNA
<213> Solanum lycopersicum

<400> 50
tttgacatgc caacttcttt ttgtaaagtg gatatatgat taactttaaa aattaaaata 60

ttttttagag aaaaatcgag aaaaaaaatt ttgcacaaat tttactacac aaaataggca 120

gaattatttt ttgagcgatt taaaaagaaa aaagattaaa aaactaattt gaagcagtcg 180

tcgctaagat gtcaagtgtc tggttgtgca ttatgaaagt tggagtctac ttaaagtgtc 240

tggttgtgc 249

<210> 51
<211> 379
<212> DNA
<213> Solanum lycopersicum

<400> 51
ttttgatata ttggtgcccc tgtcgttcaa aaactagaac atatatgcac ttcactctaa 60

cgaaagatta aataaaaata tgtggcataa ttttattcat cgatccgaca tcgaattggt 120

aggtaaaatt gtgatatatg tatgtttgct aatataaagg atatgtatat tttaattttt 180

gaacggcaaa aaacaatcga agggttacaa tatgttatat taaagcctat cgtatatatt 240

aagatccact ttccagcata tattccctat tttatgtttc ttctctgaaa cttcaagaag 300

gatctatgta gaaatatact atagtgttat aagtaatact tattaattaa ttactccata 360

ggaaattggg gaggaggta 379

<210> 52
<211> 360
<212> DNA
<213> Solanum lycopersicum

<400> 52
tggatggcta ccatgttcat acagaaagat caacttttac aaatttaata tatacataca 60

tatatatata tatatatata tatatatata aatatatata tatatatata tatatatata 120

tatatatata taaattacaa atttctttta ttatttcacc tgatgtatat attgaattta 180

aatttaaatt taaatacaat atcatactta atacttacat tcaaaatatc taattactga 240

taaataaata tttattgcta tatcataatt ctgattgctg tttatatatc gtatatatga 300

gtactttaaa atatctatct taccaaatat cattcaaaaa caatgaagtg gggagaagaa 360

<210> 53
<211> 22
<212> DNA
<213> Solanum lycopersicum

<400> 53
aggatggcta ccatgttcat ac 22

<210> 54
<211> 24
<212> DNA
<213> Solanum lycopersicum

<400> 54

gtttcttctc cccacttcat tgtt 24

<210> 55
<211> 1041
<212> DNA
<213> Solanum lycopersicum

<400> 55
aaatttcaag ttgggatatt ctaaaatgtt atatgaatct ttttattata atatcatatg 60

ttatcgttat acttgagtac gattatattt ttcttttatt aaatatgaga ttctgagaga 120

gacacatacc acatgattta tgaggaaaaa gtaatgttac atcaaggtcc aaaaacttag 180

gcaaggacca tatatgattg taggtaagaa tatttttatt tacataatac ataaaagaac 240

aagttaattt tggactttct ttttttataa gaaaaatact agaagaggga ttttcaaagt 300

ccactatgaa aagtagaaat caaagtgtaa aaaaattatt ggaaagttga ggatagtggg 360

gctaagtcta taaatagttt ctcccaaagt tgttctcttg catatggcca acatgtatat 420

acaaatttag gtggaataat gtatattaat ttgggccctt gtattctctc ctacttgcta 480

tttcactctc ctcaactttc atatatatat atatatatat atatatatat atatatatat 540

taaaatttta tgagttcaga taaaccgttg catcgaaaaa gagttacgag ataacacaac 600

atgagatagt gttccatctt tcaacttttt taacatatgc ataatgttga gtgcaccaaa 660

atcagaaaca agataagggt acaagaaaaa acagttatcc attttgctaa cttagttggt 720

atactgattg aagcccaaca ttaaaaaagt gttctttttg ttcaatcatg ttcatatttt 780

gggataacct tagcttcata gtggaatgac acctatgtac tatgtttggc tttggtctct 840

ttcacatcag atggaggaat tggacaacta aaatcttgat aacttccatg aaaactaaaa 900

tcatgtttcc atatgatgtt ttggatggag aatgtgtttg cataagacaa caagacaaga 960

tttaacagat tatatagcaa aaagcaaagt ggcgtccgag accggttctt cgttctttca 1020

aatcgaaaga aagctagagg a 1041

<210> 56
<211> 22
<212> DNA
<213> Solanum lycopersicum

<400> 56
atgaggatag tggggctaag tc 22

<210> 57
<211> 25
<212> DNA
<213> Solanum lycopersicum

<400> 57
gtttcgtaac tctttttcga tgcaa 25